(19) **Europäisches Patentamt / European Patent Office / Office européen des brevets**

(11) **EP 2 544 696 B1**

(12) # EUROPEAN PATENT SPECIFICATION

(45) Date of publication and mention
of the grant of the patent:
**22.05.2019 Bulletin 2019/21**

(21) Application number: **11752778.8**

(22) Date of filing: **10.03.2011**

(51) Int Cl.:
*A61K 31/736* (2006.01)   *A61K 31/723* (2006.01)
*A61K 31/734* (2006.01)   *A61P 1/18* (2006.01)
*A61P 3/00* (2006.01)   *A61P 3/06* (2006.01)
*A61P 3/10* (2006.01)   *G01N 30/02* (2006.01)
*G01N 30/90* (2006.01)   *G01N 33/15* (2006.01)
*A61K 45/06* (2006.01)   *A61P 5/50* (2006.01)
*A23L 29/244* (2016.01)   *A23L 29/256* (2016.01)
*A23L 29/269* (2016.01)   *A23L 33/21* (2016.01)

(86) International application number:
**PCT/CA2011/000260**

(87) International publication number:
**WO 2011/109900 (15.09.2011 Gazette 2011/37)**

(54) **FOOD COMPRISING GLUCOMANNAN, XANTHAN GUM AND ALGINATE FOR THE TREATMENT OF METABOLIC DISORDERS**

NAHRUNGSMITTEL MIT GLUCOMANNAN, XANTHANGUMMI UND ALGINAT ZUR BEHANDLUNG VON STOFFWECHSELSTÖRUNGEN

ALIMENT COMPRENANT DU GLUCOMANNANE, DE LA GOMME DE XANTHANE ET UN ALGINATE POUR LE TRAITEMENT DE TROUBLES MÉTABOLIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2010 US 357658 P**
**10.03.2010 US 312630 P**

(43) Date of publication of application:
**16.01.2013 Bulletin 2013/03**

(73) Proprietor: **Inovobiologic, Inc.**
**Calgary, Alberta T2N 4X7 (CA)**

(72) Inventors:
• **GAHLER, Roland J.**
**Burnaby**
**British Columbia V3N 4S9 (CA)**
• **LYON, Michael**
**Nanaimo**
**British Columbia V9T 2S3 (CA)**
• **WOOD, Simon**
**Victoria**
**British Columbia V8N 1V1 (CA)**

• **LAWSON, Christopher John**
**Reading**
**RG6 5QQ (GB)**

(74) Representative: **Crooks, Elizabeth Caroline**
**Kilburn & Strode LLP**
**Lacon London**
**84 Theobalds Road**
**London WC1X 8NL (GB)**

(56) References cited:
**US-A1- 2006 228 397     US-A1- 2008 027 024**

• **CARABIN IOANA G ET AL: "Supplementation of the diet with the functional fiber PolyGlycoplex TM is well tolerated by healthy subjects in a clinical trial", NUTRITION JOURNAL, BIOMED CENTRAL, GB, vol. 8, no. 1, 5 February 2009 (2009-02-05), page 9, XP021050053, ISSN: 1475-2891, DOI: 10.1186/1475-2891-8-9**

EP 2 544 696 B1

**(Cont. next page)**

- DATABASE BIOSIS [Online] BIOSCIENCES INFORMATION SERVICE, PHILADELPHIA, PA, US; April 2008 (2008-04), JENKINS ALEXANDRA ET AL: "Glycemic Index Reduction by a Viscous Polysaccharide Blend Independent of Food Form: Determination of the Glycemic Reduction Index Potential (GRIP)", XP002700441, Database accession no. PREV201200748181
- GARY J GROVER ET AL: "Effects of the soluble fiber complex PolyGlycopleX TM (PGX TM) on glycemic control, insulin secretion, and GLP-1 levels in Zucker diabetic rats", LIFE SCIENCES, PERGAMON PRESS, OXFORD, GB, vol. 88, no. 9, 15 November 2010 (2010-11-15), pages 392-399, XP028145756, ISSN: 0024-3205, DOI: 10.1016/J.LFS.2010.11.014 [retrieved on 2010-11-27]
- GARNA, H. ET AL.: 'New method for a two-step hydrolysis and chromatographic analysis of pectin neutral sugar chains' JOURNAL OF. AGRICULTURAL AND FOOD CHEMISTRY vol. 52, 2004, pages 4652 - 4659, XP055099498
- GALLAGHER EMILY JANE ET AL: "The metabolic syndrome--from insulin resistance to obesity and diabetes", ENDOCRINOLOGY AND METABOLISM CLINICS OF NORTH AMERICA, W.B. SAUNDERS COMPANY, PHILADELPHIA, vol. 37, no. 3, 1 September 2008 (2008-09-01), pages 559-579,vii, XP009121934, ISSN: 0889-8529, DOI: 10.1016/J.ECL.2008.05.002

**Description**

CROSS-REFERENCES TO RELATED APPLICATIONS

This application claims the benefit of Application No. 61/312,630, filed March 10, 2010, and Application No. 61/357,658, filed June 23, 2010.

FIELD OF THE INVENTION

The invention relates to dietary fiber compositions, medical foods comprising dietary fiber compositions, and their use to delay the onset and/or reduce the severity of metabolic syndrome and of Type II diabetes.

BACKGROUND

Obesity and metabolic syndrome, conditions that may lead to the development of Type II diabetes, have become more and more common. An increase in visceral obesity, serum glucose, and insulin levels, along with hypertension and dyslipidemia are a group of clinical conditions that are collectively known as the metabolic syndrome (E.J. Gallagher et al., Endocrinol. Metab. Clin. North Am. 37:559-79 (2008)). It has been found that these conditions are due to increasing insulin resistance of the cells, and in many cases, these symptoms are a precursor to Type II diabetes. There are currently controversies over the exact diagnostic criteria that identify metabolic syndrome, and no pharmaceuticals have been approved for its treatment, although associated dyslipidemias and hypertension do have specific drug interventions. Type II diabetes is typically managed with various pharmaceuticals to regulate blood sugar and, in more severe cases, insulin injections. However, diet and weight loss play a major role in correcting many metabolic abnormalities associated with both metabolic syndrome and Type II diabetes (Yip et al., Obesity Res. 9:341S-347S (2001)). Research has shown that those who have metabolic syndrome have a 50% greater risk of a experiencing a major coronary event (D.E. Moller et al., Annu. Rev. Med. 56:45-62 (2005)). As such, any reductions in weight, fasting insulin, and glucose would confer significant health benefits on those individuals so afflicted.

Intake of foods with a high glycemic index is known to lead to overeating and obesity (Ludwig et al., Pediatrics 103(3):E26 (1999)). Therefore, it is preferable that any agent used in the management of diabetic or pre-diabetic conditions as well as weight loss be low in glycemic index. It is most preferable if such agents reduce the glycemic index of foods.

A reduction in carbohydrate intake is also required in successful management of diabetic conditions. Diet counseling is helpful, but diabetics experience more food cravings as they experience more frequent states of hypoglycemia (Strachan et al., Physiol. Behav. 80(5):675-82 (2004)). Additionally, therapies lowering blood glucose levels in diabetic patients are often associated with the undesirable side effect of body weight gain (Schultes et al., J. Clin. Endocrinol. Metabol. 88(3): 1133-41 (2003)). It has been reported that diets high in soluble fiber may reduce the risk of diabetes through increased insulin sensitivity (Ylonen et al., Diabetes Care 26:1979-85 (2003)). This may result from the possible role of dietary fiber in blood sugar regulation. It has also been reported that high viscosity meals produce a greater sense of fullness compared to low viscosity meals (Marciani et al., Am. J. Physiol. Gastrointest. Liver Physiol. 280:G1227-33 (2001)).

US2008/027024 discloses a dietary fiber composition that is effective to lower plasma blood glucose, to improve glycemic response and to lower cholesterol levels. US2006/228397 discloses a dietary fiber composition that is effective to increase insulin sensitivity and to reduce body fat in a subject.

Thus, there is a need for dietary fiber compositions that assist in the management of metabolic syndrome including diabetic conditions by lowering blood sugar levels and promoting satiety. The present invention addresses this need and others.

SUMMARY

In one aspect, the invention provides a medical food for use in the prevention, treatment, or amelioration of one or more symptoms associated with metabolic syndrome as defined in the claims. The medical food according to this aspect of the invention comprises a highly viscous polysaccharide dietary fiber composition comprising a viscous fiber blend ("VFB") or complex ("VFC") thereof, comprising from 48% to 90% (w/w) glucomannan, from 5% to 20% (w/w) xanthan gum, and from 5% to 30% (w/w) alginate, and at least one macronutrient selected from the group consisting of protein, carbohydrate, and fat.

It is disclosed herein a method of preparing a medical food product comprising the step of adding an effective amount of a dietary fiber composition comprising a viscous fiber blend (VFB) or complex ("VFC") thereof, comprising glucomannan, xanthan gum, and alginate, to the medical food product. In some aspects of the disclosure, the medical food product is compounded for the prevention, treatment or amelioration of one or more symptoms associated with a metabolic disease or disorder. In some aspects of the disclosure, the dietary fiber composition added to the medical food product comprises from about 48% to about 90% (w/w) glucomannan, from about 5% to about 20% (w/w) xanthan gum, and from about 5% to about 30% (w/w) alginate.

In another aspect, disclosed is a medical food for use in a method for preventing, treating, or ameliorating one or more symptoms associated with a metabolic disease or disorder. The use according to this aspect comprises administering to a human subject in need thereof from about 25 mg/kg/day to about 1000 mg/kg/day of a highly viscous polysaccharide dietary fiber composition comprising a viscous fiber blend (VFB) or complex (VFC) thereof, comprising from about 48% to about 90% (w/w) glucomannan, from about 5% to about 20% (w/w) xanthan gum, and from about 5% to about 30% (w/w) alginate effective for a period of time effective to prevent, treat, or ameliorate one or more symptoms associated with the metabolic disease or disorder in the subject.

In yet another aspect, disclosed is a medical food for use in a method for ameliorating at least one symptom associated with the progression of insulin resistance in a mammalian subject suffering from, or at risk for, developing type II diabetes. The use according to this aspect comprises administering to the mammalian subject in need thereof from about 25 mg/kg/day to about 1000 mg/kg/day of a highly viscous polysaccharide dietary fiber composition comprising a viscous fiber blend (VFB), or complex thereof (VFC), comprising from about 48% to about 90% (w/w) glucomannan, from about 5% to about 20% (w/w) xanthan gum, and from about 5% to about 30% (w/w) alginate for a period of at least two weeks.

It is disclosed herein a method for determining the component sugars in a sample comprising at least one polysaccharide. The methods disclosed herein comprise: (a) hydrolyzing a sample comprising at least one polysaccharide with an acid to produce a hydrolysate; (b) separating the hydrolysis products in the hydrolysate with a chromatographic method; (c) detecting the hydrolysis products separated in step (b); and (d) comparing the hydrolysis products detected in step (c) to one or more reference standards to determine the component sugars in the sample.

DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

FIGURE 1A graphically illustrates the effect of VFC, cellulose, or inulin diets on body weight (g) over time during the eight week study in Zucker diabetic rats, as described in Example 1;

FIGURE 1B graphically illustrates the effect of VFC, cellulose, or inulin diets on food consumption (g/day) over time during the 8-week study in Zucker diabetic rats, as described in Example 1;

FIGURE 2A graphically illustrates the effect of VFC-, cellulose-, or inulin-containing diets on fasted blood glucose levels (mg/dL) over time during the 8-week study in Zucker diabetic rats, as described in Example 1;

FIGURE 2B graphically illustrates the effect of VFC-, cellulose-, or inulin-containing diets on fasted serum insulin levels (ng/mL) over time during the 8-week study in Zucker diabetic rats, as described in Example 1;

FIGURE 2C graphically illustrates the effect of VFC-, cellulose-, or inulin-containing diets on non-fasted blood glucose levels (mg/dL) over time during the 8-week study in Zucker diabetic rats, as described in Example 1;

FIGURE 2D graphically illustrates the effect of VFC-, cellulose-, or inulin-containing diets on fasted Homeostasis Model Assessment (HOMA) scores (mg*U/ml$^2$) over time during the 8-week study in Zucker diabetic rats, as described in Example 1;

FIGURE 3A graphically illustrates the composite insulin sensitivity index (CISI) scores for fasted Zucker diabetic rats fed either VFC, cellulose, or inulin diets during the 8-week study, as described in Example 1;

FIGURE 3B graphically illustrates the composite insulin sensitivity index (CISI) scores for non-fasted Zucker diabetic rats fed either VFC, cellulose, or inulin diets during the 8-week study, as described in Example 1;

FIGURE 3C graphically illustrates the HOMA scores for non-fasted Zucker diabetic rats fed either VFC, cellulose, or inulin diets during the 8-week study, as described in Example 1;

FIGURE 4 graphically illustrates the level of serum triglycerides measured in fasted Zucker diabetic rats fed either VFC, cellulose, or inulin diets during the 8-week study, as described in Example 1;

FIGURE 5A graphically illustrates the effect of VFC-, cellulose-, or inulin-containing diets on Zucker diabetic rats after 8 weeks on renal tubule dilation, based on a histologic score of 0-5, with 5 being the most severe, as described in Example 1;

FIGURE 5B graphically illustrates the effect of VFC-, cellulose-, or inulin-containing diets on Zucker diabetic rats after 8 weeks on renal tubule degeneration/regeneration, based on a histologic score of 0-5, with 5 being the most severe, as described in Example 1;

FIGURE 5C graphically illustrates the effect of VFC-, cellulose-, or inulin-containing diets on Zucker diabetic rats after 8 weeks on renal mesangial expansion, based on a histologic score of 0-5, with 5 being the most severe, as described in Example 1;

FIGURE 6 graphically illustrates the percentage of pancreatic islet insulin immunoreactive area present in Zucker diabetic rats fed either VFC, cellulose, or inulin diets at the end of the 8-week study, as determined by staining with anti-rat insulin antibody, as described in Example 1;

FIGURE 7A graphically illustrates the histological score for pancreatic islet mononuclear inflammatory cell infiltrates present in Zucker diabetic rats fed either VFC, cellulose, or inulin diets at the end of the 8-week study, based on a histologic score of 0-5, with 5 being the most severe, as described in Example 1;

FIGURE 7B graphically illustrates the histological score for pancreatic islet cell degeneration present in Zucker diabetic rats fed either VFC, cellulose, or inulin diets at the end of the 8-week study, based on a histologic score of 0-5, with 5 being the most severe, as described in Example 1;

FIGURE 7C graphically illustrates the histological score for the amount of pancreatic islet fibrosis present in Zucker diabetic rats fed either VFC, cellulose, or inulin diets at the end of the 8-week study, based on a histologic score of 0-5, with 5 being the most severe, as described in Example 1;

FIGURE 8A graphically illustrates the effect of VFC-, cellulose-, or inulin-containing diets on Zucker diabetic rats after 8 weeks on hepatic steatosis, as measured by reduced Sudan black staining, based on a histologic score of 0-5, with 5 being the most severe, as described in Example 1;

FIGURE 8B graphically illustrates the effect of VFC-, cellulose-, or inulin-containing diets on Zucker diabetic rats after 8 weeks on hepatic microvesicular vacuolation, based on a histologic score of 0-5, with 5 being the most severe, as described in Example 1;

FIGURE 8C graphically illustrates the effect of VFC-, cellulose-, or inulin-containing diets on Zucker diabetic rats after 8 weeks on hepatic macrovesicular vacuolation, based on a histologic score of 0-5, with 5 being the most severe, as described in Example 1;

FIGURE 9 graphically illustrates the effect of VFC or cellulose on body weight gain and serum triacylglycerols (TAG) in Sprague-Dawley sucrose-fed rats over the 43-week study, as described in Example 2;

FIGURE 10A graphically illustrates the effect of VFC or control (skimmed milk powder) on plasma PYY levels for all healthy adult study participants over a 3-week study period (VI = study initiation day 0; V2 = day 14; V3 = day 21), as described in Example 4;

FIGURE 10B graphically illustrates the effect of VFC or control (skimmed milk powder) on plasma PYY levels in healthy adults study participants with a BMI <23 over a 3-week study period (VI = study initiation day 0; V2 = day 14; V3 = day 21), as described in Example 4;

FIGURE 10C graphically illustrates the effect of VFC or control (skimmed milk powder) on fasting ghrelin levels in healthy adult study participants over a 3-week period (VI = study initiation day 0; V2 = day 14; V3 = day 21), as described in Example 4;

FIGURE 11A graphically illustrates the flow curve comparison of ungranulated VFB (referred to as Ternary Mixture 1 ("TM1") and processed (e.g., granulated) VFC (PGX®) at 0.5% (w/w), as described in Example 6;

FIGURE 11B graphically illustrates the flow curve comparison of ungranulated VFB (referred to as Ternary Mixture 1 ("TM1")) and processed (e.g., granulated) VFC (PGX®) at 0.2% (w/w), as described in Example 6;

FIGURE 11C graphically illustrates the flow curve comparison of ungranulated VFB (referred to as Ternary Mixture 1 ("TM1") and processed (e.g., granulated) VFC (PGX®) at 0.1% (w/w), as described in Example 6;

FIGURE 12A graphically illustrates the power law K comparison of ungranulated VFB (TM1), processed (e.g., granulated) VFC (PGX®) and xanthan gum, as described in Example 6;

FIGURE 12B graphically illustrates the power law η comparison of ungranulated VFB (TM1), processed (e.g., granulated) VFC (PGX®) and xanthan gum, as described in Example 6;

FIGURE 13A graphically illustrates the flow curve of konjac glucomannan at 0.1%, 0.2%, and 0.5% (w/w) as measured at 25°C, as described in Example 6;

FIGURE 13B graphically illustrates the flow curve of xanthan gum at 0.1%, 0.2%, and 0.5% (w/w) as measured at 25°C, as described in Example 6;

FIGURE 13C graphically illustrates the flow curve of sodium alginate at 0.1%, 0.2%, and 0.5% (w/w) as measured at 25°C, as described in Example 6;

FIGURE 14A graphically illustrates the flow curve of unheated aqueous solutions (0.5% concentration) of ternary mixtures comprising konjac glucomannan, xanthan gum, and sodium alginate, containing konjac glucomannan (KM) and xanthan gum (XG) at a constant ratio (KM:XG = 4.12:1) and variable amounts of sodium alginate (0%, 2%, 5%, 8%, 11%, 13%, 17%, 21%, 24%, 27%, 30%, and 33%), measured at 25°C, as described in Example 6;

FIGURE 14B graphically illustrates the flow curve of aqueous solutions (0.5% concentration) heated for 1 hour of ternary mixtures comprising konjac glucomannan, xanthan gum, and sodium alginate, containing konjac glucomannan (KM) and xanthan gum (XG) at a constant ratio (KM:XG = 4.12:1) and variable amounts of sodium alginate (0%, 2%, 5%, 8%, 11%, 13%, 17%, 21%, 24%, 27%, 30%, and 33%), measured at 25°C, as described in Example 6;

FIGURE 14C graphically illustrates the flow curve of aqueous solutions (0.5% concentration) heated for 4 hours of ternary mixtures comprising konjac glucomannan, xanthan gum, and sodium alginate, containing konjac glucomannan (KM) and xanthan gum (XG) at a constant ratio (KM:XG = 4.12:1) and variable amounts of sodium alginate (0%, 2%, 5%, 8%, 11%, 13%, 17%, 21%, 24%, 27%, 30%, and 33%), measured at 25°C, as described in Example 6;

FIGURE 15A graphically illustrates the dependency of K on the proportion of sodium alginate in the mixture for unheated or heated (one hour) 0.5% aqueous solutions of mixtures of konjac glucomannan, xanthan gum, and sodium alginate at a constant KM:XG ratio (4.12:1) and variable amounts of alginate (0 to 33%), as described in Example 6;

FIGURE 15B graphically illustrates the dependency of n on the proportion of sodium alginate in the mixture for the unheated and heated (one hour) 0.5% aqueous solution of mixtures of konjac glucomannan, xanthan gum, and sodium alginate at a constant KM:XG ratio (4.12:1) and variable amounts of alginate (0 to 33%), as described in Example 6;

FIGURE 16A graphically illustrates the apparent sedimentation concentration distributions g*(s) vs s for glucomannan at a loading concentration of 2 mg/ml and at I = 0.0, with a Rotor speed 45,000 rpm, temperature = 20.0°C. The ordinate is expressed in fringe units per Svedberg (S) and the abscissa is in Svedberg units, as described in Example 6;

FIGURE 16B graphically illustrates the apparent sedimentation concentration distributions g*(s) vs s for sodium alginate at a loading concentration of 2 mg/ml and at I = 0.0, with a Rotor speed 45,000 rpm, temperature = 20.0°C. The ordinate is expressed in fringe units per Svedberg (S) and the abscissa is in Svedberg units, as described in Example 6;

FIGURE 16C graphically illustrates the apparent sedimentation concentration distributions g*(s) vs s for xanthan at a loading concentration of 2 mg/ml and at I = 0.0, with a Rotor speed 45,000 rpm, temperature = 20.0°C. The ordinate is expressed in fringe units per Svedberg (S) and the abscissa is in Svedberg units, as described in Example 6;

FIGURE 17A graphically illustrates the apparent sedimentation concentration distributions for unprocessed/nongranulated VFB (referred to as "TM1") at ionic strengths 0-0.2 M, as described in Example 6;

FIGURE 17B graphically illustrates the apparent sedimentation concentration distributions for unprocessed/nongranulated VFB (referred to as "TM1") at ionic strengths 0-0.01 M, as described in Example 6;

FIGURE 17C graphically illustrates the apparent sedimentation concentration distributions for processed/granulated) VFC (PGX®) at ionic strengths 0-0.01 M, as described in Example 6;

FIGURE 17D graphically illustrates the apparent sedimentation concentration distributions for processed/granulated VFC (PGX®) at ionic strengths 0-0.2 M, as described in Example 6;

FIGURE 18A graphically illustrates the effect of ionic strength (expressed in molar concentration units M) on the amount of material with a sedimentation coefficient > 3.5S for unprocessed/ungranulated VFB (TM1), as described in Example 6;

FIGURE 18B graphically illustrates the effect of ionic strength (expressed in molar concentration units M) on the amount of material with a sedimentation coefficient > 3.5S for processed/granulated) VFC (PGX®), as described in Example 6;

FIGURE 19A graphically illustrates the sedimentation coefficient distributions for unheated mixtures containing a fixed glucomannan:xanthan ratio (KM:XG = 4.12:1) and varying alginate concentrations (from 0% to 33%), as described in Example 6; and

FIGURE 19B graphically illustrates the sedimentation coefficient distributions for heated (1 or 4 hours) mixtures containing a fixed glucomannan: xanthan ratio (KM:XG = 4.12:1) and varying alginate concentrations (from 0% to 33%), as described in Example 6.

## DETAILED DESCRIPTION

The scope of the invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human (or animal) body by therapy. The present invention provides dietary supplements, medical foods, and uses of such medical foods effective to delay the onset, slow the progression, and/or ameliorate at least one of the symptoms of a metabolic disease or disorder, such as metabolic syndrome, type I diabetes, type II diabetes, pancreatic disease, and/or hyperlipidemia.

As used herein, the term "metabolic syndrome" refers to one or more of the following symptoms: an increase in visceral obesity, serum glucose, and insulin levels, along with hypertension and dyslipidemia (E.J. Gallagher et al., Endocrinol. Metab. Clin. North Am. 37:559-79 (2008)). Metabolic syndrome is a name for a group of symptoms that occur together and are associated with the increased risk of developing coronary artery disease, stroke, and type II diabetes. The symptoms of metabolic syndrome include extra weight around the waist (central or abdominal obesity), high blood pressure, high triglycerides, insulin resistance, low HDL cholesterol, and tissue damage caused by high glucose. It is believed that insulin resistance is the main cause of metabolic syndrome.

As used herein, the term "ameliorate at least one of the symptoms of metabolic disease or disorder," includes symptomatic therapy to lessen, alleviate, or mask the symptoms of the disease or disorder, as well as therapy for preventing, lowering, stopping, or reversing the progression of severity of the condition or symptoms being treated. As such, the term "treatment" includes both medical therapeutic treatment of an established condition or symptoms and/or prophylactic administration, as appropriate.

As used herein, the term "treating" also encompasses, depending on the condition of the subject in need thereof, preventing the metabolic disease or disorder, or preventing one or more symptoms associated with the pathology of the metabolic disease or disorder, including onset of the metabolic disease or disorder or of any symptoms associated therewith, as well as reducing the severity of the metabolic disease or disorder or preventing a recurrence of one or more symptoms associated with the metabolic disease or disorder.

As used herein, the term "medical food" refers to a food that is formulated to be consumed or administered enterally under the supervision of a physician and that is intended for the specific dietary management of a disease or condition for which distinctive nutritional requirements, based on recognized scientific principles, are established by medical evaluation.

As used herein, the term "glucomannan" refers to a water-soluble dietary fiber with β-(1,4)-linked-D-mannose and β-(1,4)-linked-D-glucose residues in approximately 3:1 ratio and various α-linked galactose end groups. It is most commonly isolated from konjac root (Amorphophallus konjac), but can also be isolated from other plant sources.

As used herein, the term "xanthan gum" refers to a heteropolysaccharide containing glucose, mannose, potassium or sodium glucuronate, acetate, and pyruvate.

As used herein, the term "alginate" refers to a mixed polymer of mannuronic acid and guluronic acid.

As used herein, the term "fiber blend" refers to a mixture of fibers.

As used herein, the term "viscous fiber blend" ("VFB") refers to a mixture of glucomannan, xanthan gum, and alginate.

As used herein, the term "viscous fiber complex" ("VFC") refers to an interlocking matrix of the three components glucomannan, xanthan gum, and alginate in which the components are processed in a manner (e.g., granulation) that allows them to interact to form a novel ingredient rather than a mixture of three separate components by forming secondary and tertiary interactions (junction zones and networks) between the raw ingredients that prevent the individual components from exhibiting the properties that they would each show in their pure state.

## Medical Foods

In one aspect, the present invention provides medical foods for use in the prevention, treatment, or amelioration of one or more symptoms associated with a metabolic disease or disorder, such as metabolic syndrome, type I or type II diabetes, exocrine pancreatic insufficiency, including patients suffering from chronic pancreatitis, and/or hyperlipidemia. The medical food for use according to this aspect of the invention comprises a highly viscous polysaccharide dietary fiber composition comprising a viscous fiber blend (VFB), or complex thereof (VFC), comprising from about 48% to about 90% (w/w) glucomannan, from about 5% to about 20% (w/w) xanthan gum, and from about 5% to about 30% (w/w) alginate, and at least one macronutrient selected from the group consisting of protein, carbohydrate, and fat.

As described in pending U.S. Patent Application No. 11/400,768, filed on April 7, 2006, and pending U.S. Patent Application No. 11/830,615, filed on July 30, 2007, a highly viscous polysaccharide dietary fiber composition comprising a fiber blend (VFB), or complex thereof (VFC), produced by combining from about 48% to about 90% (w/w) glucomannan, from about 5% to about 20% (w/w) xanthan gum, and from about 5% to about 30% (w/w) alginate, has been developed, commercially referred to as "PolyGlycopleX®" or "PGX®," that possesses a very high water hold capacity and gel-forming property. The constituent polysaccharide components of this fiber composition are complementary to each other and act synergistically to form strong interactions that lead to a level of viscosity that is three to five times higher than any other currently known polysaccharide. As described in Examples 5 and 6 herein, it has been determined that when processed (e.g., granulated), the three components glucomannan, xanthan gum, and alginate interact to form a novel ingredient (complex ("VFC")) rather than a mixture of 3 separate components by forming secondary and tertiary interactions (junction zones and networks) between the raw ingredients that prevent the individual components from exhibiting the properties that they would each show in their pure state.

This highly viscous dietary fiber composition imparts a significant increase in the viscosity of gastrointestinal contents at a lower gravimetric quantity than that which would be required with other soluble fibers. This highly concentrated property allows this fiber composition to impart substantial physiological effects at doses that are significantly lower than other soluble fibers, thus making it easier to incorporate meaningful quantities of this material into foodstuffs.

In one embodiment, the polysaccharides used in the production of the viscous fiber blend (VFB) are processed via granulation to produce an interlocking matrix of the three components (i.e., a complex (VFC)). As used herein, "granulation" refers to any process of size enlargement in which small particles are gathered together into larger, permanent aggregates. Granulation may be accomplished by agitation in mixing equipment, by compaction, extrusion, or globulation. The dietary fiber compositions may be granulated using various mesh sizes. The term "mesh" refers to the size of the particle as determined by its ability to pass through a screen having holes of defined dimensions. The mesh sizes used herein are Tyler equivalents, as set forth in Table 21-12 of the Chemical Engineers Handbook (5th ed., Perry & Chilton, eds.). The larger the granulation (i.e., the smaller the mesh size) of the dietary fiber composition/complex, the longer it takes for a desired viscosity to be attained. In some embodiments, the dietary fiber composition/complex is granulated using a combined mesh size by separating granulated materials by their particle size, then recombining the particle-size separated granules to give the desired viscosity profile. For example, a combined mesh size of 30 to 60 is obtained by combining granules of 30 mesh (about 600 microns), granules of about 40 mesh (about 400 microns), and granules of about 60 mesh (250 microns).

The proportions of glucomannan, xanthan gum, and alginate in the viscous dietary fiber blend/complex (VFB/C) contained in the medical food may be from about 48% to about 90% of glucomannan (such as from about 60% to about 80%, or from about 60% to about 90%, or from about 65% to about 75%, or from about 50% to about 80%, or from about 50% to about 70%, or about 70%), from about 5% to about 20% of xanthan gum (such as from about 10% to about 20% or from about 11% to about 13%, or from about 13% to about 17%, or about 13%, or about 17%), and from about 5% to about 30% of alginate (such as from about 10% to about 20% or from about 13% to about 17%, or about 13%, or about 17%). In some embodiments, proportions of glucomannan, xanthan gum, and alginate in the dietary compositions contained in the medical food are about 70% glucomannan, from about 13% to about 17% xanthan, and from about 13% to about 17% alginate.

In some embodiments, the medical foods are formulated to provide a total daily consumption in a human subject of from 1.0 g to 100 g of a viscous fiber blend, or complex thereof (VFB/C), comprising from about 48% to about 90% (w/w) glucomannan, from about 5% to about 20% (w/w) xanthan gum, and from about 5% to about 30% (w/w) alginate (VFB/C), such as from about 5 g to about 50 g VFB/C per day, such as from about 10 g to about 35 g VFB/C per day, from about 12 g to 35 g VFB/C per day, or such as from about 15 g to 35 g VFB/C per day, such as from about 20g to 35g VFB/C per day, such as from about 12g to about 25g VFB/C per day, such as from about 15g to about 25g VFB/C per day. In some embodiments, the medical foods are formulated to provide a daily dosage of VFB/C in a human subject of from about 25 mg/kg/day to about 1000 mg/kg/day, such as from about 50 mg/kg/day to about 600 mg/kg/day, such as from about 100 mg/kg/day to about 500 mg/kg/day, such as from about 200 mg/kg/day to about 400mg/kg/day.

The medical food products for use in the invention may further contain additional components such as proteins or amino acids, carbohydrates, lipids, vitamins, minerals, and cofactors, natural or artificial flavors, dyes or other coloring additives, and preservatives. The term "vitamins" includes, thiamin, riboflavin, nicotinic acid, pantothenic acid, pyridoxine, biotin, folic acid, vitamin B12, lipoic acid, ascorbic acid, vitamin A, vitamin D, vitamin E, and vitamin K. Also included within the term "vitamins" are cofactors and coenzymes such as coenzymes including thiamine pyrophosphates (TPP), flavin mononucleotide (FMM), flavin adenine dinucleotide (FAD), nicotinamide adenine dinucleotide (NAD), nicotinamide adenine dinucleotide phosphate (NADP), Coenzyme A (CoA), pyridoxal phosphate, biocytin, tetrahydrofolic acid, coenzyme B12, lipoyllysine, 11-cis-retinal, and 1,25-dihydroxycholecalciferol. The term "vitamins" also includes choline, carnitine, and alpha, beta, and gamma carotenes. The term "minerals" refers to inorganic substances, metals, required in the human diet, including, calcium, iron, zinc, selenium, copper, iodine, magnesium, phosphorus, chromium, manganese, potassium, and mixtures thereof. The mineral may be in the form of a salt, an oxide, or a chelated salt.

In some embodiments, the medical foods for use in the invention further comprises one or more lipids. As used in accordance with this embodiment of the invention, a lipid is defined as a substance such as a fat, oil, or wax that dissolves in alcohol but not in water. As used herein, the terms "fat" and "oil" are used interchangeably and comprise fatty acids. In some embodiments, the lipid for use in the composition comprises a fat selected from the group consisting of a dairy fat (e.g., milk fat, butter fat), an animal fat (e.g., lard) or a vegetable fat (e.g., coconut oil, cocoa butter, palm oil, or margarine).

In some embodiments, the lipid for use in the medical foods of the invention comprises an edible oil or a mixture of oils. Such oils include vegetable oils (e.g., canola oil, soybean oil, palm kernel oil, olive oil, safflower oil, sunflower seed oil, flaxseed (linseed) oil, corn oil, cottonseed oil, peanut oil, walnut oil, almond oil, grape seed oil, evening primrose oil, coconut oil, borage oil, and blackcurrant oil); marine oils (e.g., fish oils and fish liver oils), or a mixture thereof.

In some embodiments, the lipid for use in the medical foods of the invention comprises oils containing medium-chain triglycerides, such as coconut oil, palm kernel oil, and butter or medium-chain triglycerides in purified form.

In some embodiments, the medical foods for use in the invention provide the sole source of calories and nutrients for a patient. In some embodiments, the medical food for use in the invention is designed to provide the primary source of fiber in the diet of a human subject. In some embodiments, the medical food for use in the invention is designed to provide the sole source of fiber in the diet of a human subject and is labeled and/or administered by a physician accordingly.

Medical foods that are to be consumed as part of a complete, balanced diet are typically formulated to replace one or more meals throughout the day, thereby decreasing the amount of fiber consumed from conventional foods. Because medical foods are administered under the supervision of a physician, it is unlikely that patients would consume additional dietary fiber supplements containing fiber.

The medical foods for use in the present invention are for use by a select population of patients that are under the care and supervision of a physician. The medical foods for use in the invention may be administered to a mammalian subject, such as a human suffering from, or at risk for, developing a metabolic condition in order to prevent, treat, or ameliorate one or more symptoms associated with the metabolic disease or disorder, such as metabolic syndrome, (also known as syndrome X and insulin resistance syndrome), type I diabetes, type II diabetes, obesity, non-alcoholic steatohepatosis (fatty liver disease), pancreatic disease, and hyperlipidemia, as further described herein.

In some embodiments, the medical food for use in the invention is administered to a subject in need thereof at least once per day. In some embodiments, the medical food for use in the invention is administered two times a day, preferably once in the morning and once in the afternoon/evening. A typical treatment regime for the medical foods will continue from at least two weeks to eight weeks or longer. Depending on such factors as the medical conditions being treated and the response in the patient, the treatment regime may be extended until the patient experiences amelioration of at least one symptom of the disease or disorder. A medical food for use in the present invention will typically be consumed in two servings per day as a meal replacement or snack between meals. In some embodiments, the medical food for use in the invention is administered to the subject as the sole source of food three to four times per day as part of a medically supervised very low calorie diet regime. An exemplary use of a very low calorie diets is in the treatment of obesity to bring about rapid weight loss and the reduction of cardiometabolic risk factors.

**Methods of Making Medical Foods**

Disclosed herein is a method of preparing a medical food product comprising the step of adding an effective amount of a dietary fiber composition comprising a viscous fiber blend (VFB), or complex thereof (VFC) comprising glucomannan, xanthan gum, and alginate, to the medical food product. It is disclosed herein, the method of preparing a medical food product comprises the step of adding an effective amount of a dietary fiber composition comprising a fiber complex (VFC) formed from a viscous fiber blend (VFB) comprising glucomannan, xanthan gum, and alginate to the medical food product.

In some embodiments, the medical food product is for use in the prevention, treatment, or amelioration of one or more symptoms associated with a metabolic disease or disorder. It is disclosed herein, the dietary fiber composition added to the medical food product comprises a fiber blend (VFB), or a fiber complex (VFC) formed from the fiber blend (e.g., granulated VFB), comprising from about 48% to about 90% (w/w) glucomannan (such as from about 60% to about 80%, or from about 60% to about 90%, or from about 65% to about 75%, or from about 50% to about 80%, or from about 50% to about 70%, or about 70%), from about 5% to about 20% (w/w) xanthan gum (such as from about 10% to about 20%, or from about 11% to about 13%, or from about 13% to about 17%, or about 13%, or about 17%), and from about 5% to about 30% (w/w) alginate (such as from about 10% to about 20% or from about 13% to about 17%, or about 13%, or about 17%). It is disclosed herein, proportions of glucomannan, xanthan gum, and alginate in the fiber blend, or in the fiber complex formed from the fiber blend, contained in the dietary fiber composition that is added to the medical food are about 70% glucomannan, from about 13% to about 17% xanthan, and from about 13% to about 17% alginate.

It is disclosed herein, the amount of the dietary fiber composition comprising the viscous fiber blend (VFB), or complex thereof (VFC), added to a medical food product formulated for the treatment or prevention of a metabolic disease or disorder is from about 5% to about 20% of the total weight of the medical food product. It is disclosed herein, the amount of the dietary fiber composition, or complex thereof (VFB/C) added to the medical food product comprises from about 1 g to 100 g per day, such as from 5 g to about 50 g per day, from about 10 g to 35 g per day, such as from about 12 g to 35g per day, such as from about 15 g to 35 g per day, such as from about 20g to 35g per day, such as from about 12g to about 25 g per day, such as from about 15g to about 25g per day, based on consumption of two servings per day. The medical food products for use in the invention are typically consumed at least once a day, preferably twice or three times a day. The medical food for use in this invention is for oral administration.

The dietary fiber composition comprising the fiber blend, or complex thereof, may be combined with any type of medical food product, including solid, liquid, or semi-solid medical food products. Exemplary solid medical food products include, grains (e.g., rice, cereal (hot or cold)), granola, oatmeal, baked goods (bread, cookies, muffins, cakes, and others), pasta (including noodles made with rice or other grains), meat (e.g., poultry, beef, lamb, pork, seafood), and dairy products (e.g., milk, yogurt, cheese, ice cream, and butter). Exemplary liquid or semi-liquid medical food products include, meal replacement drinks, fruit juices, soups (including dry soup mixes), dietary supplements, and smoothies.

The dietary fiber composition comprising the fiber blend or complex thereof may be added to the medical food product prior to consumption using any suitable method. For example, the dietary fiber composition may be baked into the medical food product, may be mixed with the medical food product, or sprinkled onto the medical food product.

The medical foods for use in the invention are packaged in unit doses, with a label clearly stating that the product is intended for use in the management of a specific metabolic disease or disorder, under the supervision of a physician.

**Methods for Preventing, Treating, or Ameliorating One or More Symptoms Associated With a Metabolic Disease or Disorder**

In another aspect, the present invention provides a medical food for use in a method for preventing, treating, or ameliorating one or more symptoms associated with a metabolic disease or disorder, such as metabolic syndrome, type I diabetes, type II diabetes, obesity, non-alcoholic steatohepatosis (fatty liver disease), pancreatic disease, and hyperlipidemia. The use according to this aspect of the invention comprises administering to a human subject in need thereof an effective dosage of a highly viscous polysaccharide dietary fiber composition comprising a viscous fiber blend (VFB) or complex thereof (VFC), comprising from about 48% to about 90% (w/w) glucomannan (such as from about 60% to about 80%, or from about 60% to about 90%, or from about 65% to about 75%, or from about 50% to about 80%, or from about 50% to about 70%, or about 70%), from about 5% to about 20% (w/w) xanthan gum (such as from about 10% to about 20%, or from about 11% to about 13%, or from about 13% to about 17%, or about 13%, or about 17%), and from about 5% to about 30% (w/w) alginate (such as from about 10% to about 20% or from about 13% to about 17%, or about 13%, or about 17%). In some embodiments, proportions of glucomannan, xanthan gum, and alginate in the fiber blend or complex thereof are about 70% glucomannan, from about 13% to about 17% xanthan, and from about 13% to about 17% alginate.

In some embodiments, the use comprises administering a dietary fiber composition comprising a viscous fiber blend (VFB) or complex thereof (VFC, such as, for example, granulated VFB), comprising from about 48% to about 90% (w/w) glucomannan, from about 5% to about 20% (w/w) xanthan gum, and from about 5% to about 30% (w/w) alginate to a human subject in need thereof at a dosage of from 1.0 g to 100 g VFB/C per day, such as from about 5 g to about 50 g VFB/C per day, such as from about 10 g to about 35 g VFB/C per day, from about 12 g to 35 g VFB/C per day, or such as from about 15 g to 35 g VFB/C per day, such as from about 20g to 35g VFB/C per day, such as from about 12g to about 25g VFB/C per day, such as from about 15g to about 25g VFB/C per day.

In some embodiments, the use comprises administering a dietary fiber blend (VFB) or complex thereof (VFC) to a mammalian subject, such as a human subject, in need thereof at a dosage of from about 25 mg/kg/day to about 1000 mg/kg/day, such as from about 50 mg/kg/day to about 600 mg/kg/day, such as from about 100 mg/kg/day to about 500 mg/kg/day, such as from about 200 mg/kg/day to about 400mg/kg/day, for a time period effective to prevent, treat or ameliorate one or more symptoms associated with the metabolic disease or disorder in the subject.

In some embodiments, the dietary fiber composition comprising a fiber blend (VFB) or complex thereof (VFC) is administered to the subject in the form of a medical food product, as described herein. In some embodiments, the dietary fiber composition is administered to a subject in need thereof at least once per day. In some embodiments, the dietary fiber composition of the invention is administered two times a day, preferably once in the morning and once in the afternoon/evening. A typical treatment regime in accordance with this aspect of the invention will continue from at least two weeks to 16 weeks or longer. Depending on such factors as the medical conditions being treated and the response in the patient, the treatment regime may be extended until the patient experiences amelioration of at least one symptom of the metabolic disease or disorder.

In one embodiment, the present invention provides a medical food for use in a method for ameliorating at least one symptom associated with the progression of insulin resistance in a human subject suffering from, or at risk for, developing type II diabetes. The use according to this aspect of the invention comprises administering to the human subject in need thereof from about 25 mg/kg/day to about 1000 mg/kg/day (e.g., from 100 mg/kg/day to 500 mg/kg/day, or from 350 mg/kg/day to about 450 mg/kg/day) of a highly viscous polysaccharide dietary fiber composition comprising a fiber blend or complex thereof (VFB/C), comprising from about 48% to about 90% (w/w) glucomannan, from about 5% to about 20% (w/w) xanthan gum, and from about 5% to about 30% (w/w) alginate for a time period effective to ameliorate at least one symptom of the progression of insulin resistance, such as a reduction in blood glucose levels. In some embodiments, the use comprises administering the dietary fiber composition for a time period of from at least two weeks up to 16 weeks or longer.

According to the American Heart Association and the National Heart, Lung, and Blood Institute, metabolic syndrome is diagnosed as being present if a subject has three or more of the following: blood pressure equal to or higher than 130/85 mmHg; blood sugar (glucose) equal to or higher than 100 mg/dL; large waist circumference (men: 40 inches or more; women: 35 inches or more); low HDL cholesterol (men: under 40 mg/dL; women: under 50 mg/dL); or triglycerides equal to or higher than 150 mg/dL. Therefore, in some embodiments, the medical food for use in a method for ameliorating at least one symptom associated with the progression of insulin resistance in a human subject suffering from, or at risk for, developing type II diabetes comprises administering to the subject an effective amount of VFB/C for a time period effective to (1) reduce the blood sugar (glucose) in the subject to a level below 100 mg/dL; (2) reduce the waist circumference to below 40 inches for a male subject, or below 35 inches for a female subject; and/or (3) reduce the level of triglycerides to a level equal to or less than 150mg/dL.

As described in Examples 1-4, the efficacy of VFC (e.g. granulated VFB) is demonstrated for ameliorating the development and progression of the early phase of metabolic syndrome in mammalian subjects, including the ability to slow the progression of glucose-induced organ damage, reduce lipid accumulation in the liver, preservation of pancreatic beta cells, and improved insulin sensitivity, as compared to the control group.

## Methods for Analyzing a Sample Comprising at Least One Polysaccharide

It is disclosed herein a method for determining the component sugars in a sample comprising at least one polysaccharide, such as a dietary fiber composition comprising a fiber blend, or complex thereof. The methods disclosed herein comprise: (a) hydrolyzing a sample comprising at least one polysaccharide with an acid to produce a hydrolysate; (b) separating the hydrolysis products in the hydrolysate with a chromatographic method; (c) detecting the hydrolysis products separated in step (b); and (d) comparing the hydrolysis products detected in step (c) to one or more reference standards to determine the component sugars in the sample.

It is disclosed herein, the sample comprises at least one dietary fiber. It is disclosed herein, the sample comprises sodium alginate. It is disclosed herein, the sample comprises a fiber blend or complex thereof, comprising alginate, glucomannan and xanthan gum.

## Hydrolysis

It is disclosed herein, the sample comprising at least one polysaccharide is hydrolyzed with an acid to product a hydrolysate. It is disclosed herein, the acid used to hydrolyze the sample is trifluoroacetic acid (TFA).

It is disclosed herein, the sample comprises alginate, a mixed polymer of mannuronic acid and guluronic acid. It is disclosed herein, the hydrolysis step of the sample comprising alginate is carried out under conditions suitable to provide for the release and preservation of L-guluronic acid as well as the D-mannuronic acid. For example, the initial hydrolysis of alginic acid can be effected with either 95% sulphuric acid at 3°C for 14 hours, or with 80% sulphuric acid at room temperature for 14 hours, as described by Fischer and Dorfel. In accordance with such embodiments, before stirring in the alginic acid, mineral acid is cooled to between -10 and -5°C. The viscous mass is stirred thoroughly to avoid formation of lumps. The mixture is then diluted with crushed ice and water until the sulphuric acid solution is about 0.5N. The solution is then heated for six hours on a boiling water bath, then neutralized with calcium carbonate. After filtration and washing of the calcium sulphate precipitate, the bright yellow filtrate wash water are concentrated, then passed through a cation-exchange column and concentrated under reduced pressure to a thin syrup. After further slow concentration in a dessicator and innoculation with D-mannofuranurono-lactone of melting point 191°C, some of the lactone crystallizes from the syrup, but only if the hydrolized alginic acid contained more D-mannuronic acid than L-guluronic acid. After removal of the crystalline D-mannuronolactone, the remaining D-mannuronolactone and L-guluronic acid are separated by chromatographic methods as described herein.

It is disclosed herein, the hydrolysis step of the sample comprising alginate comprises the use of trifluoroacetic acid (TFA). TFA has the advantage over mineral acids of being sufficiently volatile to allow for its removal simply by freeze-drying the hydrolysate. For example, hydrolysis in 2M TFA at 100°C under nitrogen for a time period of from about eight hours to about 18 hours has been shown to be a suitable alternative to hydrolysis in 1M $H_2SO_4$ under the same conditions. (Hough et al.). It is noted that a hydrolysis time of 6-8 hours typically suffices for degredation of polysaccharides composed of neutral sugars, however, the presence of uronic acid residues in appreciable proportion introduces the further difficulty that glycosiduronic acid linkages are, in general, much more resistant to acid hydrolysis than other glycosidic linkages. For polysaccharides such as the capsular polysaccharides of bacteria, containing uronic acid to the extent of approximately 16 to 30 % molar, hydrolysis in 2 M TFA at 100°C under nitrogen for 18 hours has been shown to be satisfactory in some cases (Hough et al.). However, where sugar residues particularly susceptible to degredation by acid (such as D-ribose, D-xylose, or L-rhamnose) are present, the time of hydrolysis is preferably limited to eight hours and subsequently correcting the analytical results for sugar remaining linked to uronic acid, the proportion of aldobiuronic acid in the hydrolysate being found by gel chromatography on a tightly cross-linked gel.

It is disclosed herein, the hydrolyzing step of a sample comprising alginate is carried out by incubating the sample with TFA for a time period of from about 48 to 72 hours at a temperature ranging from about 95°C to about 110°C. As described in Example 6, it was determined by the present inventors that hydrolysis with TFA for 72 hours was effective for hydrolytic release of the sugars from a sample comprising alginate, such as a VFB/C containing sample.

## Chromatographic separation of the hydrolysate

It is disclosed herein, the hydrolysis products in the hydrolysate are then separated with a chromatographic method, such as, for example, thin layer chromatography, gas chromatography (GLC), or liquid chromatography (LC), including the use of C18 reversed phase materials. In some embodiments, the chromatographic method is capable of separating neutral sugars from uronic acids, such as Dionex chromatography.

The hydrolysis products separated by the chromatographic method are detected and compared to one or more reference standards to determine the component sugars in the sample. Representative detectors suitable for detecting sugars include the Pulsed Amperometric Detector by Dionex, or an Evaporative Light Scattering Detector (ELSD) or mass spectrometer attached to an HPLC system. The reference standards, such as samples with known components, can be run as control samples in parallel with the test sample. Alternatively, the reference standard may be the known characteristics of one or more particular component sugars (e.g., retention time/height/relative area) in reference sample analyzed by a particular chromatographic method, as described in Example 5.

It is disclosed herein, the method comprises hydrolyzing a sample comprising at least one polysaccharide, such as alginate, with TFA; separating the hydrolysis products in the hydrolysate with a chromatographic method, such as an HPLC system with a C18 column; detecting the hydrolysis products with a detector, such as an ELSD or mass spectrometer; and comparing the detected products to one or more reference standards to determine the component sugars in the sample.

**EXAMPLE 1**

This example describes the effects of the dietary fiber composition comprising a granulated viscous fiber blend, (also referred to as the viscous fiber complex (VFC) commercially known as PolyGlycopleX (PGX®)) on Insulin Resistance, Body Weight, Pancreatic $\beta$-cell viability, and Lipid Profile in Zucker Diabetic Rats.

**Rationale:** The Male Zucker Diabetic Rat (ZDF) (ZDF/Cr1-Lepr$^{fa/fa}$) was chosen as the animal model for use in this study because this animal model is considered to be an excellent model of adult-onset obesity with co-morbid type II diabetes and/or reduced insulin sensitivity at earlier ages (C. Daubioul et al., J. Nutr. 132:967-973 (2002); J.M. Lenhard et al., Biochem. & Biophys. Res. Comm. 324:92-97 (2004); J.N. Wilson, Atheriosclerosis 4:147-153 (1984)). ZDFs are mutants that were found to lack brain leptin receptors. Leptin is a protein secreted by adipose tissue that signals appetite suppression. Therefore, in these mutant rats, there is no feedback signaling to reduce appetite or to induce satiety. ZDF rats consume food at very high rates and become obese very rapidly. This model therefore mimics people who are obese through overeating. As the ZDF rats become obese, they rapidly become insensitive to insulin, just as seen in man (also referred to as metabolic syndrome). The ZDF rats are also hyperlipidemic, showing this rat model to be a good model for metabolic syndrome in humans. Over time, the diabetes progresses in the ZDF model, similar to the progression in humans, becoming florid with loss of pancreatic $\beta$ cell (insulin secreting cells) population. Proteins become glycated by the excess glucose, causing problems in both ZDFs and man with organ function, particularly in the kidneys. High glucose levels cause glycation of proteins, causing diabetic nephropathy and vascular damage. Early ages of ZDFs (five weeks old) were used in this study without high fat feeding in order to determine if the administration of Viscous Fiber Complex (VFC) granules could delay the onset and/or reduce the severity of diabetes.

The standard marker of the degree of glucose damage to proteins is glycated hemoglobin (HbA1c), which is elevated in ZDFs, is now one of the most important markers for drug approval in man. Measurement of albumin in the urine is also a standard marker of diabetic injury to the kidney. The FDA guidelines for treatment of diabetes require glycemic control and reduction of tissue damage caused by high glucose.

**Methods**

***Fiber Enhanced Rat Chow***

Viscous fiber complex (VFC) (konjac/xanthan/alginate (70:13:17) granules (i.e., the fiber blend was processed by granulation to form a complex, commercially known as PGX®) was incorporated into basic rat chow (D11725: Research Diets, New Brunswick, New Jersey). Alternate diets used in this study incorporated other fiber forms, as shown below in TABLE 1. All diets were formulated to be as isoenergetic as possible given the different energy contribution of each fiber source (VFC and inulin diets provided 3.98 kcal/g and cellulose provided 3.90 kcal/g).

Cellulose was selected as the basic reference fiber that is insoluble and is non-fermentable and is considered to be an inert reference compound (J.W. Anderson et al., J. Nutr. 124:78-83 (1994). Inulin is plant-derived fructose polymer that is water soluble and non-digestible and has shown efficacy in some studies with respect to lipid reduction and glycemic control in some studies; but the results are variable (see P. Rozan et al., Br. J. Nutr. 99:1-8 (2008). The number of fructose or glucose units (degree of polymerization "DP") of the inulin was 99.9% $\geq 5$, with the average DP being $\geq 23$.

TABLE 1: Composition of the Three Diets Containing Either VFB, Cellulose, or Inulin (Percent Contribution of Ingredients by Weight)

| | Viscous Fiber Complex (VFC) (Konjac/Xanthan/Alginate(70:13:17)) PGX® Granules | Insoluble Fiber (Cellulose) | Soluble, Non-Viscous Fiber (Inulin) |
|---|---|---|---|
| Research Diets Formula # | D08012504 | D08012507 | D08012503 |
| Casein | 20% | 20% | 20% |
| Methionine | 0.3% | 0.3% | 0.3% |
| Corn Starch | 50% | 50% | 50% |
| Maltodextrin | 15% | 15% | 15% |
| Fiber* | 5% VFC (PGX®) | 5% cellulose | 5% inulin |
| Corn oil | 5% | 5% | 5% |
| Salt/mineral mix | 3.5% | 3.5% | 3.5% |
| Vitamin mix | 1% | 1% | 1% |
| Choline bitartrate | 0.2% | 0.2% | 0.2% |
| Dye | 0.1% | 0.1% | 0.1% |
| *VFC fiber granules commercially known as PolyGlycopleX® (PGX®) (InnovoBiologic Inc., Calgary, Alberta, Canada), Cellulose (Research Diets, New Brunswick, New Jersey), and Inulin (Raftiline® HP, Orafti, Tienen, Belgium), respectively. | | | |

**Study Design**

Thirty (30) male ZDF/Crl-Lepr[fa/fa] rats were obtained from Charles River (Kingston, New York) at five weeks of age. The animals were housed singly in suspended wire mesh cages that conformed to the size recommended in the most recent *Guide for the Care and Use of Laboratory Animals,* DHEW (NIH). All studies were approved by the Eurofins Institutional Animal Use and Care Committee. The animal room was temperature and humidity controlled, had a 12-hour light/dark cycle, and was kept clean and vermin free. The animals were conditioned for one week after arrival, and the animals had access to food and water *ad libitum.*

After habituation, rats were randomly assigned to one of three groups on the basis of initial blood glucose and body weight. Each group of rats was given one type of chow containing either VFC (commercially known as PGX®), cellulose, or inulin (Raftiline® HP, a chicory-derived inulin), all at 5% (wt/wt), as shown above in TABLE 1, for a time period of 8 weeks. Basic monitoring procedures were conducted throughout the 8-week study, including thrice-weekly measurement of food weight, weekly measurement of body weight, and collection of blood samples for glucose and insulin. It is noted that the non-fasted analysis of glucose was started at week 3, while the fasted analysis was started at week 1. In the non-fasted animals, insulin was only measured at the last time point. The analysis of the non-fasted state was added to the study due to the observation that greater effects of VFC on stabilizing glucose levels were observed while the fiber was physically present in the gastrointestinal tract, likely due to the fact that the Zucker rats eat continuously both day and night. In fasted animals, serum triglycerides were measured throughout the study, while in non-fasted animals, only a terminal measurement was taken (IDEXX, North Grafton, Massachusetts).

For all studies, the blood samples used for glucose and insulin were taken at approximately the same time of the day (mid-morning). The study was concluded with two oral glucose tolerance tests, separated by a week, and a necropsy.

**Measurements**

The following measurements were taken throughout the 8-week study:

**Food Intake:** Before and after introduction of experimental food chow, food weight was measured 3 times a week.
**Body weight** was measured once a week.

*Blood Glucose and Insulin:* Before and at weekly intervals after introduction of experimental chow, blood was collected via retroorbital bleed after an overnight fast. Blood samples were taken once a week for glucose and insulin at approximately the same time of day (mid-morning). A small quantity was analyzed with a handheld glucometer. After removing a sample for insulin analysis, 1 mL was allowed to clot; 0.5 mL of serum was removed and analyzed for triglyceride content. Additional samples were collected via a tail nick when the animals had access to food. Blood glucose was measured using a Bayer Ascensia Elite Glucometer (Bayer Health Care, Tarrytown, New York). Insulin was measured using an ELISA (Ani Lytics, Gaithersburg, Maryland).

### Oral Glucose Tolerance Tests (OGTTs)

At week 9 and at week 10, the study was concluded with two oral glucose tolerance tests (OGTTs) in fasted and non-fasted rats, with the non-fasted OGTT done last. For both fasted and non-fasted OGTTs, a baseline blood sample for insulin analysis and glucose measurement was taken. The initial blood sample for the final non-fasted OGTT was also used for a clinical chemistry panel as described below.

The OGTT for both fasted and non-fasted animals was induced by oral glucose treatment (2 g/kg glucose, by gavage). Blood samples were taken at 30, 60, 90, and 120 minutes after the glucose load and were analyzed for glucose and insulin content. At the conclusion of the second glucose tolerance test, the rats were sacrificed by isofluorane overdose, and the relevant organs were harvested for histopathological analysis.

*Homeostatis Model Assessment (HOMA)* scores were calculated throughout the study as mg glucose x insulin (U/mL$^2$). This is generally accepted as a reliable method of showing changes in insulin resistance, with lower HOMA scores representing greater reductions in peripheral insulin resistance. Composite insulin sensitivity index (CISI) scores for the oral glucose tolerance test (OGTT) studies were also calculated using the following formula:

$$CISI = \frac{1000}{\sqrt{\left(Gluc_{base} x\ Ins_{base}\right) x \left(Gluc_{mean} x\ Ins_{mean}\right)}}$$

This CISI score takes into account glucose excursion and area under the curve with a higher score showing improved insulin sensitivity.

For both fasted and non-fasted OGTTs, a baseline blood sample for insulin analysis and glucose measurement was taken. The initial blood sample for the final (non-fasted) OGTT was also used for a clinical chemistry panel including: electrolytes, blood urea nitrogen (BUN), creatinine, alkaline phosphatase, aspartate aminotransferase (ALT), alanine aminotransferase (AST), and bilirubin (direct + indirect) and total plasma cholesterol (Analysis done by IDEXX, North Grafton, Massachusetts).

*Serum Triglycerides:* In fasted animals, serum triglycerides were measured throughout the study, while in non-fasted animals, only a terminal measurement was taken (Analysis done by IDEXX, North Grafton, Massachusetts).

*Clinical Chemistry Panel:* The initial blood sample for the final non-fasted OGTT was used for a clinical chemistry panel including electrolytes, blood urea nitrogen (BUN), creatinine, alkaline phosphatase, aspartate aminotransferase (ALT), alanine aminotransferase (AST), and bilirubin (direct + indirect) and total plasma cholesterol (Analysis done by IDEXX, North Grafton, Massachusetts).

*Tissue Analysis:* One lobe of the liver, one kidney, and the pancreas were fixed in 10% neutral buffered formalin (NBF). The pancreas was transferred to 70% ethanol after 24 hours. Tissues were processed and embedded in paraffin. The liver and kidney were sectioned at approximately 5 microns and stained with hematoxylin and eosin (H&E). The pancreas was serially sectioned twice at approximately 5 microns, and the sections were either stained with H&E or immunohistochemically stained with a mouse antibody against rat insulin (1:300 rabbit anti-rat insulin, Cell Signaling Technology, Danvers, Massachusetts).

### Immunohistochemistry:

Immunohistochemistry was performed as follows. An isotype control antibody (normal rabbit IgG, R&D Systems, Minneapolis, Minnesota) was used to assess the overall level of non-specific and background staining. Following deparaffinization, antigen retrieval was performed using Declere® solution (Cell Marque™ Corporation, Rocklin, California) for 15 minutes at 120°C, followed by 5 minutes room temperature in hot Declere® solution. Endogenous peroxidase activity was quenched by incubation in 3% hydrogen peroxide in deionized water for 10 minutes. Slides were incubated for 20 minutes in 5% normal goat serum. The slides were then incubated with the primary antibody for 60 minutes, followed by incubation for 30 minutes in biotinylated goat anti-rabbit antibody. The slides were then incubated in ABC Elite Reagent® (Vector, Burlingame, California) for 30 minutes. Finally, specimens were incubated in diaminobenzidine for 5 minutes, followed by hematoxylin counterstaining.

Following necropsy, an additional liver lobe was snap-frozen, embedded in OCT and sectioned at 5 $\mu$M and stained with Sudan black for analysis of lipid content (free fatty acids and triglycerides).

All slides stained with H&E were evaluated for morphologic changes related to those commonly observed in ZDFs, such as an increase in tubular dilation and an increase in tubular degeneration in the kidney, pancreatic islet cell degeneration, and hepatic steatosis. These changes were graded semi-quantitatively on a scale of 0 to 5 based upon the severity of that finding, with 5 being the most severe.

The liver slides stained with Sudan black were evaluated and graded semi-quantitatively for the presence of Sudan black positive vacuoles on a scale of 0 to 5, with 5 being the most severe.

The percent of the islet area with insulin positive cells was measured on the pancreas slides immunohistochemically stained with anti-insulin antibody. This measurement was performed morphometrically. Ten islets per pancreas were manually outlined by a veterinary pathologist. Areas positive for insulin staining within these islets were similarly outlined, and the percent of islet areas positive for insulin was calculated using ImagePro®Plus imaging software.

### *Statistical Methods:*

Interval data collected at multiple times was analyzed by two-way repeated measures analysis of variance (ANOVA). Significant effects were followed by *post hoc* comparison using Bonferroni's multiple comparisons test, as described in Motulsky H., Intuitive Biostatistics, NY, University Press (1995).

Insulin, cholesterol, and blood chemistries measured only at the end of the study in non-fasted rats were analyzed by one-way ANOVA. Significant effects were followed by *post hoc* comparisons using Dunnets' multiple comparisons test (MCT), as described in Motulsky H., Intuitive Biostatistics, NY, University Press (1995). Non-interval or discrete data (e.g., histology scores) were analyzed by the Kruskal Wallis test as described in Motulsky H., Intuitive Biostatistics, NY, University Press (1995). Significant effects were followed by *post hoc* comparisons using Dunnets' MCT.

### Results

### *Body Weight and Food Consumption*

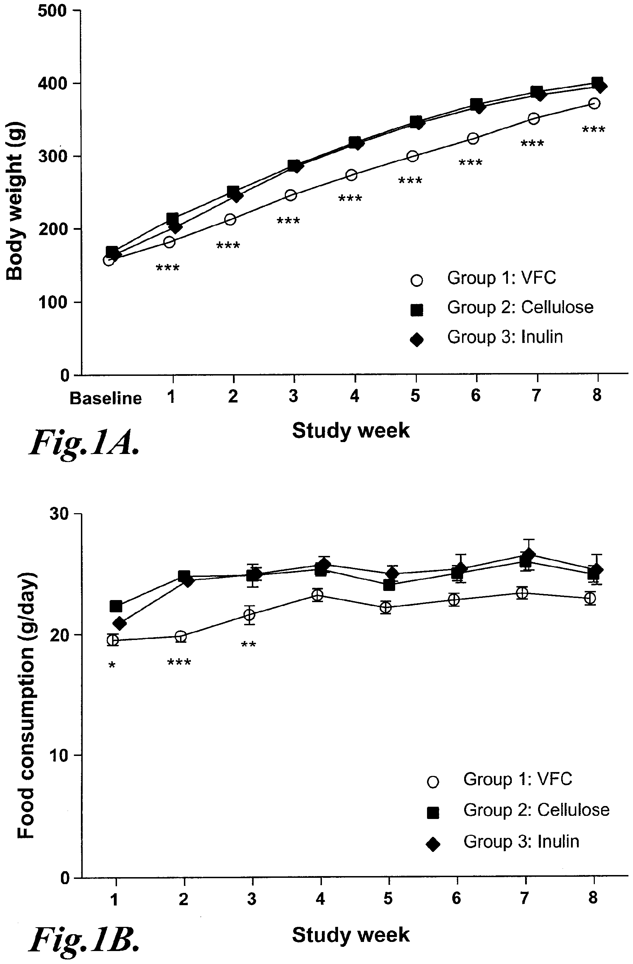

FIGURE 1A graphically illustrates the effect of VFC, cellulose, or inulin diets on body weight (g) over time during the 8-week study in Zucker diabetic rats. As shown in FIGURE 1A, the increase in body weight with respect to time was significantly obtunded in the VFC-treated rats versus cellulose-fed animals or inulin-fed animals from week 1 on. At the start of the study, all Zucker diabetic rats had similar body weights (approximately 160 g). Over the next three weeks, rats fed cellulose or inulin-containing chow gained approximately 40 g more on average than rats fed VFC containing chow. Significant differences between rats fed VFC versus cellulose- or inulin-containing diets were observed from week 1 to week 8 (The symbol "***" indicates p <0.001, Bonferroni's MCT). No significant differences were observed between rats fed inulin and cellulose containing diets.

FIGURE 1B graphically illustrates the effect of VFC, cellulose, or inulin diets on food consumption (g/day) over time during the 8-week study in Zucker diabetic rats. As shown in FIGURE 1B, food consumption was significantly reduced in VFC-treated rats for the first three weeks (the symbol "*" indicates p <0.05 at week 1; the symbol "***" indicates p <0.001 at week 2; and the symbol "**" indicates p <0.01 at week 3). Food intake in the VFC group remained lower throughout the remainder of the protocol, although after 4 weeks into the study, the levels were no longer statistically different from the other two groups. No significant differences were observed between rats fed inulin- and cellulose-containing diets.

Rats fed VFC-containing chow typically ate 20-23 g/day (corrected for spillage; equivalent to approximately 70-85 kcal/day). Rats fed cellulose or inulin-containing chow typically ate 21-27 g/day (corrected for spillage; equivalent to approximately 75-100 kcal/day).

In summary, these results demonstrate that increase in body weight with respect to time typically observed in the ZDF rat model was significantly obtunded in the VFC-treated animals.

### *Glycemic Control: Blood Sugar and Metabolism*

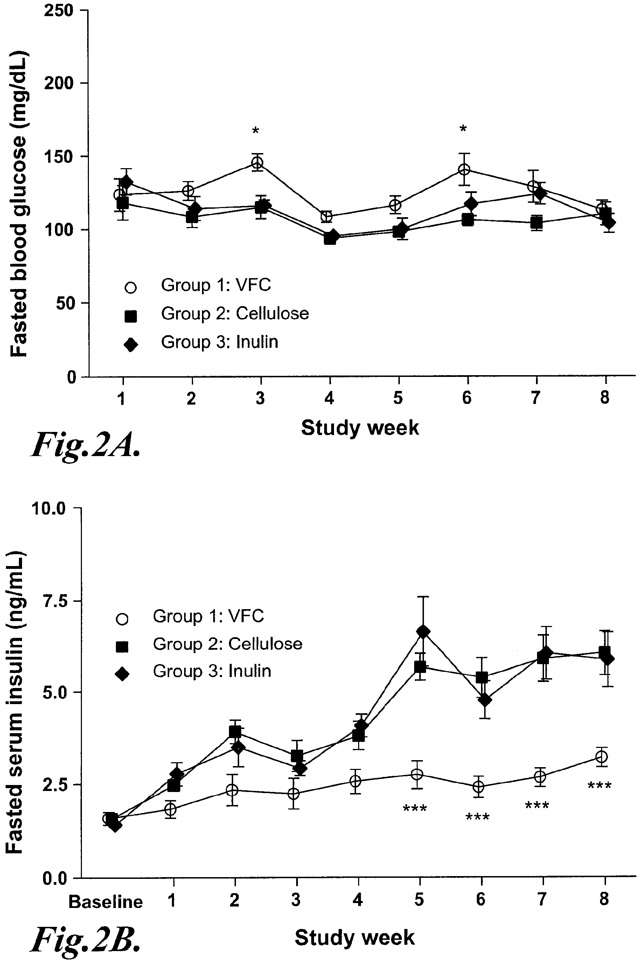

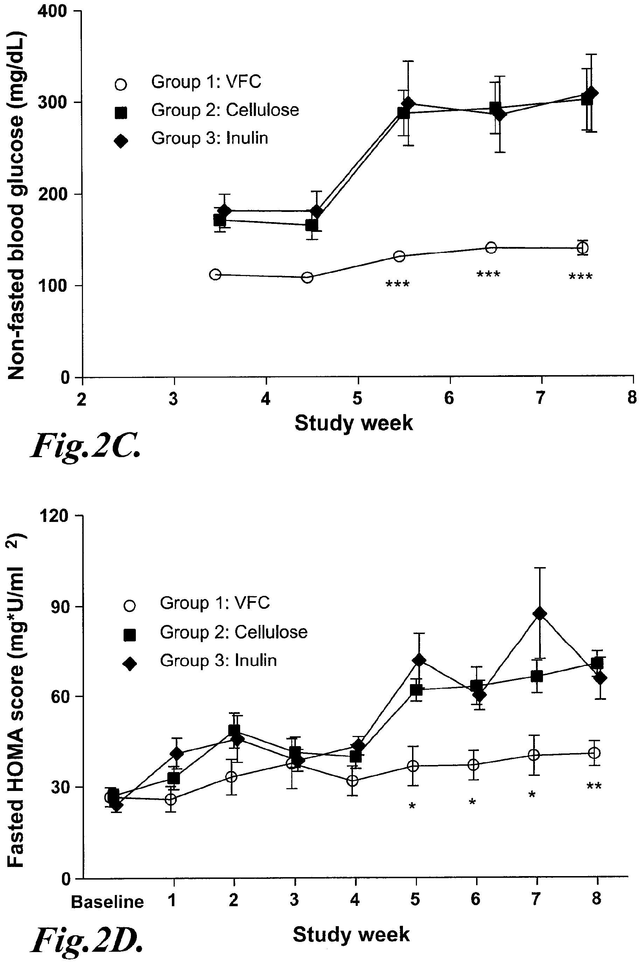

FIGURES 2A-D graphically illustrate the effect of VFC-, cellulose-, or inulin-containing diets on fasted blood glucose (FIGURE 2A), fasted serum insulin (FIGURE 2B), non-fasted blood glucose (FIGURE 2C), and fasted Homeostatis Model Assessment (HOMA) scores (FIGURE 2D) in ZDF rats over time during the 8-week study. As shown in FIGURE 2A, the blood glucose values in the fasted rats were not greatly elevated in any of the rats, with slight increases observed in glucose values for the VFC-treated rats (the symbol "*" indicates p <0.05 at weeks 3 and 6).

As shown in FIGURE 2B, the serum insulin levels in fasted rats was reduced in the VFC-treated rats throughout the study period, and the serum insulin levels were reduced at statistically significantly levels starting at five weeks (the symbol "***" indicates p <0.001 after week 4).

As shown in FIGURE 2C, the blood glucose values in the non-fasted rats were significantly reduced in VFC-treated rats starting at approximately five weeks (the symbol "***" indicates p <0.0001 after week 5) as compared to the cellulose- and inulin-fed rats.

As shown in FIGURE 2D, VFC-treated rats had significantly reduced HOMA scores starting at five weeks into the study (the symbol "*" indicates p <0.05), with weeks 5-7 (p <0.05), and week 8 (the symbol "**" indicates p <0.01).

Generally, under fasted conditions (i.e., animals tested in the morning after approximately 16 hours without food access), the ZDF rats maintained much lower blood glucose concentrations than those seen under food-replete (non-fasted) conditions (compare FIGURE 2A to FIGURE 2C). As shown in FIGURE 1A, for all fasted groups, blood glucose values were typically observed in the range between 95 mg/dL and 145 mg/dL, which is considered marginally diabetic, with little differences observed between the VFC-, cellulose-, or inulin-fed groups.

As shown in FIGURE 2B, under fasted conditions, rats fed a VFC-containing diet maintained much more stable serum insulin concentrations than rats fed cellulose- or inulin-containing chow. As shown in FIGURE 2B, fasted serum insulin levels were reduced in VFC-treated ZDF rats throughout the time course of the study, with significant reductions observed starting at five weeks and remained significant through week 8 (p <0.001 after week 4, as indicated by the symbol "***"). No significant differences were observed between rats fed inulin and cellulose-containing diets.

Insulin resistance during the course of this study in fasted rats was assessed by calculating homeostasis model assessment (HOMA). As shown in FIGURE 2D, HOMA scores rose over the course of the study for all groups, but significantly less so for rats fed a VFC-containing diet than for rats fed cellulose or inulin. Significant differences between VFC versus cellulose or inulin were seen at 5, 6, and 7 weeks (p <0.05, as indicated by the symbol '*") and at 8 weeks (p <0.01, as indicated by the symbol "**"). No significant differences were observed between rats fed cellulose- or inulin-containing diets.

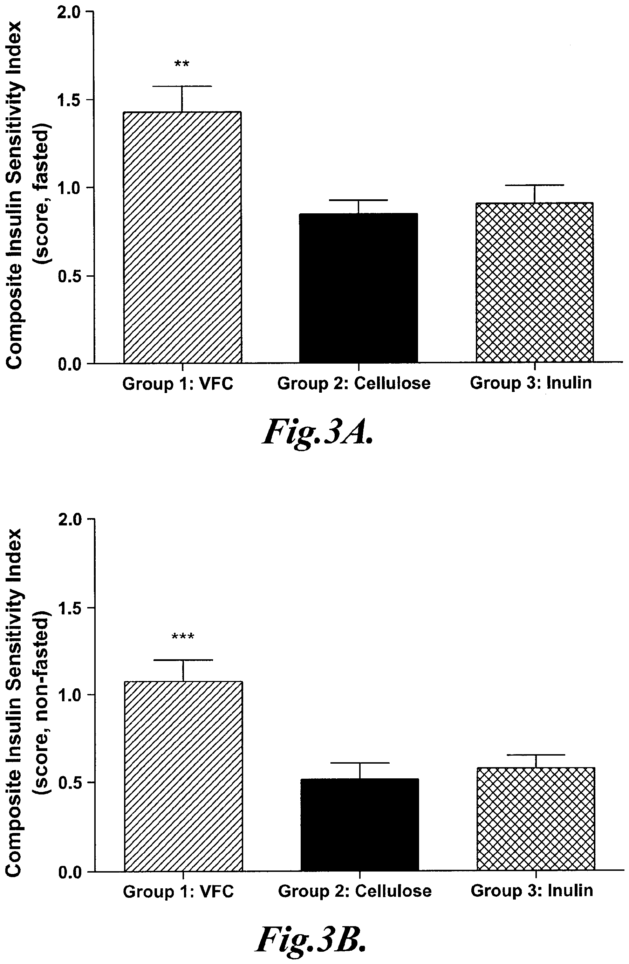

FIGURE 3A graphically illustrates the composite insulin sensitivity index (CISI) scores for fasted Zucker diabetic rats fed either VFC, cellulose, or inulin diets during the 8-week study. As shown in FIGURE 3A, CISI scores calculated for the OGTT test in fasted animals were significantly higher (p <0.01, indicated by the symbol "**") for VFC-treated animals, further demonstrating improved insulin sensitivity for this VFC group as compared to the cellulose- and inulin-fed groups.

FIGURE 3B graphically illustrates the composite insulin sensitivity index (CISI) scores for non-fasted Zucker diabetic rats fed either VFC, cellulose, or inulin diets during the 8-week study. As shown in FIGURE 3B, the VFC-treated, non-fasted animals showed a significantly higher CISI score (p <0.001, indicated by the symbol "***"), therefore higher insulin sensitivity, as compared to the cellulose- and inulin-treated groups. Peak glucose levels were seen at 30 minutes post-glucose challenge, and the VFC group had a significantly lower peak value compared to the other two groups.

As shown in FIGURE 2C, under non-fasted (fed) conditions (i.e., animals tested in the morning with continuous food access during the previous 24 hours), rats fed a VFC-containing diet maintained lower blood glucose levels than rats fed cellulose- or inulin-containing diets during all weeks tested. Blood glucose testing began during the third week of the study and continued until the eighth week. Glucose testing of the animals in the fed state was added to the study protocol given the observation that fasted glucose values were very close to the normal range and, while not wishing to be bound by any particular theory, it is believed that many of the mechanistic actions of VFC involve its direct contact with food.

Although under the fed conditions insulin was only measured at the last time point, an improved insulin sensitivity was observed similar to that seen in the fasted animals when measured at the final time point. As shown in FIGURE 2C, fed state blood glucose response was significantly lowered in VFC treated animals (p <0.001, as indicated by the symbol "***"), as compared to inulin or cellulose treated animals. No significant differences were observed between rats fed inulin- or cellulose-containing diets.

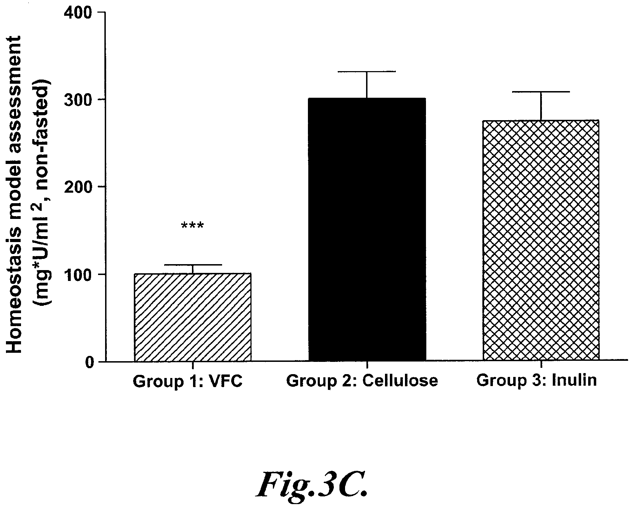

FIGURE 3C graphically illustrates the HOMA scores calculated for non-fasted Zucker diabetic rats fed either VFC, cellulose, or inulin diets for the final blood draw of the 8-week study. As shown in FIGURE 3C, the HOMA score was found to be significantly lower in the VFC treated group (p <0.001) as compared to the cellulose and inulin groups. As noted above, lower HOMA scores represent greater reductions in peripheral insulin resistance.

### *Lipid Profile*

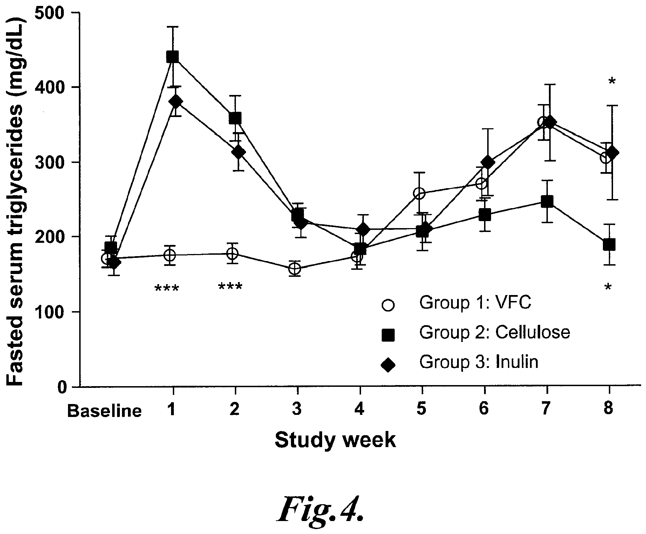

Serum triglycerides were measured in the fasted (measured throughout the study) and non-fasted (measured only at the end of the 8-week study) animals. FIGURE 4 graphically illustrates the level of serum triglycerides measured in fasted Zucker diabetic rats fed either VFC, cellulose, or inulin diets over time during the 8-week study. As shown in FIGURE 4, for the fasted animals, VFC-treated animals showed an early and significant lowering effect on triglycerides as compared to the inulin- and cellulose-treated groups. After 2-3 weeks, serum triglycerides were lowered in all groups, with a trend for cellulose-treated animals having somewhat lower triglycerides as compared to inulin- and VFC-treated animals. As shown below in TABLE 2, in non-fasted animals, as measured at the end of the study, serum triglycerides were similar for VFC-treated and cellulose-treated animals, with inulin-treated animals found to have significantly lower triglyceride levels than the other two groups.

At the end of the 8-week study, plasma cholesterol was measured in the baseline sample obtained from the fed animals before the last OGTT. As shown below in TABLE 2, the animals were hypercholesterolemic, and VFC significantly reduced cholesterol levels by more than half as compared to cellulose- and inulin-fed groups.

### *Target Organ Effects: Histological Evaluation of Liver, Pancreas and Kidneys*

While the data described above for glucose and insulin show improved insulin sensitivity and glycemic control with VFC treatment, tissue analysis was carried out to assess the effect of VFC on ameliorating the degree of damage to organs such as the kidney. The kidney in particular is known to be sensitive to diabetic nephropathy, which is likely related to hyperglycemia and excessive glycation. For all tissues measured, the degree of damage was assessed as an indicator of the ability of VFC treatment to delay the progression of diabetes and/or ameliorate the symptoms associated with diabetes.

### *Kidney*

All slides of diabetic kidney tissue stained with H&E were evaluated for morphologic changes related to those commonly observed in ZDFs, including an increase in tubular dilation and an increase in tubular degeneration/regeneration. Several renal pathology parameters showed differences between Zucker diabetic rats (ZDF) rats fed a VFC-containing diet and ZDF rats fed inulin- or cellulose-containing chow.

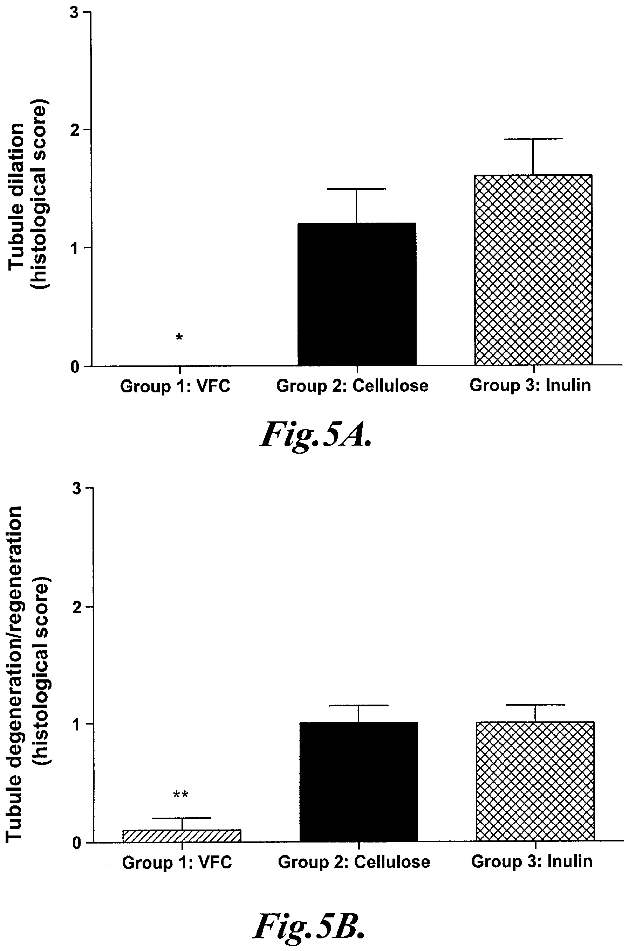

FIGURE 5A graphically illustrates the effect of VFC-, cellulose-, or inulin-containing diets on Zucker diabetic rats after eight weeks on renal tubule dilation, based on a histologic score of 0-5, with 5 being the most severe. As shown in FIGURE 5A, tubule dilation was scored as being absent in VFC-treated ZDF rats. In contrast, tubule dilation was found to be present in ZDF rats fed cellulose and inulin. The scores shown in FIGURE 5A showed a significant treatment effect (p <0.001, indicated by the symbol "*") between the groups fed VFC and cellulose or inulin. No significant difference was observed in the amount of tubule dilation between the animals fed inulin and cellulose.

FIGURE 5B graphically illustrates the effect of VFC-, cellulose-, or inulin-containing diets on Zucker diabetic rats after eight weeks on renal tubule degeneration/regeneration, based on a histologic score of 0-5, with 5 being the most severe. As shown in FIGURE 5B, rats fed a VFC-containing diet showed an average tubule degeneration/regeneration score of 0.1, which score consists of a score of 1 (minimal severity) in one rat, and a score of 0 (within normal limits) for the other 9 rats in the group. In contrast, the average score for rats fed with cellulose- or inulin-containing diets was 1.0, as further shown in FIGURE 5B. The treatment effect of VFC on reducing the severity of tubule degeneration/regeneration was significant (p <0.01, indicated by the symbol "**"), with a significant difference observed between groups fed VFC versus cellulose or inulin. There was no significant difference observed between cellulose or inulin.

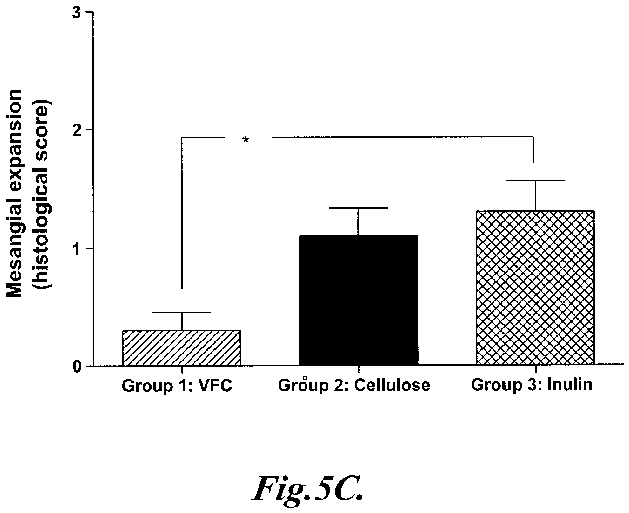

FIGURE 5C graphically illustrates the effect of VFC-, cellulose-, or inulin-containing diets on Zucker diabetic rats after 8 weeks on renal mesangial expansion, based on a histologic score of 0-5, with 5 being the most severe. As shown in FIGURE 5C, the glomerular mesangial expansion scoring showed lower scores for the group receiving the VFC-containing diet as compared to the cellulose- or inulin-containing diets. Although the scores for mesangial expansion reached overall statistical significance (p <0.05), the only pair of treatment groups that differed significantly on *post hoc* testing were the groups fed VFC and inulin containing diets (p <0.05, indicated by the symbol "*"), with a strong tendency to be reduced as compared to the cellulose diet.

### *Pancreas*

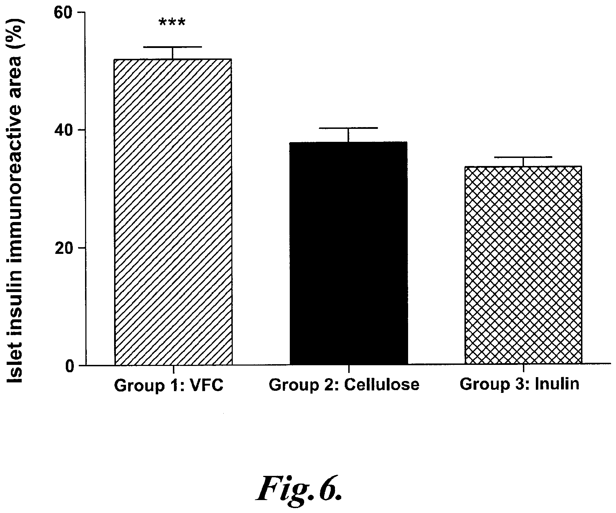

FIGURE 6 graphically illustrates the percentage of pancreatic islet insulin immunoreactive area present in Zucker diabetic rats fed either VFC, cellulose, or inulin diets at the end of the 8-week study, as determined by staining with anti-rat insulin antibody. As shown in FIGURE 6, rats fed a VFC-containing diet maintained a higher area of insulin immunoreactivity as a percent of total islet area (i.e., a larger pancreatic beta cell mass), as measured by insulin immunohistochemistry, as compared to rats fed cellulose- or inulin-containing diets. ANOVA analysis showed a significant treatment effect (p <0.0001, indicated by the symbol "***"), while *post hoc* testing showed differences between rats fed VFC- and cellulose-containing diets (p <0.001). No differences were seen between the animals fed inulin- and cellulose-containing diets. Importantly, these data combined with data showing lower fasting serum insulin concentrations (FIGURE 2B) and greater insulin sensitivity (FIGURES 3B), indicate that Zucker diabetic rats fed a VFC-containing diet maintain a significantly greater reserve capacity for insulin secretion in comparison to rats fed a cellulose- or inulin-containing diet.

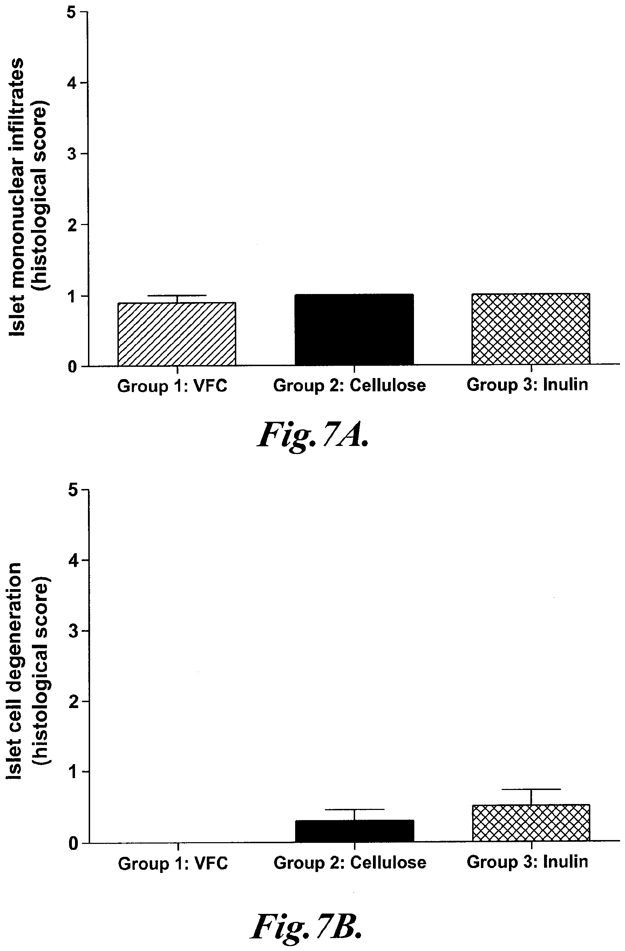

FIGURE 7A graphically illustrates the histological score for pancreatic islet mononuclear inflammatory cell infiltrates present in Zucker diabetic rats fed either VFC, cellulose, or inulin diets at the end of the 8-week study, based on a histologic score of 0-5, with 5 being the most severe. As shown in FIGURE 7A, there was no difference in treatment effect observed on the scores for the presence of islet mononuclear infiltrates.

FIGURE 7B graphically illustrates the histological score for pancreatic islet cell degeneration present in Zucker diabetic rats fed either VFC, cellulose, or inulin diets at the end of the 8-week study, based on a histologic score of 0-5, with 5 being the most severe. As shown in FIGURE 7B, scores for the degeneration of islet cells were absent in rats fed a VFC-containing diet, and tended to be higher in rats fed cellulose- or inulin-containing diets; however, these differences did not reach statistical significance.

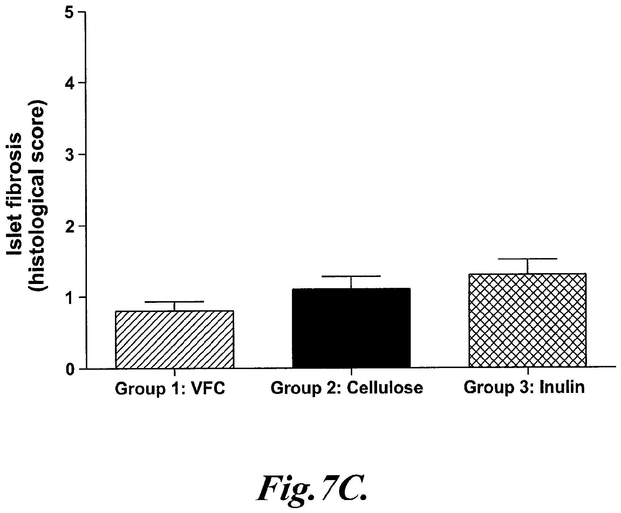

FIGURE 7C graphically illustrates the histological score for the amount of pancreatic islet fibrosis present in Zucker diabetic rats fed either VFC, cellulose, or inulin diets at the end of the 8-week study, based on a histologic score of 0-5, with 5 being the most severe. As shown in FIGURE 7C, scores for the amount of islet fibrosis tended to be lower in rats fed a VFC-containing diet compared to rats fed cellulose- or inulin-containing diets; however, these differences did not reach statistical significance. Scores for the presence of hemorrhage or hemosiderin revealed a trend for lower scores in rats fed a VFC-containing diet, but the results were not statistically significant (data not shown).

*Liver*

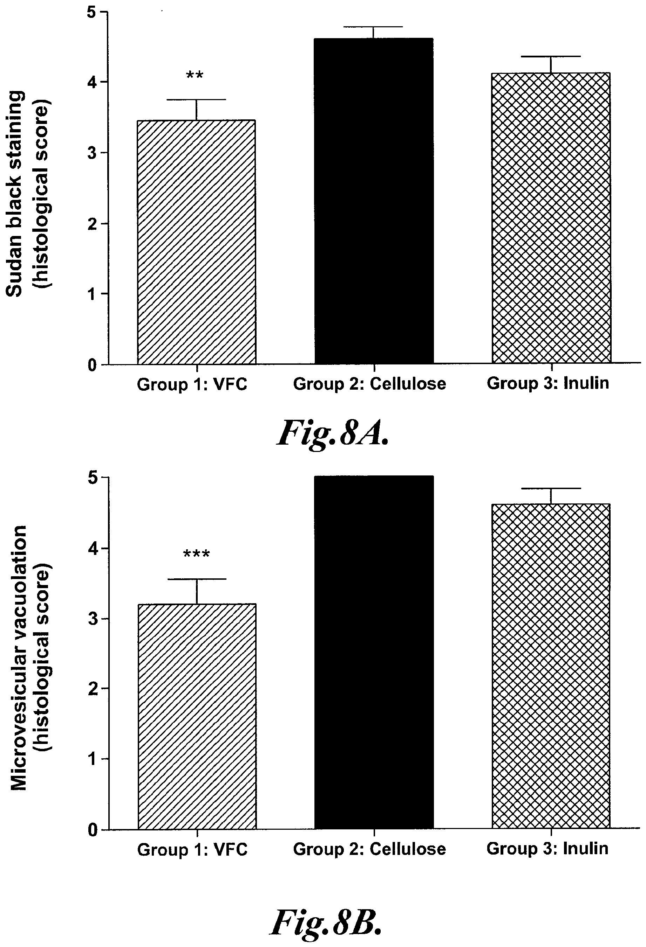

FIGURE 8A graphically illustrates the effect of VFC-, cellulose-, or inulin-containing diets on Zucker diabetic rats after eight weeks on hepatic steatosis, as measured by reduced Sudan black staining, based on a histologic score of 0-5, with 5 being the most severe. As shown in FIGURE 8A, rats fed a VFC-containing diet showed less hepatic steatosis (measured by Sudan black staining) than rats fed cellulose- or inulin-containing diets. On a scale of 0 (within normal limits) to 5 (severe), rats fed a VFC-containing diet averaged 3.4. This compares with a score of 4.6 for rats fed a cellulose-containing diet and 4.1 for rats fed an inulin-containing diet. The groups differed significantly between rats fed VFC- versus cellulose- and inulin-containing diets ($p < 0.01$, as indicated by the symbol "**"). No significant differences were observed between rats fed inulin- and cellulose containing diets.

Rats fed a VFC-containing diet also showed less hepatocyte microvesicular vaculoation than rats fed cellulose- or inulin-containing diets. FIGURE 8B graphically illustrates the effect of VFC-, cellulose-, or inulin-containing diets on Zucker diabetic rats after 8 weeks on hepatic microvesicular vacuolation, based on a histologic score of 0-5, with 5 being the most severe. As shown in FIGURE 8B, microvesicular vacuolation was scored as severe in all rats fed a cellulose- or inulin-containing diet (average score of 4.6, high). In contrast, rats fed a VFC-containing diet averaged a score of 3.2 (mild). Dunnets' MCT showed a significant difference between groups fed VFC-containing and cellulose-containing diets ($p < 0.001$, as indicated by the symbol "**"), but not between groups fed inulin- and cellulose-containing diets.

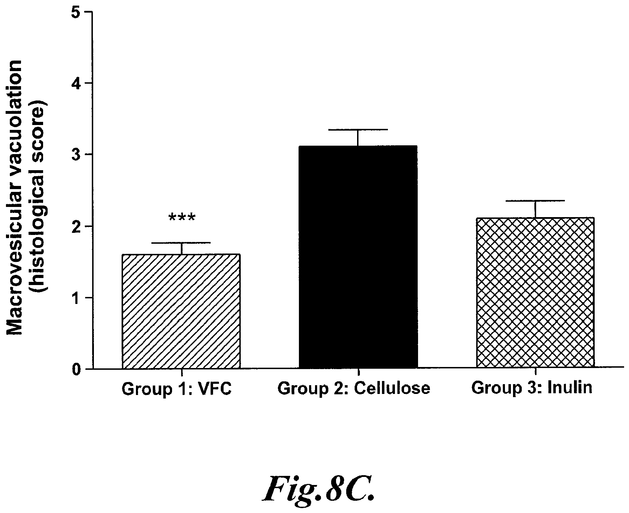

FIGURE 8C graphically illustrates the effect of VFC-, cellulose-, or inulin-containing diets on Zucker diabetic rats after eight weeks on hepatic macrovesicular vacuolation, based on a histologic score of 0-5, with 5 being the most severe. As shown in FIGURE 8C, in all treatment groups, macrovesicular hepatocyte vacuolation was generally less prominent than microvesicular hepatocyte vacuolation, as reflected in lower severity scores (compare FIGURE 8B to 8C). While rats given an inulin-containing diet showed a tendency to have reduced vacuolation as compared to rats given a cellulose-containing diet, this difference was not statistically significant. There was a significant difference between groups fed VFC- versus cellulose-containing and inulin-containing diets ($p < 0.001$, as indicated by the symbol "***"). No significant differences were seen between groups fed inulin- and cellulose-containing diets. Cystic hepatocyte degeneration and fibrosis also showed a trend toward less severe scores in rats receiving VFC-containing diet, but this did not reach statistical significance (data not shown).

As shown below in TABLE 2, several clinical chemistry indicators for hepatic damage showed substantial treatment effects with VFC. The hepatic enzymes alanine aminotransferase (ALT) and aspartate aminotransferase (AST) are released into the blood by hepatocellular injury, even with intact cell membranes. Sprague-Dawley rats with no overt liver disease range have ALT levels from 22-48 IU/L (IDEXX reference data). The data shown in TABLE 2 showed overall treatment effects with regard to ALT and AST levels. *Post hoc* testing showed lower blood ALT levels in rats receiving VFC-containing diet compared to rats receiving cellulose- or inulin-containing diets ($p < 0.05$), and significantly higher blood ALT levels in rats receiving inulin-containing diets as compared to rats receiving a cellulose-containing diet ($p < 0.05$).

Blood AST showed a similar pattern of results as further shown in TABLE 2. Sprague-Dawley rats with no overt liver disease range have AST levels from 33-53 IU/L (IDEXX reference data). Rats receiving VFC-containing diet averaged approximately 170 IU/L, rats receiving cellulose-containing diet averaged 870 IU/L, while rats receiving inulin containing diets averaged 1010 IU/L. The overall treatment effect was statistically significant ($p < 0.0001$), although the difference between the groups fed inulin- and cellulose-containing diets was not significant, the difference between groups fed VFC- and cellulose-containing diets was significant ($p < 0.001$, Dunnets' MCT).

Rats fed a VFC-containing diet had lower serum alkaline phosphatase levels than rats fed cellulose- or inulin-containing diets, as shown in TABLE 2. The normal range for this parameter in Sprague-Dawley rats with no known liver disease or bone disease is 0-267 IU/L (IDEXX reference data). As shown in TABLE 2, the average serum alkaline phosphatase levels for rats fed a VFC-containing diet were within this normal range, whereas the averages for rats fed cellulose- or inulin-containing diets were both outside the normal range. The reduction of alkaline phosphatase between groups fed VFC-versus cellulose- or inulin-containing diets was significant (p <0.001). Lower serum alkaline phosphatase levels with VFC suggests a protective effect on cholestasis, while increases in ALT and AST indicate hepatocellular injury (D.S. Pratt et al., Harrison's Principles of Internal Medicine 15th Edition, pp. 1711-1715 (2001). Conversely, globulin and bilirubin are cleared by the liver, and elevations reflect compromised hepatic function.

The normal range for globulin for Sprague-Dawley rats is 2.8-4.5 g/dL (IDEXX reference data). As shown in TABLE 2, globulin concentrations averaged 3.4 g/dL for rats receiving a VFC-containing diet, and 4.0 and 3.9 g/dL for rats receiving cellulose and inulin containing diets, respectively. The effect of fiber type was significant (p<0.001), with a significant difference between groups fed VFC and cellulose containing chow (p<0.001, Dunnett's MCT), but not between groups fed inulin and cellulose containing diets. Similarly, rats fed a VFC containing diet averaged 0.13 mg/dL total (direct and indirect) bilirubin, as shown in TABLE 2, while rats fed cellulose and inulin containing diets averaged 0.19 and 0.18 mg/dL, respectively. The reference range for Sprague-Dawley rats is 0-0.4 mg/dL (IDEXX reference data). Treatment effects were statistically significant (1W ANOVA, $F(2,28)=4.93$, $p<0.05$), with a significant difference between groups fed VFC-containing diets (p<0.05, Dunnett's MCT) but not between groups fed inulin and cellulose containing diets. Although globulin and bilirubin levels were within normal limits for all groups, rats fed a VFC containing diet showed significantly lower concentrations (p<0.001) of both analytes versus the other groups, suggesting improved liver function.

The normal range for bilirubin for Sprague-Dawley rats is 0-0.4 mg/dL (IDEXX reference data). No significant differences in bilirubin were observed between the treatment groups. Albumin, which is synthesized by the liver, was similar in all treatment groups.

TABLE 2: Plasma Chemistry of key analytes taken at the termination of the study in non-fasted Zucker diabetic rats (baseline measurement of final non-fasted OGTT)

| Diet | VFC (PGX®) | Cellulose | Inulin |
| --- | --- | --- | --- |
| Cholesterol (mg/dL) | 179.6 ± 6.4*** | 383.7 ± 232 | 350.8 ± 213 |
| Aspartate aminotransferase (AST) (IU/L) | 165.9 ± 24.5*** | 871.4 ± 109.3 | 1010.1 ± 169.1 |
| Alanine aminotransferase (ALT) (IU/L) | 93.3 ± 13.3* | 299.4 ± 30.9 | 472.7 ± 77.7* |
| Bilirubin (mg/dL) | 0.1 ± 0.0* | 0.2 ± 0.0 | 0.2 ± 0.0 |
| Alkaline Phosphatase (IU/L) | 134.2 ± 7.7*** | 327.7 ± 46.8 | 302.3 ± 30.3 |
| Globulin (g/L) | 3.4 ± 0.1*** | 4.0 ± 0.1 | 3.9 ± 0.1 |
| Albumin (g/dL) | 3.1 ± 0.1 | 2.9 ± 0.1 | 2.8 ± 0.1 |
| Blood Urea Nitrogen (mg/dL) | 10.4 ± 0.6 | 13.4 ± 0.7 | 17.0 ± 2.6 |
| Triglycerides (mg/dL) | 276.4 ± 24.6 | 276.7 ± 43.5 | 352.6 ± 67.6 |
| * significantly different from cellulose group (p < 0.05) ** significantly different from cellulose group (p < 0.01) *** significantly different from cellulose group (p < 0.001) | | | |

**Discussion of Results:**

This study in the Zucker diabetic rat model demonstrates that a VFC-containing diet significantly improves glycemic control, reduces kidney damage, preserves pancreatic beta cells, improves insulin sensitivity, and therefore reduces total glucose load in the body. In addition, a reduction in the rate of body weight gain by approximately 10% over the course of the study was observed in rats fed a VFC containing diet in comparison to the other fiber enriched diets. This may be partially due to the reduced food intake seen during the study, although the reduced food intake was significant for only the first three weeks of the study. As further shown in Example 4, VFC also increases secretion of GLP-1 and satiety-inducing PYY.

The amount of VFC granules used in this study was 5% VFC added to the rat chow. As shown in FIGURES 1A and 1B, the consumption of food per day for VFC fed rats averaged approximately 22 g/day, so in 22 grams, 1.1 g VFC. Assuming the average weight of the Zucker rats is approximately 300 g (see FIGURE 1A), then the dosage in this study per kg was approximately 3.66 g/d/kg. Assuming a human is about 60 kgs, then this dosage would be the equivalent of about 219.6 g/day for a human. Using the conversion for dosages based on body surface area to volume of from 0.1 to 0.15 the rat dose to human, as described in Reagan-Shaw et al., FASEB Journal 22:659-661 (2007), this would translate into a dosage range in a 60 kg human of from about 22 grams to about 33 grams VFC per day, or from about 366 mg/kg/day to about 550 mg/kg/day.

In this study, the fasted Zucker diabetic rats (i.e., animals tested in the morning after approximately 16 hours without food access) did not have greatly elevated glucose levels, likely due to the adequate compensation provided by the hyperinsulinemia observed as the disease was just beginning to become apparent. In the animals fasted for 16 hours prior to testing, across diet groups, insulin levels were higher in the cellulose and inulin-treated groups as compared to the VFC-treated group, which in conjunction with the HOMA and CISI scores is indicative of a greater peripheral insulin resistance in the inulin- and cellulose-treated groups in comparison to VFC-treated animals. Therefore, it appears that VFC does not need to be present in the gut to improve insulin sensitivity. While not wishing to be bound by any particular theory, the improved insulin sensitivity observed in the VFC-treated animals that were fasted for 16 hours prior to testing may be due to increased proglucagon expression (S.P. Massimino et al., J. Nutr. 128:1786-1793 (1998); R.A. Reimer etal., Endocrinology 137:3948-3956 (1996)); or may be due to upregulation of muscle GLUT-4 (Y.J. Song et al., Clin. Exp. Pharm. Physiol. 27:41-45 (2000)).

Since the animals that were fasted for 16 hours prior to testing were only slightly hyperglycemic, starting at three weeks into the study, plasma glucose was also measured in the rats under a non-fasted state (i.e., continuous access to food prior to testing). It was determined that in the animals tested in the non-fasted state, the animals in the cellulose and inulin treated groups were hyperglycemic, whereas the animals in the VFC-treated group had glucose levels that were reduced to nearly non-diabetic levels. The insulin levels in the non-fasted state were only measured at study termination, and it was found that insulin was significantly reduced in the VFC-treated animals, and the HOMA and CISI scores also showed improved insulin sensitivity in the VFC-treated animals as compared to the other groups.

Therefore, in view of the results that serum insulin was significantly reduced in both fasted and non-fasted states in the VFC-treated animals, and the blood glucose was significantly reduced in VFC-treated animals tested in the non-fasted state, it appears that VFC treatment of the Zucker diabetic rats was effective to delay early progression of diabetes.

In addition to improvements in glycemic control, it was determined that the VFC-treated animals also had reduced organ damage in comparison to the cellulose and inulin-treated animals. Diabetic nephropathy is a clinically important sequela of diabetes, particularly thickening of the glomerular basement membrane and expansion of the mesanguim and tubules and tubular degeneration, resulting from metabolic disturbances and hemodynamic alterations (H.R. Brady and B.M. Brenner: Pathogenesis of Glomerular Injury, in Harrison's Principles of Internal Medicine 15th ed., E. Braunwald et al., pp. 1572-1580 (2001)). Interestingly, it was determined in this study that significant organ damage occurs very quickly in the younger Zucker diabetic rats with the early onset of diabetes, despite a relatively mild diabetes. Importantly, it was observed that VFC-treated animals had a significantly greater density of pancreatic β cells present at the end of the 8-week study as compared to the inulin or cellulose-treated groups. This data indicates that Zucker diabetic rats fed a VFC-containing diet for eight weeks maintained a significantly greater reserve capacity for insulin secretion. It is noted that preservation of pancreatic β cells has been seen for DPP IV inhibitors that increase the levels of the insulin secretagogue GLP-1, and in some studies using DPP IV inhibitors, insulin is higher than control in models of type II diabetes, particularly postprandial insulin levels (A. Viljanen et al., J. Clin. Endocrinol. Metab. 94:50-55 (2009)).

In the Zucker diabetic rat model used in this study, non-fasting glucose levels appeared to be sufficient to cause kidney damage. It was determined that there was less renal injury in the VFC-treated group, in particular with respect to mesangial expansion. Enhanced glycemic control during normal feeding and subsequent reduction in tissue glycation likely served as a major factor in reduced renal injury. Interestingly, and unexpectedly, histology showed that VFC significantly protected kidneys from glycation damage, indicating a reduction in total glucose load, and therefore reduced glycation. The FDA considers reduced glycation as a primary marker for anti-diabetic effects, as a mere reduction of blood glucose is no longer considered sufficient for drug approval.

With regard to the effect of VFC-treatment on serum and hepatic lipid profiles, plasma cholesterol was significantly reduced in the VFC-treated group. The effect on serum triglyceride levels was more variable. Nevertheless, hepatic lipid levels (steatosis) and hepatic measurements such as serum bilirubin, ALT, and AST were significantly reduced in the VFC-treated group, which indicated reduced liver damage in the VFC-treated group. Moreover, based on histological analysis, it was also determined that VFC-treated animals had reduced indices of hepatocellular injury and reduced serum levels of alkaline phosphatase, which may indicate that VFC treated animals had reduced cholestasis as well as a reduction in steatohepatosis, a common accompaniment of metabolic syndrome (A. Viljanen et al., J. Clin. Endocrinol. Metab. 94:50-55 (2009)).

Therefore, efficacy for the use of VFC is demonstrated in ZDFs in terms of glycemic control, reduction of kidney damage and preservation of pancreatic beta cells. As demonstrated in this Example, the VFC-treated rats had less renal injury, in particular mesangial expansion. Enhanced glycemic control and subsequent reduction in tissue glycation likely served as a major factor in reduced renal injury. Therefore, VFC may be used as a dietary additive to help ameliorate the development and progression of the early phase of the metabolic syndrome, including the ability to slow the progression of glucose-induced organ damage, lipid accumulation in the liver, and inhibit loss of pancreatic beta cells.

REFERENCE EXAMPLE 2

This example describes a study carried out in a high-sucrose diet induced obesity rat model to determine the effect of a dietary fiber composition, comprising a granulated viscous fiber blend (VFC granules) (also referred to as the fiber complex PolyGlycopleX (PGX®)) on pancreatic dysregulation, dyslipidemia, and obesity.

**Rationale:** As described in Example 1, the novel, water soluble fiber complex, VFC granules, also referred to as PolyGlycopleX® (PGX®) (manufactured from konjac mannan, xanthan gum, and alginate to form a highly viscous polysaccharide complex with high water holding and gel-forming properties), reduces body weight and increases insulin sensitivity in Zucker diabetic rats. However, the effect of VFC granules observed in the Zucker diabetic rats with respect to serum triacylglycerols (TAG) was variable. The variability of various fibers to reduce serum TAG levels has been observed in other studies, and may relate to fiber type and the particular animal model (W.U. Jie et al., Biomed. Environ. Sci. 10:27-37 (1997); A. Sandberg et al., Am. J. Clin. Nutr. 60:751-756 (1994); R. Wood et al., Metab. Clin. Exp. 56:58-67 (2007); and N.M. Delzenne et al., J. Nutr. 129:1467S-1470S (1999)). For example, a study by Mao-Yu et al. showed that reduction of TAG by non-digestible fibers is dependent on the severity of TAG increase and stability over time (Z. Mao-Yu et al., Biomed. Environ. Sci. 3:99-105 (1990)).

The study described in this Example was carried out to determine the effects of granulated VFC (PGX®) on body weight gain, serum triacylglycerols (TAG), and hepatic steatosis in sucrose-fed Male Sprague-Dawley rats, a model of diet-induced obesity (high sucrose 65% wt/wt), known to result in weight gain and consistent increases in liver and serum TAG levels, particularly when given chronically, which closely mimics human type II diabetes (A.M. Gadja et al., An. Lab News 13:1-7 (2007); M. Hafidi et al., Clin. Exp. Hyperten. 28:669-681 (2006); and P. Rozan et al., Br. J. Nutr. 98:1-8 (2008)). The study described in this example was carried out for 43 weeks in order to capture a reasonable part of the life cycle of the rats and maximize consistent increases in serum TAG levels that are characteristic of this model.

**Methods:**

*Fiber enhanced rat chow:*

Viscous fiber complex (VFC) (konjac/xanthan/alginate (70:13:17)) granules (i.e., the fiber blend was processed by granulation to form a complex, commercially known as PGX®) was incorporated into basic rat chow (D11725: Research Diets, New Brunswick, New Jersey). Alternate diets used in this study incorporated other fiber forms as shown below in TABLE 3. Cellulose was selected as the basic reference fiber that is insoluble and is non-fermentable and is considered to be an inert reference compound (J.W. Anderson et al., J. Nutr. 124:78-83 (1994)).

TABLE 3: Composition of the three diets containing either VFC or cellulose (percent contribution of ingredients by weight)

|  | Viscous fiber complex (VFC) (konjac/xanthan/alginate (70:13:17)) PGX® granules | Insoluble fiber (cellulose) |
| --- | --- | --- |
| Research Diets Formula # | D08012504 | D08012507 |
| Casein | 20% | 20% |
| Methionine | 0.3% | 0.3% |
| Corn Starch | 50% | 50% |
| Maltodextrin | 15% | 15% |
| Fiber* | 5% VFC (PGX®) | 5% cellulose |
| Corn oil | 5% | 5% |
| Salt/mineral mix | 3.5% | 3.5% |
| Vitamin mix | 1% | 1% |
| Choline bitartrate | 0.2% | 0.2% |
| Dye | 0.1% | 0.1% |
| *VFC fiber granules commercially known as PolyGlycopleX® (PGX®) (InnovoBiologic Inc. Calgary, AB, Canada). | | |

*Animal Model:* The male Sprague-Dawley (SD) rat was chosen because the sucrose-fed rat is considered to be an excellent model of hypertriglyceridemia in the presence of a normal genetic background (A.M. Gadja et al., An Lab News 13:1-7 (2007)).

*Study Design:* 30 male SD rats were obtained from Charles River (Kingston NY) at six weeks of age. The animals were housed singly in suspended wire mesh cages, which conformed to the size recommended in the most recent *Guide for the Care and Use of Laboratory Animals,* DHEW (NIH). The animal room was temperature and humidity controlled, had a 12-hour light/dark cycle, and was kept clean and vermin free. The animals were conditioned for four days prior to testing.

*Water:* Filtered tap water was supplied *ad libitum* by an automatic water dispensing system.

*Food:* After habituation, rats were randomly assigned to one of two groups with n=10 per group, cellulose at 5% (wt/wt) or 5% VFB (wt/wt), and with 65% (wt/wt) sucrose added to the diet of both groups. The diets were nearly isoenergetic, with the cellulose diet being 3.90 kcal/g and the VFC diet being 3.98 kcal/g, for a total of approximately 3902 dietary kcal. The rats were fed the high sucrose diet *ad libitum* with either cellulose (starting body weight of 214.7 $\pm$ 2.6 g) or VFB (starting weight of 220.8 $\pm$ 3.5 g) for a total of 43 weeks.

*Study Measurements:* Food consumption (daily), body weight (weekly), and weekly collection of blood samples for measurement of serum triacylglycerols (TAG) (analyzed by IDEXX, North Grafton, MA), blood glucose (via Acensia Elite Glucometer) and serum insulin (Ani Lytics, Gaithersburg, MD) were followed throughout the study. The study was concluded with a final blood analysis to measure hemoglobin glycation and blood urea nitrogen. A limited necropsy was then carried out as follows. One lobe of the liver was snap-frozen for analysis of lipid content using Sudan black histochemistry. One lobe of the liver was post-fixed for hematoxylin and oesin staining.

*Statistical Methods:* Body weight gain was analyzed for statistical differences using repeated measures ANOVA and one-way ANOVA for differences in weight gain throughout the study. Alpha error rates for multiple comparisons were controlled using the Bonferroni correction. Histology scores were measured using Kruskall-Wallis test for non-parametric data.

**Results:**

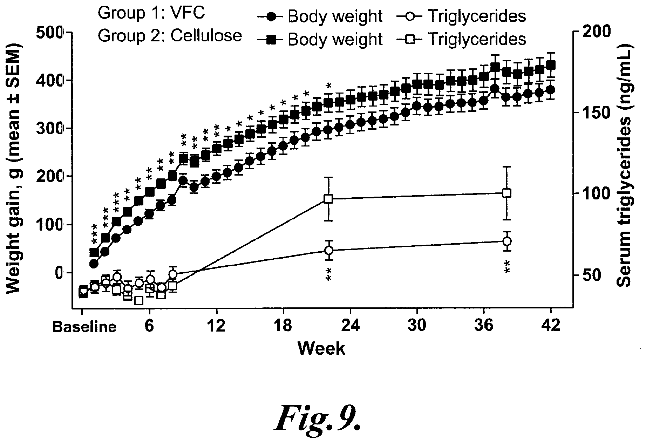

FIGURE 9 graphically illustrates the effect of granulated VFC (PGX®) or cellulose diets on body weight gain and serum triacylglycerols (TAG) in Sprague-Dawley sucrose-fed rats over the 43 week study ("*" symbol indicates $p<0.05$; "**" symbol indicates $p<0.01$; "***" symbol indicates $p<0.001$). Initial body weights did not differ between the VFC fed group (215 $\pm$ 3 grams) and the cellulose fed group (221 $\pm$ 3 g). As shown in FIGURE 9, body weight increased over time in both groups due to the sucrose-rich diet, however weight gain was significantly attenuated in the VFC fed group from study initiation up to week 22 in comparison to the cellulose fed group ($p<0.05$). Repeated measures showed a significant treatment effect on weight gain ($p=0.04$) with VFC fed rats showing reduced weight gain. Although final body weight did not differ significantly between the groups ($p=0.20$; 660 $\pm$ 22 versus 645 $\pm$ 26 g for VFC and cellulose fed, respectively), the VFC rats maintained a 7% lower body weight at study termination. Food consumption for VFC fed rats was similar to cellulose fed rats (data not shown).

As further shown in FIGURE 9, serum TAG levels were stable for the early part of the study, but climbed over time in the cellulose-fed group up to study termination at 43 weeks. In contrast, the VFC fed rats showed significantly lower serum TAG levels versus the cellulose fed group ($p<0.01$). The VFC fed group had baseline TAG levels that were not significantly different from baseline TAG levels in the cellulose fed group.

Rats fed a VFC-containing diet showed less hepatic steatosis (measured by Sudan black staining) that rats fed cellulose diets. Lipid content was determined by staining liver tissue sections with Sudan black, and slides were evaluated and graded semi-quantitatively for the presence of Sudan black positive vacuoles on a scale of 0 to 5, with 5 being the most severe. Severity scores were 3.9 $\pm$ 0.3 for the cellulose treated group and 2.7 $\pm$ 0.4 for the VFC treated group, which was significantly different. A strong tendency for reduction in hepatocellular injury in VFC fed rats was also observed in comparison to cellulose fed rats, although the difference was not statistically significant ($p<0.07$ for macrovesicular vacuolation and $p<0.11$ for microvesicular vacuolation, data not shown).

Blood glucose and insulin levels were monitored weekly throughout the study and were not altered, which is expected for this animal model (A.M. Gadja et al., An. Lab News 13:1-7 (2007)).

**Discussion:**

As expected in this diet-induced model of obesity, the Sprague-Dawley (SD) sucrose-fed rats gained weight rapidly with time until approximately 18-25 weeks, at which time the weight stabilized to a slower rate of growth. As shown in FIGURE 9, during the rapid weight growth period, VFC granules significantly reduced body weight changes in comparison to cellulose, with smaller reductions observed during the slower growth phase in the later part of the study (i.e., older ages of the rats). As further shown in FIGURE 9, plasma TAG only increased above baseline in the older rats, and VFC significantly obtunded this rise in TAG. Consistent with this data, liver steatosis was significantly reduced in VFC fed animals as measured by histomorphometry in comparison to cellulose fed animals.

Weight reduction in subjects consuming non-digestible fibers is thought to be related to one or more of the following: reduced food intake, altered satiety hormone response, reduced nutrient adsorption secondary to gastric slowing and/or nutrient adsorption by the fiber (see N.C. Howarth et al., Nutr. Rev. 59:163-169 (2001); A. Sandberg et al., Am. J. Clin. Nutr. 60:751-756 (1994); G. Grunberger et al., Diabet. Metab. Res. Rev. 23:56-62 (2006); and J.R. Paxman et al., Nutr. Res. 51:501-505 (2008)). It is interesting to note that in the present study, little reduction in food consumption was observed, therefore this factor likely did not contribute to the observed weight reduction in VFC fed animals. While not wishing to be bound by any particular theory, it is possible that slower gastric emptying and reduced nutrient absorption of the food eaten may be responsible for the weight reduction, which may be due to increased secretion of Glucagon-like protein (GLP-1) (N.N. Kok et al., J. Nutr. 128:1099-1103 (1998)).

Reduction of liver or plasma TAG has been the subject of many dietary fiber studies, and the results vary widely (W.U. Jie et al., Biomed. Environ Sci. 10:27-37 (1997); A. Sandberg et al., Am. J. Clin. Nutr. 60:751-756 (1994); R. Wood et al., Metab. Clin. Exp. 56:58-67 (2007); and N.M. Delzenne et al., J. Nutr. 129:1467S-1470S (1999); P. Rozan et al., Br. J. Nutr. 98:1-8 (2008)). Not all studies show TAG absorption to be markedly reduced, with some differences observed between fiber types. For example, a study by Delzenne and Kok showed that oligofructose reduced hepatic steatosis by reducing lipogenesis in fructose-fed rats (N.M. Delzenne et al., J. Nutr. 129:1467S-1470S (1999)). Similarly, Kok et al. suggest that GLP-1 secretion induced by oligofructose fiber may also be responsible for reduced lipogenesis and fat mobilization (N.N. Kok et al., J. Nutr. 128:1099-1103 (1998)). While not wishing to be bound by any particular theory, it is likely that both reduced lipogenesis and reduced fat absorption played a role in the TAG reductions observed in the VFC fed animals in this study. Reduced nutrient absorption would explain the reduction in weight gain observed without a reduction in food consumption.

In conclusion, this study demonstrates that VFC granules significantly lower serum TAG in the Sprague-Dawley (SD) sucrose-fed rat model, which current pharmaceuticals are not very effective at lowering. The reduced liver steatosis parallels the reduced serum TAG, and such properties makes VFC granules a useful food additive for treating patients with hyperlipidemia as well as other aspects of metabolic syndrome including weight loss.

REFERENCE EXAMPLE 3

This Example describes a study in overweight and obese adult human subjects demonstrating the effect of a dietary fiber composition, comprising a granulated viscous fiber complex (VFC granules) (also referred to as the fiber complex PolyGlycopleX (PGX®)) on short-term weight loss and associated risk factors.

**Rationale:** According to recent data published by the World Health Organization, obesity has reached global epidemic proportions with more than 1 billion overweight adults affected by this chronic disorder (www.who.int, accessed 3/15/08). Coronary artery disease and stroke, insulin resistance, (metabolic syndrome), type II diabetes, hypertension, and cancer are all well known medical co-morbidities of excess body weight (K. Fukioka Obesity Res 10(Supp 12):116S-123S (2002)). In addition, a recent epidemiological study confirmed that adult obesity is associated with a significant reduction in life expectancy. This study showed that 40-year-old male and female non-smokers lost on average 7.1 and 5.8 years of their lives respectively due to obesity (A. Peeters et al., Ann. Intern. Med. 138:24-32 (2003)). Given these latter risk factors, a number of therapeutic interventions are available for the overweight/obese that can include surgery, drug therapy, and lifestyle modifications such as diet and exercise.

An important dietary strategy of any weight control program should involve the intake of significant amounts of high fiber foods, particularly foods or food supplements containing viscous soluble fiber (K.M. Queenan et al., Nutr. J. (2007)). It is estimated that the average U.S. citizen consumes approximately 2.4 grams of viscous soluble fiber per day-half the 5 to 10 grams of dietary viscous soluble fiber recommended to be consumed on a daily basis (T.A. Shamliyan et al., J. Family Practice 55:761-69 (2006)).

Due to the difficulty in obtaining ideal amounts of soluble fiber through diet alone, there is a clear need for soluble fiber concentrates that can be used as food ingredients or consumed as supplements to allow for a consistently high intake of soluble fiber. Granulated VFC, also referred to as PGX® (PolyGlycopleX®) is a novel, highly viscous polysaccharide complex that is manufactured by reacting glucomannan, xanthan gum, and alginate using a process referred to as EnviroSimplex®. The resulting polysaccharide complex ($\alpha$-D-glucurono-$\alpha$-D-manno-□$\beta$-D-manno-$\beta$-D-glucan), ($\alpha$-L-gulurono-$\beta$-D-mannuronan), $\beta$-D-gluco-$\beta$-□D-mannan, □$\alpha$-D-glucurono-□$\alpha$-D-manno □-$\beta$-D-manno-$\beta$-D-gluco), ($\alpha$-L-gulurono-$\beta$-D-mannurono), $\beta$-D-gluco-$\beta$-□D-mannan is a novel entity, as demonstrated in the structural analysis described in Examples 5 and 6, and has the highest viscosity and water holding capacity of any currently known fiber.

This example describes a study carried out to examine the efficacy of VFC granules and modest lifestyle modifications on weight loss, body mass index (BMI), as well as cardiometabolic risk factors including cholesterol, low density lipoprotein (LDL) cholesterol, high density lipoprotein (HDL), triglycerides, fasting insulin, fasting glucose, and 2 hour glucose tolerance test during a 14 week time span in overweight and obese adults.

**Methods:**

*Participants:* A total of 29 sedentary adults (23 women; 6 men), aged 20 to 65 years, with a body mass index (BMI) range of approximately 25 kg/m$^2$ to 36 kg/m$^2$, were invited to participate through a series of advertisements placed in local newspapers. Subjects provided informed consent prior to participation in this program. The observational analysis was conducted in accordance with the ethical standards set forth in the Helsinki Declaration of 1975.

*Anthropometric and other measurements:* Participants were evaluated on a bi-weekly basis for height (cm), weight (kilograms), and waist-hip measurements (cm) using a standard medical-type tape measure. Waist-hip measurements were taken at consistent anatomical locations approximately 2.2 cm above the navel and around the hip at the greater trochanter in subjects wearing a disposable paper gown. Percent body fat was determined using bioelectrical impedance testing (RJL Systems, Michigan, USA) at baseline (prior to initiation of the study) and every two weeks thereafter. A computerized analysis of the impedance data was employed in order to determine the body mass index (BMI) and percent body fat.

*Diet and supplementation:* Each volunteer received general directions from a physician for healthy eating, weight loss and exercise. Moreover, dietary and exercise counseling sessions were presented to the group every two weeks for 14 weeks. The emphasis in these lectures was not on calorie counting, but primarily focused on portion control and how to follow and maintain a low fat, low glycemic index diet. General recommendations were also included in this program focusing on the variety, type, and timing of exercise (e.g., strength and cardiovascular-aerobic training) that would augment overall weight reduction. In addition, subjects were provided with granulated viscous fiber complex (VFC) (konjac/xanthan/alginate (70:13:17) granules, also referred to as the fiber complex PolyGlycopleX (PGX®)) that could be added to a beverage or food (e.g., a non-fat yogurt).

Five grams of VFC granules was to be consumed with 500 ml of water 5 to 10 minutes before each meal, two to three times a day for 14 weeks, for a daily total intake of from 10 to 15 grams granulated VFC/day.

*Blood collection and laboratory biochemical analysis:* All laboratory measurements were performed by an independent laboratory in British Columbia, Canada. At baseline (prior to study initiation), subjects were asked to fast ten hours before the blood draw procedure that included the following tests: total cholesterol, triglycerides, HDL, LDL, glucose, insulin, and 2-hour insulin. A 75 gram oral glucose tolerance test was also performed according to the criteria and procedures as determined by the laboratory. Only those with aberrant risk factors were re-tested using the latter laboratory parameters at week 14.

*Statistical analysis:* A computerized statistical analysis was performed using the paired t-test in order to assess several types of variables including height, weight, BMI, % body fat, and various laboratory values before and after treatment. Significant results were obtained in those variables that yielded a p-value of <0.05.

**Results:**

*Weight Loss and Other Anthropometric Parameters:* During the 14 weeks of VFC use, there were significant reductions in group weight (-5.79 ± 3.55 kg), waist measurements (-12.07 ± 5.56 cm), % body fat (-2.43 ± 2.39 %), and BMI (-2.26 ± 1.24 kg/m$^2$). Full results are shown below in TABLES 4 and 5.

Table 4: Group 1: Men and Women Combined

| Test | Sample size | Week 0 Mean & SD | Week 14 Mean & SD | Change & SD | % Change |
|------|-------------|------------------|-------------------|-------------|----------|
| *Waist | 29 | 103.58[b] ± 12.78 | 91.51[b] ± 12.95 | -12.07 ± 5.56[b] | - 11.65 |
| *Hip | 29 | 116.30[b] ± 7.67 | 106. 83[b] ± 7.44 | - 9.47 ± 4.15[b] | - 8.14 |
| *% Fat | 29 | 40.30 ± 8.28 | 37.87 ± 8.88 | - 2.43 ± 2.39 | - 6.02 |
| *p < 0.05 from week 0<br>a= weight is in kilograms (kg)<br>b= waist and hip is in centimeters (cm) | | | | | |

TABLE 5: BMI for all the Groups Combined

| Test | Sample size | Week 0 Mean & SD | Week 14 Mean & SD | Change & SD | % Change |
|------|-------------|------------------|-------------------|-------------|----------|
| *Male | 6 | 35.03[c] ± 4.09 | 32.47[c] ± 3.78 | -2.56[c] ± 1.22 | - 7.31 |

(continued)

| Test | Sample size | Week 0 Mean & SD | Week 14 Mean & SD | Change & SD | % Change |
|---|---|---|---|---|---|
| *Female | 23 | $33.45^c \pm 7.57$ | $31.27^c \pm 8.17$ | $-2.18^c \pm 1.26$ | - 6.52 |
| *All | 29 | $33.78^c \pm 6.96$ | $31.52^c \pm 7.43$ | $-2.26^c \pm 1.24$ | - 6.70 |
| *p < 0.05 from week 0<br>c=BMI in kg/m$^2$ | | | | | |

Similarly, both sexes individually demonstrated significant reductions in the tested weight loss variables as shown in TABLE 6 and TABLE 7 below. As shown below in TABLE 7, men lost on average $8.30 \pm 2.79$ kg over the 14 week study (average of 7.43% weight loss). As shown in TABLE 6, women lost an average of $5.14 \pm 3.49$ kg over the 14 week study (average of 6% weight loss).

TABLE 6: Group 1: Women (n=23)

| Test | Week 0 Mean & SD | Week 14 mean & SD | Change & SD | % Change |
|---|---|---|---|---|
| * Weight | $84.29^a \pm 7.85$ | $79.15^a \pm 8.77$ | $-5.14^a \pm 3.49$ | -6.00 |
| *Waist | $98.98^b \pm 8.99$ | $87.55^b \pm 10.57$ | $-11.43^b \pm 5.71$ | -12.00 |
| *Hip | $115.19^b \pm 6.73$ | $105.92^b \pm 7.34$ | $-9.27^b \pm 4.29$ | -8.00 |
| * % Fat | $43.88 \pm 4.52$ | $41.33^b \pm 6.15$ | $-2.55^b \pm 2.63$ | -6.00 |
| * p < 0.05 from week 0<br>a= weight is in kilograms (kg)<br>b= waist and hip is in centimeters (cm) | | | | |

TABLE 7: Group 2: Men (n=6)

| Test | Week 0 Mean & SD | Week 14 Mean & SD | Change & SD | % Change |
|---|---|---|---|---|
| * Weight | $111.81^a \pm 9.18$ | $103.51^a \pm 13.05$ | $-8.30^a \pm 2.79$ | -7.43 |
| *Waist | $121.13^b \pm 9.65$ | $106.63^b \pm 10.23$ | $-14.50^b \pm 4.59$ | -12.00 |
| *Hip | $120.57^b \pm 7.62$ | $110.36^b \pm 7.39$ | $-10.21^b \pm 3.63$ | -8.00 |
| * % Fat | $26.58 \pm 3.01$ | $24.62^b \pm 2.97$ | $-1.97^b \pm 1.15$ | -7.00 |
| * p < 0.05 from week 0<br>a= weight is in kilograms (kg)<br>b= waist and hip is in centimeters (cm) | | | | |

*Lipid levels:* Compared to the baseline values obtained prior to study initiation, after 14 weeks of VFC use, subjects had an average reduction of 19.26% in total cholesterol values (n = 17; p < 0.05 from week 0) and an average reduction of 25.51% in LDL cholesterol values (n = 16; p < 0.05 from week 0). As shown in TABLE 8, a trend was also observed towards a reduction in triglyceride and an increase in HDL cholesterol values observed in this study, however the differences observed were not statistically significant.

*Fasting Insulin and Glucose:* After 14 weeks of VFC use, subjects in this study experienced an average of a 6.96% reduction in fasting glucose (n = 20; p < 0.05 from week 0), an average of a 12.05% decline in 2 hour glucose tolerance (n = 21; p < 0.05 from week 0), and an average of a 27.26% reduction in fasting insulin levels (n = 17; p < 0.05 from week 0) as compared to baseline measurements taken prior to study initiation.

TABLE 8: Summary of the overall laboratory data obtained during the 14 week trial with VFC (PGX®)

| | Sample size | Week 0 Mean & SD | Week 14 Mean & SD | Change & SD | % Change |
|---|---|---|---|---|---|
| *Total cholesterol (mmol/L) | 17 | $5.69 \pm 1.07$ | $4.60 \pm 0.82$ | $-1.09 \pm 0.63$ | -19.26 |

(continued)

|  | Sample size | Week 0 Mean & SD | Week 14 Mean & SD | Change & SD | % Change |
|---|---|---|---|---|---|
| **Triglycerides (mmol/L) | 17 | 1.92 ± 0.98 | 1.52 ± 0.56 | -0.40 ± 0.89 | -20.97 |
| **HDL (mmol/L) | 17 | 1.48 ± 0.53 | 1.53 ± 0.77 | 0.05 ± 0.67 | 3.33 |
| *LDL (mmol/L) | 16 | 3.40 ± 0.96 | 2.53 ± 0.64 | -0.87 ± 0.56 | -25.51 |
| *Fasting glucose (mmol/L) | 20 | 5.75 ± 0.78 | 5.34 ± 0.49 | -0.40 ± 0.65 | -6.96 |
| *2 hr Glucose (mmol/L) | 21 | 6.09 ± 2.10 | 5.35 ±1.81 | -0.73 ± 1.43 | -12.05 |
| *Insulin Fasting (pmol/L) | 17 | 89.41 ± 44.84 | 65.04 ± 33.21 | -24.37 ± 36.29 | -27.26 |
| **2 hr Insulin (pmol/L) | 17 | 433.53 ± 270.32 | 355.76 ± 332.44 | -77.76 ± 196.51 | -17.94 |
| * p < 0.05 from week 0; **NS (non significant) from baseline | | | | | |

*Analysis of efficacy using self-reporting scales:* In a self-reporting scale completed by the participants at the end of the study, 97.7% of the VFC users noted that they had a positive response to the product both in curbing food cravings and hunger.

*Side effects of the test preparation:* The use of VFC was generally well tolerated by the participants, with minor gastrointestinal (GI) symptoms comprising the majority of all the reported complaints. Sixty-eight percent noted that mild GI symptoms (e.g., gas, bloating, constipation, loose stools) resolved within approximately three weeks of beginning VFC. Thirty-two percent of the participants found that they had mild GI side effects throughout the program, but that these were not sufficient in severity for them to discontinue use. A recent controlled study on the tolerance of VFC (PGX®) was conducted in France which also confirmed these latter findings (I.G. Carabin et al., Nutrition J. 8:9 (2008)).

*Discussion:* The medically supervised weight loss study described in this example demonstrates that the use of VFC granules along with general changes in diet and physical activity over a 14 week time period was of benefit in modifying the cardiometabolic risk factors in overweight and obese subjects. Overall, there was a significant reduction in group weight (-5.79 ± 3.55 kg), waist measurements (-12.07 ± 5.56 cm), and percent body fat (-2.43 ± 2.39%) from baseline. Moreover, these latter physical changes were paralleled by a significant decrease in fasting LDL (-25.51%), fasting glucose (-6.96%), and fasting insulin (-27.26%) levels over a relatively short time span of 14 weeks.

It is interesting to note that men lost more weight on average (-8.30 ± 2.79 kg) than the women (-5.14 ± 3.49 kg) over the 14 week time period. This change could be ascribed to the basic sex differences seen in resting energy expenditure. Dr. Robert Ferraro et al. has shown that the sedentary 24 hour energy expenditure is approximately 5 to 10% lower in women compared to men after statistical adjustments for age, activity, and body composition. (R. Ferraro et al., J. Clin. Invest. 90:780-784 (1992)).

The results obtained by VFC in reducing body weight (-5.79 kg) are comparable to those who have taken the anti-obesity medication orlistat (Xenical®, Alli®). Orlistat is a lipase inhibitor that reduces the absorption of fat (J.B. Dixon et al., Aust. Fam. Physician 35:576-79 (2006)). In a controlled study, 391 mild to moderately overweight individuals who employed the drug orlistat at a dose of 60 mg, three times daily over a 16 week time period, lost 3.05 kg compared to 1.90 kg in the placebo group (J.W. Anderson et al., Ann. Pharmacother. 40:1717-23 (2006).

VFC use also resulted in the reduction of other risk factors associated with mild to moderate obesity. Overall, a significant reduction was observed of total cholesterol levels (-19.26%; -1.09 mmol/L) and LDL cholesterol levels (-25.51%; -0.87 mmol/L) from baseline values (p < 0.05) after 14 weeks of VFC therapy. The reduction in lipid values achieved with VFC was comparable to the use of such early generation statin drugs like lovastatin (Mevacor™). For example, one study noted that within one month of beginning lovastatin therapy, total and LDL cholesterol was decreased by 19% and 27% respectively in those with elevated cholesterol levels (W.B. Kannel et al., Am. J. Cardiol. 66:1B-10B (1990)).

Moreover, as described in Examples 1 and 2, the use of VFC not only decreases blood lipid levels, but also may be used to ameliorate the development and progression of the early phase of metabolic syndrome. An increase in visceral obesity, serum glucose, and insulin levels along with hypertension and dyslipidemia are a group of clinical conditions that are collectively known as the metabolic syndrome (E.J. Gallagher et al., Endocrinol. Metab. Clin. North Am. 37:559-79 (2008)). Research has shown that those who have metabolic syndrome have a 50% greater risk of a experiencing a major coronary event (D.E. Moller et al., Annu. Rev. Med. 56:45-62 (2005)). As such, any reductions in weight, fasting insulin, and glucose would confer significant health benefits on those individuals so afflicted.

In this 14-week study, VFC use resulted in a decrease of fasting insulin levels from $89.41 \pm 44.84$ pmol/L to $65.04 \pm 33.21$ pmol/L (p < 0.05). The reduction in fasting insulin reflects improved insulin sensitivity and may be due in part to increased GLP-1 activity and decreased postprandial hyperglycemia along with the improvements in insulin sensitivity that accompanies weight loss (see G. Reaven et al., Recent Prog. Horm. Res. 59:207-23 (2004)).

These findings are consistent with the results obtained in the Zucker diabetic rat study described in Example 1, and suggest that the therapeutic use of VFC in concert with lifestyle modifications is of practical benefit to those suffering with obesity and certain cardiometabolic risk factors. Unlike other types of standard medical interventions available to treat obesity and elevated cholesterol levels, VFC use is associated with minimal side effects. This advantageous safety profile, along with its therapeutic efficacy, suggest that VFC should be considered as a first line therapy for those who are overweight/obese, have elevated cholesterol levels and/or are insulin resistant.

## REFERENCE EXAMPLE 4

This example describes a study in healthy adults with normal weight showing increased plasma PYY levels and increased fecal short chain fatty acids (SCFA) following supplementation with viscous fiber complex (VFC) in comparison with control subjects fed skim milk powder.

### Rationale:

Numerous dietary fibers have been shown to have numerous health benefits, including enhancing the secretion of gut satiety hormones and improving bowel function (R.A. Reimer et al., Endocrinology 137:3948-3956 (1996); Reimer and Russell, Obesity 16:40-46 (2008); P.D. Cani et al., Br. J. Nutr. 92:521-526 (2004); T.C. Adam and R.S. Westererp-Plantenga, Br. J. Nutr. 93:845-851 (2005)). Glucagon-like peptide-1 (GLP-1) and peptide YY (PYY) are anorexigenic peptides involved in reducing food intake, while ghrelin, the only known orexigenic peptide, is associated with hunger (Wren and Bloom, Gastroenterology 132:2116-2130 (2007)).

Although the mechanisms regulating these benefits of dietary fibers are not fully understood, the production of short chain fatty acids (SCFA) is believed to mediate some of the effects. SCFA, and chiefly acetate, butyrate, and propionate, are produced in the large bowel by anaerobic fermentation of fermentable dietary fibers, and have been linked to stimulation of satiety hormones and modulation of serum cholesterol.

The objective of this study was to examine the levels of the gut satiety hormone GLP-1, PYY and ghrelin as well as the fecal SCFA concentrations in healthy subjects after consuming either VFC (PGX®) or control (skim milk powder) for 21 days.

### Methods:

*Subjects*: Participants were healthy, non-smoking males and females between the ages of 18 and 55 years with a BMI between 18.5 and 28.4 $kg/m^2$ (i.e., normal weight).

*Study Design:* The randomized, double-blind, placebo controlled trial was carried out as follows: Participants were randomly assigned into two groups:

Group 1 (n=27) consumed the test product Viscous fiber complex (VFC) (konjac/xanthan/alginate (70:13:17)) granules (i.e., the fiber blend was processed by granulation to form a complex, commercially known as PGX® supplied by Inovobiologic Inc., Calgary, CA).
Group 2 (n=27) consumed the control product (skimmed milk powder, which was of similar color and texture as the test product).

The control and test product were pre-mixed with 10 g of a commercial breakfast cereal by CRID Pharma, France, and packaged with 135 ml of a commercially available plain yogurt. Participants combined the yogurt and pre-mixed product prior to ingestion.

For the first seven days of the study, participants consumed 2.5 g of product (test or control) twice a day as part of two main meals. For the last 14 days of the study, participants consumed 5 g of product (test or control) twice a day. For the duration of the study, participants were instructed to abstain from consuming fiber-rich foods and limit dietary fiber intake to approximately 10g per day. With the exception of the pre-mixed product and yogurt, all other foods were purchased and prepared by the participants as per their usual diet.

*Assessments:* Participants had assessments performed at four separate visits. Screening (Visit 0, a screening visit, "V0") involved a physical examination.

*Blood Samples:* A fasted blood sample was collected at Visit 1, day 0 of the study (baseline). Visit 2 = seven days into the study, following the one week of 5 g of product ingestion. Visit 3 = 21 days into the study, after two weeks of 10 g of product ingestion. During each visit, blood was collected in an EDTA treated tube with the addition of Diprotin A (0.034 mg/ml blood; MP Biomedicals, Illkirch, France) and centrifuged at 3000 rpm for 12 min at 4°C. Plasma was stored at -80°C until analysis.

*Stool Collection:* Fecal samples were obtained from subjects at baseline (VI, Day 0), following one week of 5 g/d of product ingestion (V2, Day 8 ± 1), and following two weeks of 10 g/d of product ingestion (V3, Day 22 ± 2). Subjects collected one fecal sample within 48 hours prior to each scheduled visit. Approximately 5 g of sample were shipped on dry ice for analysis.

### Blood Plasma Analysis:

*GLP-1:* Active GLP-1 was quantified using an ELISA kit from LINCO research (Millipore, St. Charles, MO). According to the manufacturer, the assay sensitivity is 2 pM for a 100 $\mu$l sample size. The intra-assay CV is 8% and the inter-assay CV is 13% at 4 pM (Millipore, St. Charles, MO).

*PYY and Ghrelin:* PYY and Ghrelin were quantified using ELISA kits from Phoenix Pharmaceuticals, Inc. (Burlingame, CA). The assay sensitivity for PYY was 0.06 ng/ml and 0.13 ng/ml for ghrelin. Intra-assay CV was <5% for both assays and inter-assay CV<14% and <9% for PYY and ghrelin, respectively.

*Insulin:* Insulin was measured using an ELISA kit from Milliport (St. Charles, MO). The assay sensitivity is 2 $\mu$U/ml with an intra-assay CV <7% and inter-assay CV <11.4%.

*Statistical Analysis:* Results are presented as mean ± SEM. Peptide levels at the three visits were analyzed by repeated measures ANOVA with a Bonferroni adjustment [two-factor analysis with time (VI, V2, V3) and diet as parameters]. Associations between two parameters were computed using Pearson correlation coefficients. The homeostatic model assessment for insulin resistance was calculated using the formula [HOMA-IR= fasting insulin ($\mu$U/ml) X fasting glucose (mmol/l)/22.5]. Data was analyzed using SPSS v 16.0 software (SPSS Inc. Chicago IL).

*Fecal Analysis:* SCFA measurements were performed according to Van Nuenen et al., Microbial Ecology in Health and Disease 15:137-144 (2003). Briefly, fecal samples were centrifuged and a mixture of formic acid (20%), methanol and 2-ethyl butyric acid (internal standard, 2 mg/ml in methanol) added to the clear supernatant. A 0.5 ml sample was injected on a GC-column (Stabilwax-DA, length 15 m, ID 0.53 mm, film thickness 0.1 mm; Varian Chrompack, Bergen op Zoom, The Netherlands) in a Chrompack CP9001 gas chromatograph using an automatic sampler. Both L- and D-lactate were determined enzymatically in clear supernatant by a Cobas Mira plus autoanalyzer (Roche, Almere, The Netherlands). The pH was measured using a microelectrode. Dry matter was measured by drying a sub-sample to dryness at 110°C for a minimum of 2 days.

*Statistical analysis:* Results are presented as mean ± SEM. SCFA at the three visits were analyzed by repeated measures ANOVA with visit (VI, V2, V3) as the within-subject factor and treatment as the between-group factor. Correlations between SCFA and other measured outcomes (satiety hormones, glucose, insulin and HOMA-IR) were determined using Pearson's correlation analysis. Significance was set at $P \leq 0.05$.

### Results:

54 subjects (25 males and 29 females) participated in the study and attended all four visits (V0-V3). No subjects withdrew from the study, and the product was well tolerated. The control group receiving the control product (11 Males, 16 females) had a mean age of 30.9 ± 10.8 and initial BMI of 22.8 ± 2.4. The group receiving the test product (VFC) had a mean age of 32.3 ± 10.3 and initial BMI of 22.7 ± 2.1. There were no differences in baseline clinical and biochemical characteristics between the groups.

Body weight, glucose, insulin and HOMA-IR scores at V1, V2 and V3 are provided below in TABLE 9.

TABLE 9: Body weight, and biochemical parameters of participants consuming control or VFC.

| | Control Group (Skim milk powder) | | | Test Group (VFC) | | |
|---|---|---|---|---|---|---|
| | V1 (day 0) | V2 (day 7) | V3 (day 21) | V1 (day 0) | V2 (day 7) | V3 (day 21) |
| Body Weight (kg) | 64.60 ±1.57 | N/M | 64.60±1.52 | 68.20±1.71 | N/M | 68.43±1.67 |
| Glucose (mmol/1) | 4.60±0.06 | 4.60±0.07 | 4.62±0.10 | 4.67±0.09 | 4.60±0.08 | 4.60±0.08 |
| Insulin ($\mu$U/ml | 5.32±0.85 | 4.52±0.33 | 5.19±0.33 | 5.52±0.56 | 4.52±0.49 | 4.61±0.47 |
| HOMA-IR | 1.11±0.20 | 0.93±0.07 | 1.07±0.07 | 1.15±0.11 | 0.96±0.11 | 0.94±0.10 |
| Values represent the mean ± SEM (n=27/group). N/M=not measured. When gender was included as a covariant in the repeated measures ANOVA, the difference between visits was significant for HOMA-IR (p=0.024) for the test group. | | | | | | |

As shown above in TABLE 9, there were no significant changes in body weight between V1 and V3 in the control and test groups. Fasting plasma glucose did not differ over time or between groups. Although there was a 14% reduction in fasting insulin between V1 and V3 in the test group (i.e. with PGX), this difference was not statistically different from the control group. The mean raw and percent change for HOMA-IR scores were -0.04 or -3.6% for the control group and -0.21 or -18.3% in the test group. The percent decrease in HOMA-IR was significantly greater in the test group than control ($P$=0.03). Repeated measures ANOVA showed a $P$=0.067 for the effect of visit. When gender was included as a covariant in the repeated measures analysis, the effect of visit was statistically significant ($P$=0.024). When analyzed separately, males showed greater decreases in HOMA-IR scores than females ($P$=0.042) between V1 and V3. The reduction in HOMA-IR was similar for control and test in male participants (-0.36 $\pm$ 0.20 and -0.31 $\pm$ 0.18, respectively). In females, however, HOMA-IR scores were increased in the control group (+0.18 $\pm$ 0.17) and decreased in the test group (-0.08 $\pm$ 0.19).

There were no significant differences in fasting GLP-1 levels across visits or between groups (data not shown).

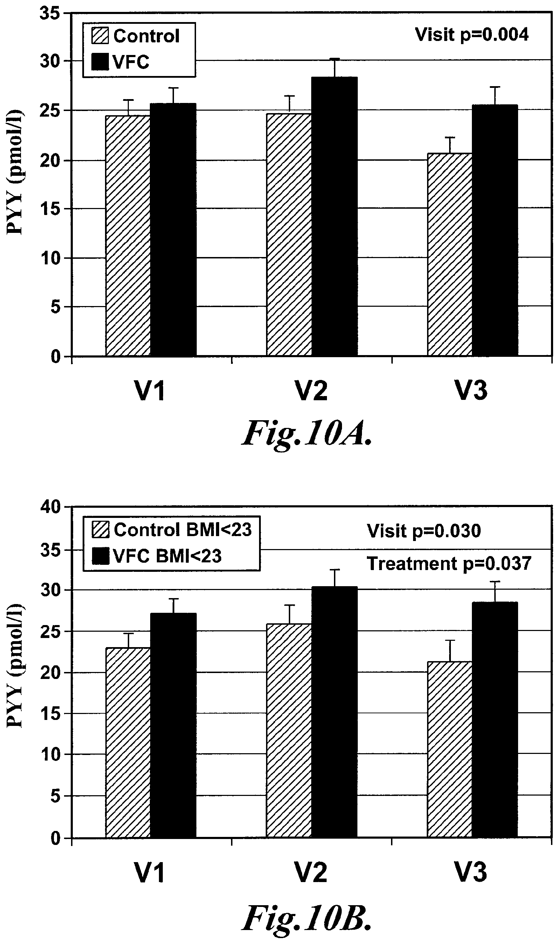

FIGURE 10A graphically illustrates the effect of control versus VFC on fasting PYY levels in healthy adults for all participants (n=54) at V1 (day 0), V2 (day 14) and V3 (day 21). Values are mean $\pm$ SEM. As shown in FIGURE 10A, repeated measures analysis showed a statistically significant effect of visit ($P$=0.004) for fasting PYY levels. When the results shown in FIGURE 10A were stratified by BMI, those participants with a BMI<23 showed a significant difference in PYY levels as an effect of visit ($P$=0.03) and treatment ($P$=0.037), as shown in FIGURE 10B. Analysis of variance showed a significantly higher level of PYY in the test group versus the control group at the end of the study ($P$=0.043). It is noted that increased PYY levels are advantageous, as it is an anorexigenic hormone that is associated with reduced food intake.

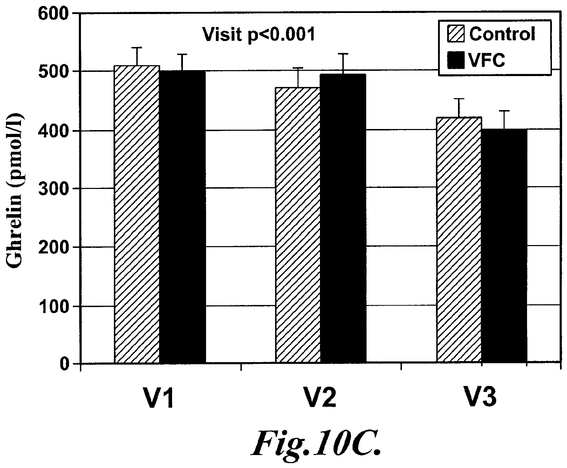

As shown in FIGURE 10C, repeated measures ANOVA showed a significant effect of visit (P<0.001) for fasting total ghrelin levels and treatment (p=0.037). As shown in FIGURE 10C, reductions of 89.7 $\pm$ 20.0 and 97.7 $\pm$ 26.6 pmol/l were observed in the control and VFC treated test groups, respectively.

PYY was negatively correlated with glucose at V2 (r = -0.27, $P$=0.046). There were also significant negative correlations between ghrelin and insulin at V1 and V2 (r = -0.28, $P$=0.038 and r = -0.31, $P$=0.022, respectively) and between ghrelin and HOMA at V1 and V2 (r = -0.27, $P$=0.052 and r = =0.28, $P$=0.041, respectively).

Fecal SCFA and Lactate:

As shown below in TABLE 10, concentrations of acetate were significantly higher with VFC (PGX®) versus the control (P=0.01) group. There were no differences in acetate concentrations between the groups at V1 (baseline; p=0.286) or V2 (p=0.096), but concentrations were significantly higher with VFC (PGX®) than the control group at V3 (p=0.018). There were no significant treatment differences in propionate, butyrate, valerate, caproate, or lactate concentrations between the groups. Repeated measures analysis showed a significant treatment effect (P=0.03) for total SCFA which was identified as higher total SCFA at V3 in subjects consuming VFC (PGX®) versus control (P=0.06). There was a significant effect of visit for fecal pH (0.02) with both groups decreasing between V1 and V3.

*Correlations with satiety hormones, insulin and glucose*

The results of the analysis of levels of plasma ghrelin, PYY, GLP-1, insulin, glucose, and HOMA-IR are shown above in TABLE 9. As shown below in TABLE 10, there was a significant negative correlation between fasting ghrelin and propionate at V3 (r= -0.29; $P$=0.03). The change in propionate between baseline and the final visit was calculated as V3-V1 and referred to as delta propionate. Delta propionate was negatively associated with delta insulin (r= -0.26; P=0.05) and delta HOMA-IR (r= -0.25; P=0.07).

TABLE 10: Fecal concentrations of short-chain fatty acids (SCFA) and lactate in subjects following VFC (PGX®) or control product supplementation.

| | Control | | | VFC (PGX®) | | | P-values | | |
|---|---|---|---|---|---|---|---|---|---|
| | V1 | V2 | V3 | V1 | V2 | V3 | Visit | Treatment | Visit $\times$ Treatment |
| SCFA (mmol/g feces) | | | | | | | | | |
| Total | 61.1±4.4 | 59.2±5.0 | 53.5±5.2[†] | 66.8±4.4 | 63.5±3.6 | 66.9±4.7 | 0.78 | 0.03 | 0.48 |
| Acetate | 35.8±2.4 | 33.2±2.5 | 30.3±2.7* | 39.5±2.4 | 38.7±2.0 | 39.9±2.8 | 0.51 | 0.01 | 0.40 |
| Butyrate | 10.0±1.1 | 11.1±1.5 | 9.5±1.2 | 12.4±1.3 | 10.7±0.9 | 11.6±1.0 | 0.77 | 0.26 | 0.31 |
| Propionate | 11.4±1.2 | 10.8±1.1 | 10.2±0.4 | 10.9±0.8 | 10.7±0.8 | 11.5±1.0 | 0.89 | 0.85 | 0.55 |
| Valerate | 3.1±0.3 | 3.7±0.4 | 3.0±0.4 | 3.4±0.3 | 3.1±0.3 | 3.3±0.3 | 0.66 | 0.98 | 0.20 |
| Caproate | 0.58±0.10 | 0.49±0.11 | 0.41±0.11 | 0.55±0.09 | 0.41±0.08 | 0.50±0.12 | 0.33 | 0.94 | 0.60 |
| Lactate (mmol/g feces) | 0.62±0.09 | 0.74±0.09 | 0.46±0.07 | 0.52±0.09 | 0.48±0.09 | 0.46±0.07 | 0.05 | 0.22 | 0.12 |
| pH | 6.82±0.09 | 6.71±0.16 | 6.43±0.25 | 6.69±0.09 | 6.68±0.09 | 6.40±0.08 | 0.02 | 0.77 | 0.88 |

Values represent the mean ± SEM (n=27/group). The symbol * represents a significant difference between control and VFC (PGX®) at visit 3 (V3). The symbol [†] represents a trend (p=0.06) for a difference between control and VFC (PGX®) at visit 3 (V3).

**Discussion**

*Analysis of Plasma PYY levels*

The results of the study described in this example demonstrate that VFC use increases levels of fasting PYY compared to control product and this is statistically significant in participants with a BMI <23. Plasma concentrations of PYY are typically reduced in overweight and obese humans (R.L. Batterham et al., Nature 418:650-654 (2002)), and this impaired secretion of PYY may promote the development of obesity and/or hinder weight loss.

While the participants in the control arm of this study saw a modest reduction in fasting PYY over the course of the three week study, the participants consuming VFC were able to maintain, and in the case of those with BMI<23, actually increase their PYY levels. It has recently been shown that microbial fermentation of prebiotics is associated with an increase in GLP-1 and PYY production in healthy adults (P.D. Cani et al., Am. J. Clin. Nutr. (2009)). In rodents, short chain fatty acids (SCFA), which are the by-products of microbial fermentation of dietary fiber, have been shown to directly stimulate PYY secretion (V. Dumoulin et al., Endocrinology 139:3780-3786 (1998)). Konjac glucomannan, one of the starting materials of VFC, has been shown to increase the fecal concentrations of acetate, proprionate, and butyrate in humans (H.L. Chen et al., J. Am. Coll. Nutr. 27:102-108 (2008)). Viscosity of fiber has also been shown independently to affect food intake, and this effect may be mediated by alterations in satiety hormone release.

Levels of fasting ghrelin were suppressed between visit 1 (day 0) and visit 3 (day 21) in both the group consuming the test product containing VFC and in the group consuming the control product. Because ghrelin stimulates food intake and promotes adiposity (A.M. Wren et al., J. Clin. Endocrinol. Metab. 86:5992-5995 (2001); M. Tschop et al., Nature 407:908-913 (2000)), compounds that attenuate the progressive rise in ghrelin prior to meals are attractive. While the 8 pmol/l greater reduction in ghrelin observed in the VFC group versus the control group was not significantly different in this study, others have shown reductions in fasting and meal-related ghrelin with dietary fiber (see e.g., Parnell and Reimer, 2009). While the mechanisms by which dietary compounds suppress ghrelin are not well known, it has been hypothesized that the absorption rate of nutrients and the osmolarity of the intestinal lumen could play a role (Overduin et al., Endocrinology 146:845-850 (2005)). Further in this regard, it is noted that VFC has a 3- to 5-fold greater viscosity than any currently known individual polysaccharide, and is therefore likely to alter nutrient absorption along the intestine.

In this study, there was no difference detected in fasting levels of GLP-1 between the two groups over the course of the three weeks. This lack of change in GLP-1 has been observed with other dietary fibers (T.C. Adam and R.S. Westererp-Plantenga, Br. J. Nutr. 93:845-851 (2005); K.S. Juntunen et al., Am. J. Clin. Nutr. 78:957-964 (2003)).

Although the concentrations of glucose and insulin in the healthy subjects that participated in this study were well within normal ranges, the 14% reduction in insulin over the course of the study in the test group and the 5.3 fold greater reduction in HOMA-IR scores in the test group versus the control group may be indicative of underlying improvements in insulin sensitivity, which is consistent with the results obtained in Examples 1 and 3. In summary, this study demonstrates that VFC (PGX®) increases fasting levels of PYY, a gut peptide involved in reducing food intake, in healthy participants.

*Analysis of Fecal SCFA levels*

As described *supra,* fermentable dietary fibers have been shown to reduce energy intake and increase the secretion of anorexigenic gut hormones. The generation of SCFA from the microbial fermentation of dietary fibers in the distal gut is thought to play a role in this regulation. Recently, Cani et al., Am J Clin Nutr 90:1236-1243 (2009) demonstrated a significant correlation between breath hydrogen excretion (measure of gut microbial fermentation) and plasma GLP-1, a potent insulinotropic hormone that also reduces food intake. The present study builds on these data by demonstrating a significant increase in fecal concentrations of total SCFA, and specifically acetate, in subjects consuming up to 10 g/d of the novel functional fiber complex, PGX®.

Acetate, propionate, and butyrate are the chief SCFA produced in the distal gut. The free fatty acid receptors (FFAR) that sense SCFA in the intestine have recently been identified as FFAR2 (also known as GPR43) and FFAR3 (also known as GPR41). See Ichimura A. et al., Prostaglandins & Other Lipid Mediators 89:82-88 (2009). FFAR2 is expressed in enteroendocrine cells that express PYY, which is consistent with data showing that SCFA stimulate PYY release (Ichimura et al., 2009). *In vitro,* acetate and propionate have been shown to inhibit lipolysis in 3T3-L1 adipocytes via FFAR2 activation and suppress plasma free fatty acids (FFA) in vivo in mice. See Ge H et al., Endocrinology 149:4519-4526 (2008). Elevated FFA have been associated with insulin resistance and dyslipidemia. There is also evidence suggesting that orally administered propionate increases leptin in mice via FFAR3 (Ichimura et al., 2009). Given that leptin acts centrally to reduce food intake, it is possible that SCFA produced by microbial fermentation of dietary fibers regulate host metabolism in part through FFAR2 and FFAR3.

The results of this study demonstrate a significant increase in acetate and total SCFA by the end of three weeks of VFC (PGX®) supplementation. While there were no changes in body weight in our subjects over the three weeks of supplementation, it is possible that consumption of the PGX® fiber at the final dose tested (10 g/d) could decrease body fat mass as has been shown with other soluble fibers such as oligofructose over a period of three months. (Parnell J.A. et al., Am J Clin Nutr 89:1751-1759 (2009). The negative correlation between propionate and ghrelin fits with the overall reduction in food intake associated with dietary fibers, particularly those with high viscosity such as VFC (PGX®). The negative correlation with insulin and HOMA-IR is consistent with the ability of this functional fiber to improve overall metabolic health and reduce insulin resistance.

In conclusion, the results of this example show an increase in fecal acetate in subjects consuming a moderate dose of the highly viscous and soluble fiber, VFC (PGX®), over a 3-week time period. The SCFA, propionate, was negatively correlated with fasting ghrelin, insulin and HOMA-IR. This is the first report to our knowledge showing an increase in fecal SCFA concentrations with VFC (PGX®) that suggest its fermentation in the colon may trigger a cascade of physiological effects, potentially mediated via FFAR2 and FFAR3.

## EXAMPLE 5

This Example describes the analysis of the primary structure of granulated viscous fiber complex (VFC) (konjac/xanthan/alginate (70:13:17) (i.e., the fiber blend was processed by granulation to form a complex, commercially known as PGX®).

### Rationale:

Polysaccharides are naturally occurring polymers composed of sugars (monosaccharides) linked through their glycosidic hydroxyl groups. They may be branched or linear and can have very high molecular weights ranging from several thousand Daltons to more than two million. The primary structure of granulated VFC (70% konjac-mannan, 17% xanthan gum, 13% sodium alginate) was determined using methylation analysis, hydrolysis and chromatography and hydrolysis and NMR spectroscopy.

Konjac glucomannan is a partially acetylated (1, 4)-$\beta$-D-glucomannan obtained from the tubers of Amorphophallus konjac or Konnyaku root (Bewley et al., 1985, Biochemistry of Storage Carbohydrates in Green Plants, Academic Press, New York, pp. 289-304).

Xanthan gum is a microbial polysaccharide produced by Xanthomonas campestris. It has unique rheological and gel forming properties. The structure of xanthan is based on a cellulosic backbone of $\beta$-(1, 4)-linked glucose unitsk, which have a trisaccharide side chain of mannose-glucuronic acid-mannose linked to every second glucose unit in the main chain. Some terminal mannose units are pyruvylated, and some of the inner mannose units are acetylated (Andrew T. R., *ACS Symposium Series No. 45* (1977)).

Sodium Alginate is a sodium salt of a polysaccharide obtained from the brown seaweeds (e.g. Laminaria hyperborea, Fucus vesiculosus, Ascophyllum nodosum). The chemical structure consists of blocks of (1, 4) linked-$\beta$-D-polymannuronic acid (poly M), (1,4) linked-$\alpha$-L-polyguluronic acid (poly G) and alternating blocks of the two uronic acids (poly MG). Grasdalen, H., et al., Carbohydr Res 89:179-191 (1981). Alginates form strong gels with divalent metal cations and the 'egg box' model has been used to describe this form of gelation. See Grant, G.T., et al., FEBS Lett 32:195-198 (1973).

### Methods:

All the polysaccharides used in this Example were supplied by InovoBiologic Inc (Calgary, Alberta, Canada). Single polysaccharides were: konjac glucomannan (lot nos. 2538 and 2681); xanthan gum (lot nos. 2504 and 2505); and sodium alginate (lot nos. 2455, 2638, and 2639). Granulated VFC (PGX, lot nos. 900495 and 2029070523) was produced by blending 70% konjac-mannan, 17% xanthan gum, and 13% sodium alginate), adding 30% to 60% (w/w) water to the VFB and then drying off the added water by applying heat. Samples of the same ternary mixture (unprocessed VFB) were taken prior to processing (e.g., granulation), which are referred to as ternary mixture #1 (TM1, lot nos. 900285, 900416, and 1112050809).

### 1. Methylation Analysis

**Rationale:** GCMS analysis of partially methylated alditol acetates has been used to reveal the monosaccharide components of polysaccharides and their positions of linkage (H. Björndal et al., Carbohydrate Research 5:433-40 (1967)). Therefore, methylation analysis can reveal new and unexpected sugars and linkage positions that have been created by the process of adding the three polysaccharides (konjac-mannan, xanthan gum, and alginate) together and processing them through heat treatment and the granulation process. However, methylation analysis does not show how sugars are linked together ($\alpha$ or $\beta$). Methylation analysis is known to be unsatisfactory for analysis of uronic acids (e.g., sodium alginate), which do not methylate and are resistant to hydrolysis (Percival et al., Chemistry and Enzymology of Marine Algal Polysaccharides, Academic Press 101 (1967)). Because sodium alginate is composed entirely of uronic acids (mannuronic and guluronic acids), additional methods were required to analyze VFC, which involved hydrolysis and analysis of the resulting neutral sugars and uronic acids by high performance anion exchange chromatography with pulsed amperometric detection (HPAEC-PAD) and $^1$H NMR spectroscopy, as described below.

**Methods:**

The samples shown below in TABLE 11 were analyzed, which includes each individual component of VFC (konjac mannan, sodium alginate, xanthan gum), ungranulated VFB (referred to as "ternary mixture #1" or "TM1") and granulated VFC (referred to as PGX®). Weighed amounts of single polysaccharides and ternary mixtures were taken, and a few drops of dimethyl sulphoxide were added to 450 µg of each sample. The samples were permethylated using sodium hydroxide (NaOH/methyl iodide (MeI)), the samples were shaken then sonicated for a total of four times over a period of two hours. The samples were purified by chloroform extraction then hydrolyzed with 2M trifluoroacetic acid (TFA) for two hours at 120°C and reduced with sodium borodeuteride ($NaBD_4$) in 2M $NH_4OH$ for two hours at room temperature. The borate produced on the decomposition of the borodeuteride was removed by three additions of a mixture of methanol in glacial acetic acid (90:10) followed by lyophilization. The samples were then acetylated using acetic anhydride (1 hour at 100°C). The acetylated samples were purified by extraction into chloroform.

**Results of Methlyation Analysis:**

TABLE 11: Retention times (in minutes) of PMAAs corresponding to the sugars and linkages in various sample lots identified by GCMS (tr=trace, nd=not detected)

| Sample | Lot No. | Terminal Mannose or Glucose | 2-Linked Mannose | 4-Linked Mannose | 4-Linked Glucose | 3,4-Linked Hexaose |
|---|---|---|---|---|---|---|
| konjac mannan | 2538 | 12.52 | nd | 13.78 | 13.86 | nd |
| sodium alginate | 2455 | nd | nd | 13.78 | nd | nd |
| xanthan gum | 2504 | 12.54 | 13.68 | nd | 13.85 | 14.65 |
| ungranulated VFB (TM1) | 900285 | nd | trace (13.65) | 13.77 | 13.85 | 14.65 |
| ungranulated VFB (TM1) | 1112050 809 | 12.47 | trace (13.62) | 13.74 | 13.83 | trace (14.63) |
| granulated VFC (PGX®) | 2029070 523 | 12.51 | trace (13.66) | 13.78 | 13.86 | 14.65 |
| granulated VFC (PGX®) | 2029070 523 | 12.48 | nd | 13.74 | 13.83 | trace (14.62) |
| "TM1": ternary mixture #1 | | | | | | |

Table 11 provides a summary of the results observed in reconstructed ion chromatograms (not shown) of the linkage analysis performed on partially methylated alditol acetates (PMAAs) derived from the seven samples. As shown in Table 11, the sample of sodium alginate only gave a weak signal for 4-linked glucose. A comparison of the signals observed in each polysaccharide sample show that components found in the konjac mannan powder were consistent with the reported structure, namely glucose and mannose linked through position 4 with short terminal side chains. The xanthan gum sample gave a weak signal for 2-linked mannose in addition to strong signals for terminal mannose and/or terminal glucose and 4-linked glucose. A signal eluting at 14.65 minutes gave a fragmentation pattern consistent with a 3, 4-linked branched hexose. All the xanthan gum signals are consistent with the reported structure. The signals present at about 14.65 minutes in the xanthan gum and all the VFB and VFC samples are consistent with the 3, 4-linked hexose branch point observed in the xanthan gum sample.

In summary, the overall profile of assignable signals in the samples contains components consistent with konjac mannan, and they also contain components that can be assigned to xanthan gum (the branch point). These methylation results are consistent with the following conclusions. First, both the ungranulated VFB (TM1) and granulated VFC (PGX®) contain both konjac mannan (4-linked mannose) and xanthan gum (3,4-linked branched glucose). The other methylated sugars in the spectrum can emanate from either of these biopolymers. Second, other common biopolymers are absent (e.g., no evidence for galactomannans, carrageenan, or the like), 6-linked glucose (starches). Third, there is no evidence from these results that new sugar-like structures have been formed (e.g., no masses consistent with other sugars, both granulated VFC and ungranulated VFB have similar mass spectra). Fourth, this analysis is not able to identify the sodium alginate component due to the fact that native uronic acids do not methylate (Percival et al., Chemistry and Enzymology of Marine Algal Polysaccharides, Academic Press 101 (1967)). The analysis of sodium alginate is addressed using hydrolysis and chromatography and hydrolysis and NMR spectroscopy, as described below.

### 2. Hydrolysis and GCMS Analysis

**Rationale:**

Xanthan gum and sodium alginate both contain uronic acids, namely glucuronic (xanthan gum) and mannuronic and guluronic (sodium alginate). These structural features are difficult to identify due to the extreme hydrolytic resistance of uronic acids in polysaccharides caused by the electron-withdrawing carboxyl group, which makes it very difficult to achieve the first stage in acid-catalyzed hydrolysis, namely protonation of the glycosidic oxygen atom (Percival et al., Chemistry and Enzymology of Marine Algal Polysaccharides, Academic Press 104 (1967)). This has the effect of making these polysaccharides very stable to attack. Methods of sodium alginate hydrolysis in older literature describes treatment with 90% $H_2SO_4$ for several hours followed by boiling for 24 hours after dilution (Fischer et al., Hoppe-Seyler's Z Physiol Chem 302:186 (1955)). More recently, however, a strong volatile acid, trifluoroacetic acid (TFA) has been used and found to hydrolyze the very resistant bonds in polyuronides with the added advantage of volatility for ease of removal (L. Hough et al., Carbohydrate Research 21:9 (1972)).

This Example describes a new analytical method that was developed for the hydrolysis of VFB and VFC (also referred to as "VFB/C") and characterization of all the hydrolysis products (glucose, mannose, glucoronic acid, mannuronic acid, and guluronic acid) through the use of Chromatography and optional use of NMR.

**Methods:**

**GCMS Analysis:** The partially methylated alditol acetates (PMAAs) were separated and identified by Gas Chromatography-Mass Spectroscopy (GCMS). GC separation was performed with a DB5 column, on-column injection at 45°C and a temperature programme of 1 min at 40°C, then 25°C/min to 100°C, then 8°C/min to 290°C, and finally holding at 290°C for 5 minutes. MS identification was performed with an ionization voltage of 70 eV in scanning mode over a mass range of 50-620 Daltons with unit resolution.

**Partial hydrolysis Conditions:** *Hydrolysis:* Conditions were devised for trifluoroacetic acid (TFA) hydrolysis of VFB/C that would hydrolyze the polysaccharides as completely as possible without attacking the sugars to such an extent that the results would be masked by unwanted degradation products.

TFA hydrolysis was carried out on 30 mg samples shown in TABLE 11 that were placed in sealed tubes with 2 M TFA and heated to 100°C for 1h, 2h, 4h, 8h, 24h, and 72h. Samples were removed from the heat at the stated times, the TFA was evaporated in the freeze drier and the sample was examined by Thin Layer Chromatography (TLC) (solvent: butanol:ethanol:water, 5:3:2) on silica gel plates (Merck TLC silica gel 60°F). Spots were visualized using sulphuric acid (5%) in methanol. It was determined that the best conditions for hydrolyzing the polysaccharides in VFB/C as completely as possible into the component sugars without attacking the sugars to such an extent that the results are masked by unwanted degradation products was 2 M TFA incubation for 72h at 100°C, filtered, freeze dried x2. The results are summarized in TABLE 12.

**Results of Hydrolysis Analysis:**

TABLE 12: Results of TFA hydrolysis

| Sample | lot no. | TFA hydrolysate results |
|---|---|---|
| konjac-mannan | 2538 | mixture of glucose and mannose |
| sodium alginate | 2638 | mixture of mannuronic and guluronic acids |

(continued)

|  | Sample | lot no. | TFA hydrolysate results |
|---|---|---|---|
|  | xanthan gum | 2504 | glucose, mannose, glucuronic acid |
|  | VFC granules (konjac/xanthan/ alginate (70: 13:17) | PGX® lot no. 2029070523 | glucose, mannose and uronic acids |

*Chromatography:*

Having established hydrolysis conditions that release the component sugars from the three polysaccharides, a chromatographic method was developed that is capable of separating both the neutral sugars (glucose, mannose) from the uronic acids (glucuronic acid, mannuronic acid, and guluronic acid).

Dionex acid chromatography is a chromatographic method that has been used extensively on sugars and related compounds. This method of detection is much more sensitive as compared to many of the methods that have been employed in the past such as Refractive Index.

**Methods:**

Equipment: Dionex ICS-3000 Dual pump IC system, electrochemical detector, Chromeleon data system.

Materials: Water (de-ionized and filtered), sodium hydroxide (50% solution, HPLC Electrochem. Grade), sodium acetate, anhydrous ($\geq$ 99.5%).

TABLE 13: Chromatographic conditions:

| Apparatus | Dionex liquid chromatography system fitted with PAD detector | | |
|---|---|---|---|
| Column | Dionex CarboPac PA1 (250 x 4 mm) Dionex CarboPac PA1 Guard (50 x 4 mm) | | |
| Eluent | A: water B: 500 mM sodium acetate in 100 mM NaOH C: 100 mM NaOH | | |
| Gradient | Time | %B | %C |
|  | 0 | 0 | 15.5 |
|  | 20 | 0 | 15.5 |
|  | 21 | 50 | 0 |
|  | 32 | 50 | 0 |
|  | 32.5 | 0 | 100 |
|  | 42 | 0 | 100 |
|  | 42.5 | 0 | 15.5 |
|  | 52 | 0 | 15.5 |
| Flow Rate | 1 ml/min | | |
| Injection Volume | 10 $\mu$l | | |
| Column Temperature | 30°C | | |
| Run Time | 52 min | | |

*Preparation of Samples:* (concentration ~0.02 mg/ml)

Sample solutions were prepared at concentrations of approximately 0.02 mg/ml from an initial concentration in D$_2$O of 30 mg/ml (NMR samples). Aliquots (15 $\mu$l) of hydrolysate and standard solutions were dissolved in deionized water (30mg/ml) and diluted to 0.0225 mg/ml with deionized water for analysis. Standard solutions of each of the expected hydrolysate components from the three polysaccharides: glucose and mannose (from konjac glucomannan and xanthan gum), glucuronic acid (from xanthan gum) and mannuronic and guluronic acids (from sodium alginate) were similarly prepared.

The samples were injected onto a Dionex CarboPac PA1 (250 x 4mm) column with guard column (50 x 4 mm) at 30°C. The column was eluted with a solvent gradient formed with A: deionised water; B: 50 mM sodium acetate (anhydrous ≥ 99.5%) in 100 mM NaOH (HPLC Electrochem grade) and C: 100 mM NaOH at a flow rate of 1ml min-1, as shown in TABLE 13.

**Results of Chromatographic Analysis:**

TABLE 14 shows the results of the Dionex Ion Chromatography of hydrolysates of the various fiber samples.

TABLE 14: Results of Dionex Ion Chromatography of Hydrolysates

| Sample | Retention Time (minutes)/ height (nC)/Rel Area (%) |
|---|---|
| **Standards:** | |
| glucose | 13.98min/175.56nC/99.95% |
| mannose | 15.22min/56.23nC/99.61% |
| glucuronic acid | 25.58min/214.36nC/94.79% |
| mannuronic acid | 25.75min/327.64nC/97.95% |
| guluronic acid* | 25.13* |
| **Test Samples (hydrolysates)** | |
| konjac mannan (Lot#: 2538) | 13.98min/45.26nC/41.05% (glucose)<br>15.22min/57.94nC/57.27% (mannose) |
| xanthan gum (Lot#: 2504) | 13.98min/6.49nC/46.99% (glucose)<br>15.22min/3.99nC/29.93% (mannose)<br>25.58min/2.73nC/4.87% (glucoronic acid) |
| sodium alginate (Lot#: 2638) | 13.95min/0.428nC/3.89% (glucose)<br>15.23min/0.275nC/1.90% (mannose)<br>25.13min/7.06nC/14.95% (guluronic acid)<br>25.75min/35.03nC/75.37% (mannuronic acid) |
| Ungranulated VFB (TM1) Lot #900416 | 13.98min/15.79nC/40.94% (glucose)<br>15.20min/18.76nC/52.42% (mannose)<br>25.58min/1.44nC/0.83% (glucuronic acid)<br>25.75min/3.09nC/1.96% (mannuronic acid) |
| Granulated VFC (PGX®) | 13.98min/15.70nC/40.62% (glucose)<br>15.20min/18.76nC/52.50% (mannose) |
| **Sample** | **Retention Time (minutes)/ height (nC)/Rel Area (%)** |
| Lot # 900495 | 25.58min/1.42nC/0.82% (glucuronic acid)<br>25.75min/3.15nC/2.03% (mannuronic acid) |
| *the use of guluronic acid as a standard was ascertained from the hydrolysis of sodium alginate (assuming that the second significant peak was guluronic acid). | |

TABLE 15: Commercial Biopolymers and their Monosaccharide Components

| Commercial Biopolymer Name | Sugar Profile |
|---|---|
| Starch | glucose |
| carrageenan | galactose |
| sodium alginate | mannuronic acid, guluronic acid |

(continued)

| Commercial Biopolymer Name | Sugar Profile |
|---|---|
| LBG/guar gum | galactose, mannose |
| konjac glucomannan | glucose, mannose |
| Ivory nut mannan | mannose |
| xanthan gum | glucose, mannose, glucuronic acid |
| Larch arabinogalactan | arabinose, galactose |
| Cellulose ethers | glucose |
| Acacia gums (Arabic) | complex mixture |
| VFC (PGX®) | glucose, mannose, glucuronic acid, mannuronic acid |

As shown in TABLE 14, the results of the GCMS analysis indicate that the component sugars and sugar acids were well separated in one 35 minute run. As further shown in TABLE 14, TFA hydrolysis of VFB/C gives a unique profile in which four of the possible monosaccharides, namely glucose, mannose, glucoronic acid, and mannuronic acid, were clearly observed in the Dionex traces. These results are consistent with the composition of VFB/C comprising konjac glucomannan (mannose, glucose), xanthan gum (glucose, mannose, glucuronic acid), and sodium alginate (mannuronic acid and guluronic acid).

TABLE 15 shows the monosaccharide components of various commercial biopolymers, showing that VFC (PGX®) has a unique profile of monosaccharide components. Therefore, these results demonstrate that a TFA hydrolysis and GCMS separation may be used to distinguish VFC from other combinations of monosaccharides.

In summary, the GCMS analysis of the PMAAs of konjac glucomannan and xanthan gum demonstrated the presence of the characteristic sugars and linkages expected from their known primary structures. Konjac glucomannan gave GC peaks corresponding to 4-linked glucose, 4-linked mannose, and terminal glucose and/or mannose (mainly from side chains). Xanthan gum gave strong peaks corresponding to terminal mannose and/or glucose (from side chains) and 4-linked glucose (in the main chain), plus a peak for 3, 4-linked hexose (glucose) and a weak peak for 2-linked mannose (both from side chains). Virtually all of these peaks were also detected in the GCMS analysis of the PMAAs of TM1 and granulated VFC (PGX®), as shown in TABLE 11, showing that they both contain konjac glucomannan and xanthan gum. The trace peak eluting at the position of 2-linked mannose from the xanthan gum component was too weak to assign categorically from the mass spectrum, but the signals at retention times of about 12.47 and 14.65 minutes were consistent with the terminal mannose and the 3, 4-linked hexose (glucose), respectively, of xanthan gum. Importantly, these analyses did not reveal any additional unexpected sugars or sugar linkages in TM1 or granulated VFC (PGX®) that might have emanated from other component biopolymers, or from any new sugars or sugar linkages that might possibly have formed during processing. As expected, the GCMS analysis of the PMAAs of TM1 and granulated VFC was not able to identify sodium alginate components.

With regard to the HPAEC-PAD analysis, TABLE 14 shows the measured retention times of the standards comprising the expected hydrolysate components of TM1 and granulated VFC along with a summary of the chromatograms obtained for hydrolysates of TM1 and granulated VFC. As shown in TABLE 14, four of the possible components (glucose, mannose, glucuronic acid and mannuronic acid) were detected in hydrolysates of TM1 and granulated VFC. The fifth component, guluronic acid, was not detected in this analysis, likely due to the relatively low sodium alginate content of the mixtures. No unexpected hydrolysate components were detected. These results were consistent with the results obtained from the GCMS analysis of PMAAs, supporting the conclusion that TM1 and granulated VFC contains chemically unchanged konjac glucomannan and xanthan gum. Further, the detection of mannuronic acid in the hydrolysates suggests the additional presence of chemically unchanged sodium alginate.

## 3. Nuclear Magnetic Resonance Spectroscopy of Intact and Partially Hydrolyzed Polymers, and Monomeric standards Rationale:

Nuclear Magnetic Resonance Spectroscopy (NMR) is a valuable tool for the analysis of organic molecules, as the spectra contain a vast amount of information about their primary structure through the location of the protons (hydrogen atoms) in the molecule. Thus, on a basic level, the power of $^1$H NMR spectra is to provide adequate levels of primary structural information which can 'fingerprint' various features using well established rules on the chemical shifts and integrals of the standard compounds and unknowns in the mixtures of interest. Carbohydrates have a number of characteristic features in the NMR which make it useful for analysis. The two major characteristic features of the NMR spectra of carbohydrates are (i) the so called "anomeric" resonances, which are protons associated with C1 in the sugar ring, and which typically occur at a lower field than the other major feature; (ii) which is the "ring envelope" of protons associated with the rest of the sugar ring. For example, for glucose, the alpha and beta anomeric resonances are at 5.2 and 4.6 ppm, respectively, and the ring proton envelope is between 3.1 and 3.9 ppm, respectively.

Interestingly, the uronic acids have NMR spectra which are somewhat different than the typical hexose spectrum described above, and their resonances are rather bunched together at slightly higher field than those for glucose and mannose (3.9-5.6 ppm). Santi et al., 12th Int. Electronic Conf on Synthetic Organic Chemistry (ECSOC-12):1-30 (2008). Therefore, the NMR spectra of hydrolysates of the polysaccharides of interest (e.g., VFB/C) may be used to determine the fingerprint of the structural units (glucose, mannose, uronic acid) in the polymers.

In the study described in this example, the NMR spectra were used to fingerprint the complex mixtures that result from hydrolysis of the various polysaccharides and VFB. It is noted that whole polysaccharides cannot be examined in the NMR at the polymer level due to problems with physical characteristics such as viscosity.

**Methods:**

Samples of single polysaccharides and ternary mixtures were partially hydrolyzed with 2M TFA at 100°C for four hours and 24 hours. Filtered hydrolysate samples (30 mg) were dissolved in $D_2O$ (1 ml) and freeze dried before redissolving in $D_2O$ and placing in NMR tubes. Standard solutions of the expected two monosaccharides and three uronic acids were similarly prepared.

NMR spectra were acquired on hydrolysate and standard solutions at 298.1 K with a Bruker 400 MHz Advance III spectrometer with an auto tune broadband multinuclear probe and variable temperature accessory running Bruker Topsin software. 16 scans were run on the majority of samples except for the guluronic acid sample which was given 256 scans.

**Results of NMR Analysis:**

The $^1$H NMR spectra for the monosaccharide and uronic acid standards were well resolved, and their characteristic chemical shifts were found in both the hydrolysates of xanthan gum and sodium alginate, and in the hydrolysate of VFC (PGX®) (data not shown). The chemical shifts observed for glucose and mannose from xanthan gum could be resolved into anomeric resonances (4.6-5.2 ppm) and into sugar ring resonances (3-4 ppm). The chemical shifts observed for mannuronic and guluronic acids from sodium alginate were closer together (between 3.6-5.2 ppm). The uronic acid resonances found in granulated VFC (PGX®) hydrolysates were clearly a combination of those found in hydrolysates of xanthan gum and sodium alginate, further supporting the presence of chemically unchanged sodium alginate in granulated VFC (PGX®).

In summary, the NMR spectra of pure standards, component hydrolysates, and VFC hydrolysates demonstrate that VFC (PGX®) is composed of polysaccharides unchanged in primary structural features of monosaccharide components with unchanged glycosidic links.

**Overall Conclusions:**

The results described in this example demonstrate that the primary chemical structure of granulated VFC (PGX®) was essentially unchanged as compared to the preformulated, unprocessed/ungranulated VFB (TM1). As described in this example, it was shown by the classical method of methylation that the konjac glucomannan and xanthan gum components contained the expected units and links, and that there were no unexplained additional structural components that might have been introduced by the mixing together or processing of the VFB/C components.

Because sodium alginate, one of the components of VFB/C, is resistant to methylation, further methods were employed to complete the structural analysis, including partial hydrolysis, chromatography and NMR, in order to provide further supporting evidence for the unchanged nature of the primary chemical structure of VFB/C.

In summary, the studies described in this Example support the conclusion that granulated VFC (PGX®) is not modified chemically in its primary features by the granulation process.

**EXAMPLE 6**

This example describes the analysis of the flow behavior and macromolecular properties of granulated viscous fiber complex (VFC) (konjac/xanthan/alginate (70:13:17)) granules, (i.e., the fiber blend was processed by granulation to form a complex, commercially known as PGX®). The results described in this Example demonstrate that an interaction is occurring between the components of granulated VFC at the polymer level to establish networks and junction zones to form a novel polysaccharide with the following nomenclature: α-D-glucurono-α-D-manno-β-D-manno-β-D-gluco),(α-L-gulurono-β-D-mannurono),β-D-gluco-β-D-mannan.

**Rationale:**

The studies described in this Example were carried out to investigate whether the ternary granulated VFB/C mixture including konjac mannan, xanthan gum, and sodium alginate contains networks and junction zones involving all three components, resulting in solution flow properties that are unique to processed/granulated VFC as compared to the unprocessed/ungranulated VFB, or the individual components konjac mannan, xanthan gum, or sodium alginate. The presence of non-covalent macromolecular interactions between the three polysaccharides in granulated VFC (PGX®) in solution was investigated with the techniques described below. Since binary interactions between konjac glucomannan and xanthan gum were expected from the results of the study described in Example 5, further analysis was carried out to specifically probe any participation of the third polysaccharide, sodium alginate, in possible ternary interactions.

**1. Rheological Measurements**

In the first study, flow curves were produced at a number of concentrations of unprocessed/ungranulated VFB (Ternary Mixture #1, referred to as "TM1") and granulated VFC (PGX®), and these were compared with flow curves for solutions of each single component of VFB/C alone at the same concentrations to reveal synergistic effects in the flow behavior of aqueous solutions of ternary mixtures.

TABLE 16: Samples used in Rheological Study

| Sample | Lot No. |
|---|---|
| Granulated VFC (PGX®) | 2029070523 |
| Granulated VFC (PGX®) | 900495 |
| Ungranulated VFB (TM1) | 1112050809 |
| Ungranulated VFB (TM1) | 900416 |
| Sodium alginate | 2638 |
| Sodium alginate | 2639 |
| Xanthan gum | 2504 |
| Xanthan gum | 2505 |
| Konjac glucomannan | 2538 |
| Konjac glucomannan | 2681 |

**Sample Preparation:**

Single polysaccharides and ternary mixtures were studied in solution in deionised distilled water. Accurately weighed samples were dispersed at concentrations of 0.1g, 0.2g, and 0.5g in 100 g of water (0.1%, 0.2%, and 0.5%, respectively) at 25°C and allowed to hydrate for two hours with stirring in which a weighed amount of water was placed on a magnetic stirrer and a vortex created before samples, which had been weighed to four decimal places, were slowly poured into the center of the vortex. After two hours, the solutions were sheared with a high speed mixer (IKA shear mixer (15K rpm)) for 1 minute to ensure that all the particulate material had been fully mixed. Samples were then further stirred for 1 hour before being considered suitable for analysis.

**Flow curve measurement:**

Solution flow behavior was measured with a Bohlin Gemini Rheometer using a C14 DIN 53019 concentric cylinder measuring system at 25.0 ± 0.1°C. Steady state shear rates were measured at a series of constant applied shear stresses ascending from 0.1 Pa to 10 Pa. Flow behavior was characterized initially using flow curves of log viscosity versus log shear rate.

**Results:**

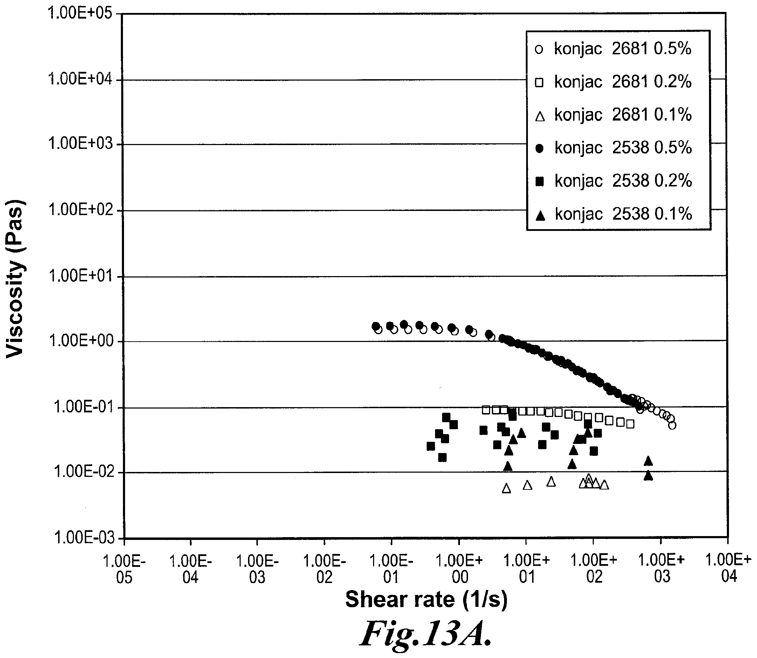

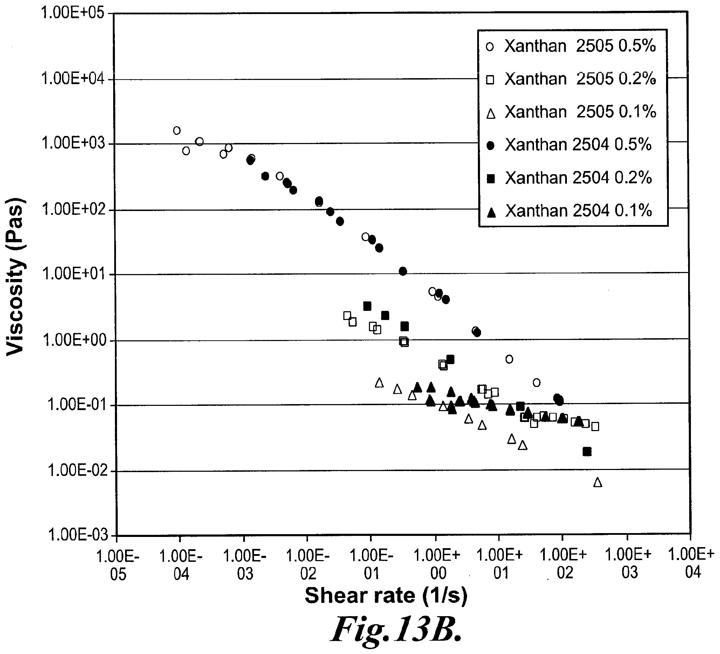

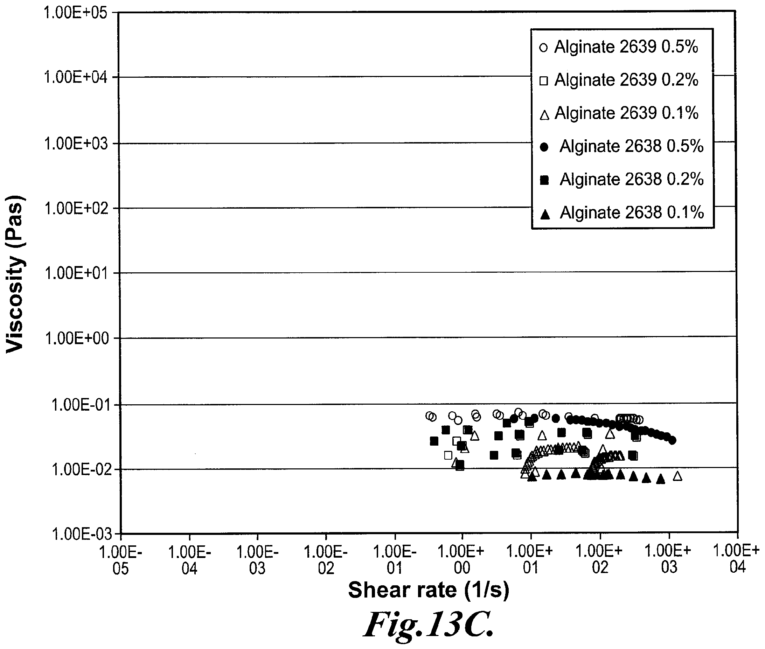

FIGURES 13A, 13B, and 13C graphically illustrate the flow curves of konjac glucomannan, xanthan gum, and sodium alginate, respectively, at 0.1%, 0.2%, and 0.5% w/w as measured at 25°C. The flow curves for the solutions of the individual polysaccharides shown in FIGURES 13A-C show that xanthan gum is the most powerful viscosifying agent (FIGURE 13B), followed by konjac glucomannan (FIGURE 13A) and finally by sodium alginate (FIGURE 13C). Little difference was seen in flow behavior between solutions of different lots of the sample single polysaccharide. Xanthan gum solutions also had the most extensive shear thinning regions across many decades of shear rate where the logarithmic plots were linear.

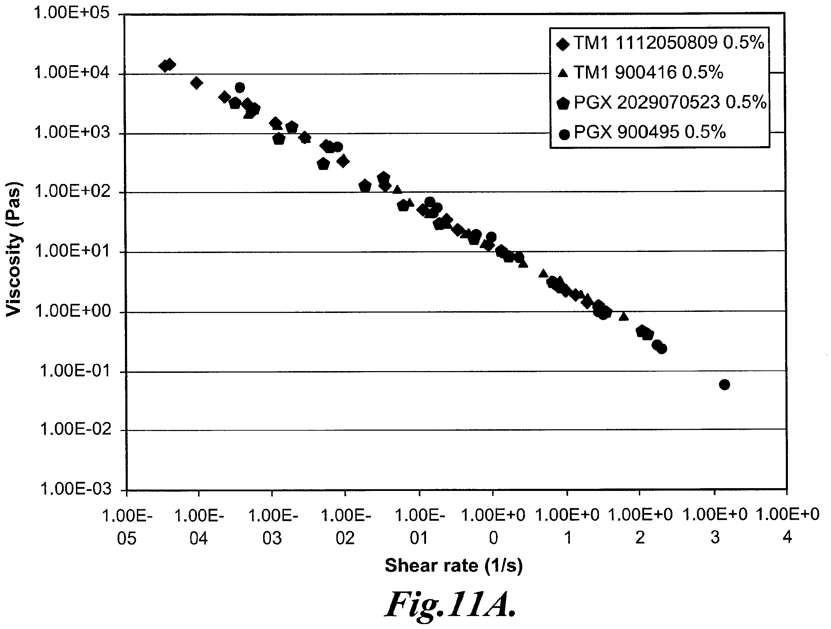

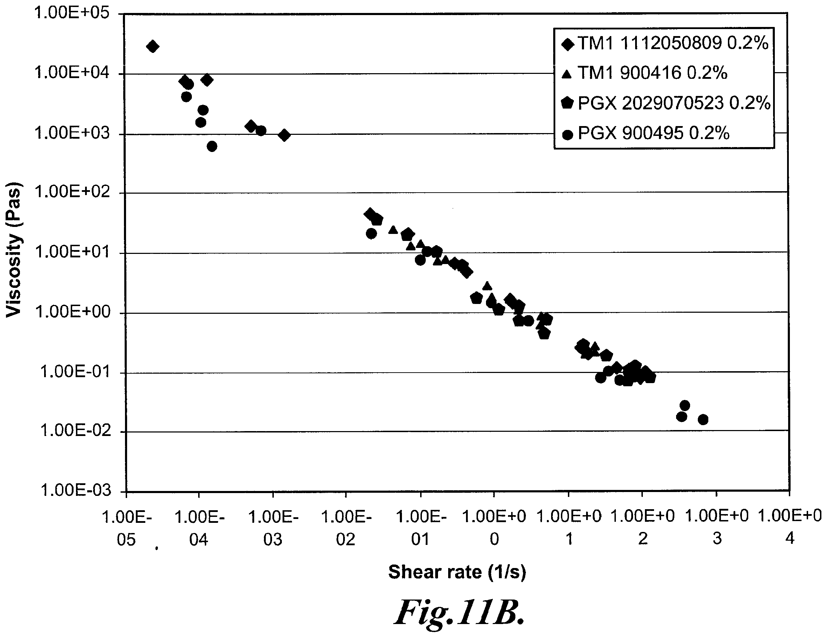

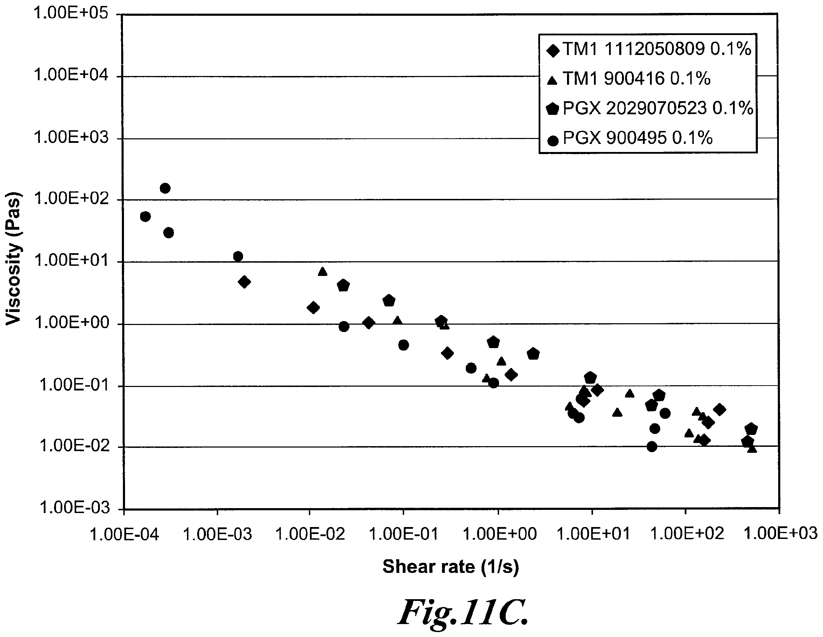

FIGURES 11A-C graphically illustrate the flow curve comparison of unprocessed/ungranulated VFB (TM1) and granulated VFC (PGX®) at 0.5% (w/w) (FIGURE 11A), 0.2% (w/w) (FIGURE 11B) and 0.1% (w/w) (FIGURE 11C).

The data shown in FIGURES 11A-C was further examined by fitting each flow curve to a power-law relationship between viscosity $\eta$ and shear rate $D$ as follows:

$$\eta = KD^{n-1}$$

Where $K$ is the consistency index (giving an overall value of thickness) and $\eta$ is the flow behavior index (indicating deviation from Newtonian behavior) derived from the intercept and slope, respectively, of a logarithmic plot of viscosity against shear rate which is linear for a power law fluid. The $K$ value indicates the overall consistency and $\eta$ indicates the deviation from Newtonian behavior ($\eta = 1$). A Newtonian fluid has an $\eta$ value of 1 and, as $\eta$ decreases below 1, the fluid becomes increasingly shear thinning.

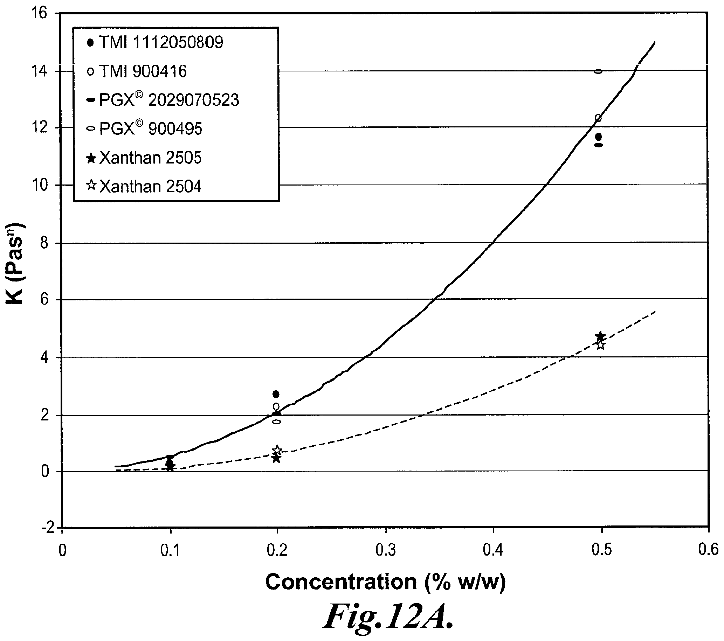

As shown in FIGURES 11A-C, all the unprocessed/ungranulated VFB (TM1) and granulated VFC (PGX®) samples gave very similar flow curves at each concentration, indicating that a low water activity in processing or in a prior aging of the premix had influenced the properties of the mixtures. It is noted that processing was at a much lower overall water activity than the dilute solution conditions of the heat treatment. The flow curves of the VFB/C mixtures were closest to those of xanthan gum; they conformed to the power law and showed extensive shear thinning behavior, but the magnitude of the viscosities and the degree of shear thinning at each concentration were actually higher than those of xanthan gum alone. This is clearly shown by the differences in power law $K$ and $\eta$ values between solutions of the VFB/C mixtures and xanthan gum, as shown in FIGURE 12A.

FIGURE 12A graphically illustrates the power law $K$ comparison of unprocessed/ungranulated VFB (TM1), granulated VFC (PGX®), and xanthan gum. As shown in FIGURE 12A, the unprocessed/ungranulated VFB (TM1) and granulated VFB (PGX®) samples gave very similar $K$ values at each concentration, and the $K$ value increased with concentration. It is noted that higher $K$ values correspond to greater viscosities, and lower $\eta$ values correspond to greater degrees of shear thinning over the concentration range.

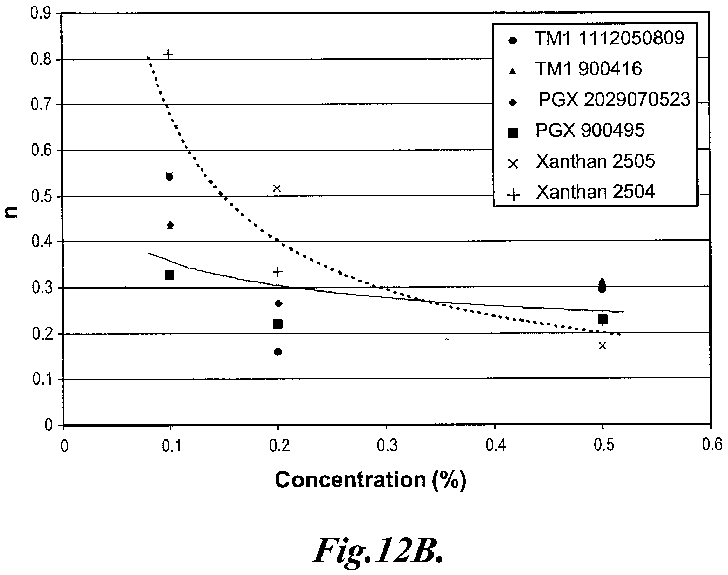

FIGURE 12B graphically illustrates the power law $\eta$ comparison of unprocessed/ungranulated VFB (TM1), granulated VFC (PGX®) and xanthan gum. As shown in FIGURE 12B, the $\eta$ values were also similar for all the VFC samples at each concentration, but appeared to suggest the possible presence of a minimum $\eta$ value, or maximum in the degree of shear thinning, in the region of 0.30 to 0.35%.

Based on the proportions used to generate the granulated VFC (70% konjac glucomannan, 17% xanthan gum, and 13% sodium alginate), the flow behavior of the mixture would be expected to be broadly similar to that of 100% konjac glucomannan, assuming no interactions between the polysaccharides. However, given that konjac glucomannan is the predominant polysaccharide in the ternary mixtures, and xanthan gum and sodium alginate are both minor components, the flow behavior of unprocessed/ungranulated VFB (TM1) and granulated VFC (PGX®) solutions provides a clear indication of an interaction between the polysaccharides in these mixtures.

**Summary:**

Comparing the flow curves of the granulated VFC (PGX®) shown in FIGURE 11 with the flow curves of the individual components shown in FIGURES 13A-13C, these results suggest that an interaction has occurred between the polysaccharides in granulated VFC, giving rise to greater viscosities and degrees of shear thinning behavior than would be expected for the particular ternary composition present in unprocessed/ungranulated VFB (TM1) or granulated VFC (PGX®). The overall flow behavior of the granulated VFC (PGX®) samples was closest to that of xanthan gum, but surprisingly, viscosities of granulated VFC (PGX®) were actually higher than those of xanthan gum alone. This is shown in FIGURES 12A and 12B, which highlight the higher $K$ values, and below approximately 0.45% concentration, the lower $\eta$ comparison values for granulated VFC (PGX®) as compared with xanthan gum. Considering the xanthan gum content of unprocessed/ungranulated VFB (TM1) and granulated VFC (PGX®) is only 17%, and the remaining 83% comprises the less powerful viscosifiers konjac mannan and sodium alginate, these results provide clear evidence of an interaction that has occurred between the polysaccharides in the granulated VFC (PGX®) samples.

## 2. The Effect of Sodium Alginate Concentration and/or Heat Treatment Studies

Additional experiments were carried out to determine the effect of various concentrations of sodium alginate and the effect of heat treatment on the flow behavior and macromolecular properties of VFB/C as follows.

### Methods:

Mixtures of konjac glucomannan, xanthan gum and sodium alginate were prepared. The mixtures contained konjac glucomannan (KM) and xanthan gum (XG) at a constant ratio (KM:XG = 4.12:1) and variable amounts of sodium alginate (A0 to A33) (0%, 2%, 5%, 8%, 11%, 13%, 17%, 21%, 24%, 27%, 30%, and 33%). All the samples were first prepared as dry mixtures of the two-way (konjac glucomannan and xanthan gum) or three-way (konjac-glucomannan, xanthan gum, and alginate) fiber combinations. Each sample (mixture) was weighed to four decimal places (dry), thoroughly mixed using a wrist shaker, and kept at -19°C until needed. Aqueous solutions of each composition were prepared at a single concentration of 0.5% by adding 5.0 g of each sample (mixture) to 1 kg of deionized water with stirring by a magnetic stirrer (i.e., a vortex was first created in the deionized water and the samples were slowly poured into the center of the vortex) and allowed to hydrate until homogeneous over four hours. The aqueous solutions of mixtures were kept at 5°C until all the samples were prepared.

### Heat treatment

20 ml aliquots of each solution were then taken and treated as follows: (i) incubated at ambient temperature (22°C) (unheated), or (ii) heated at 90°C in an oven with thermostatic control (samples were in sealed containers to avoid losses due to evaporation and periodically shaken to ensure complete hydration) for either one hour (A0H to A33H)or four hours (A0H4 to A33H4).

The flow curves at 25°C of the aqueous solutions of the mixtures of konjac glucomannan, xanthan gum, and sodium alginate, containing konjac glucomannan (KM) and xanthan gum (XG) at a constant ratio (KM:XG = 4.12:1) and variable amounts of sodium alginate (0%, 2%, 5%, 8%, 11%, 13%, 17%, 21%, 24%, 27%, 30%, and 33%) were measured at a single concentration of 0.5%. As described above, the solutions were either unheated, heated for one hour, or heated for fourhours.

### Results:

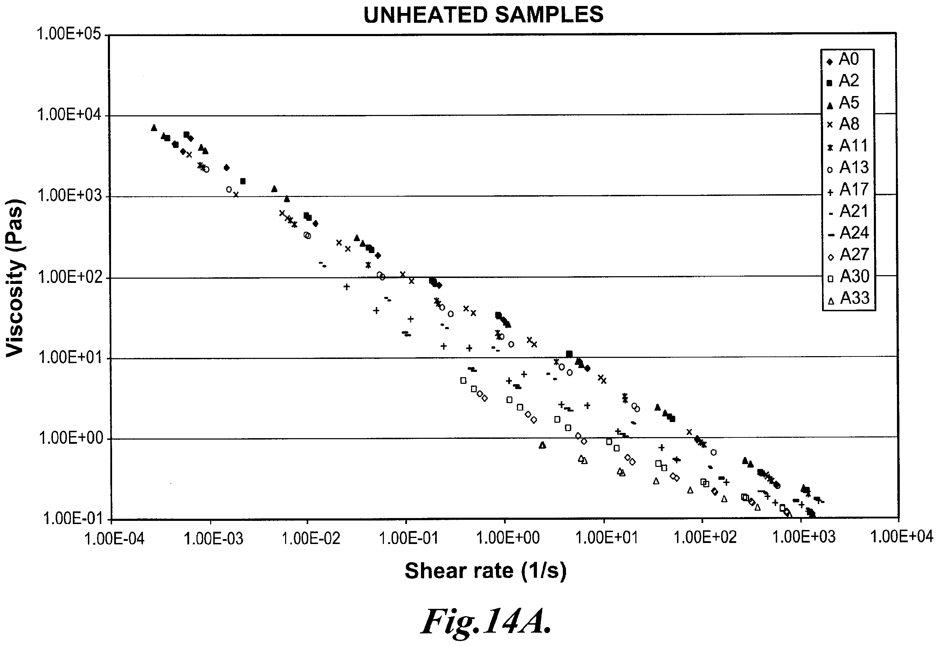

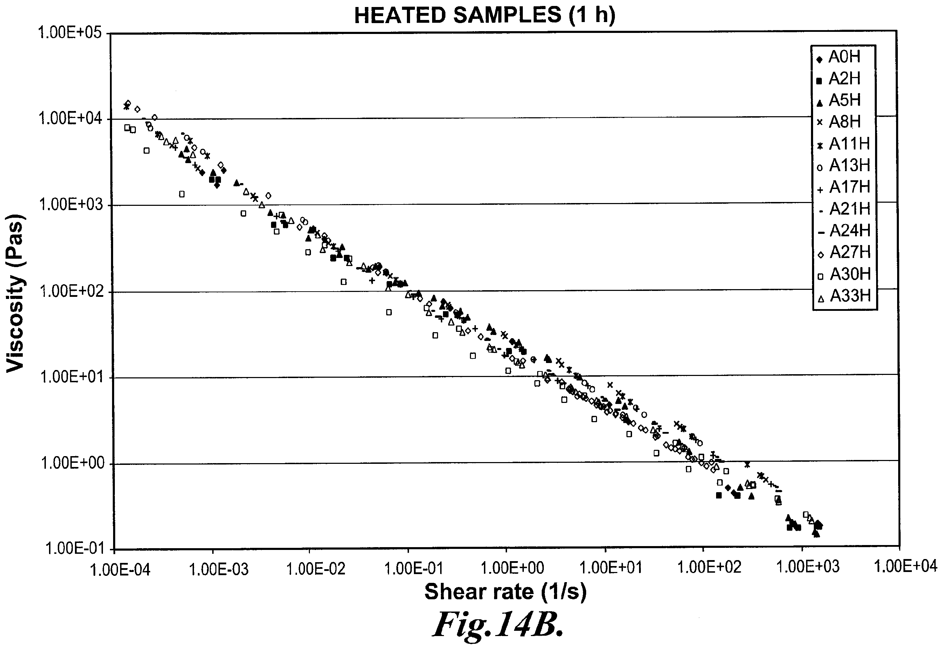

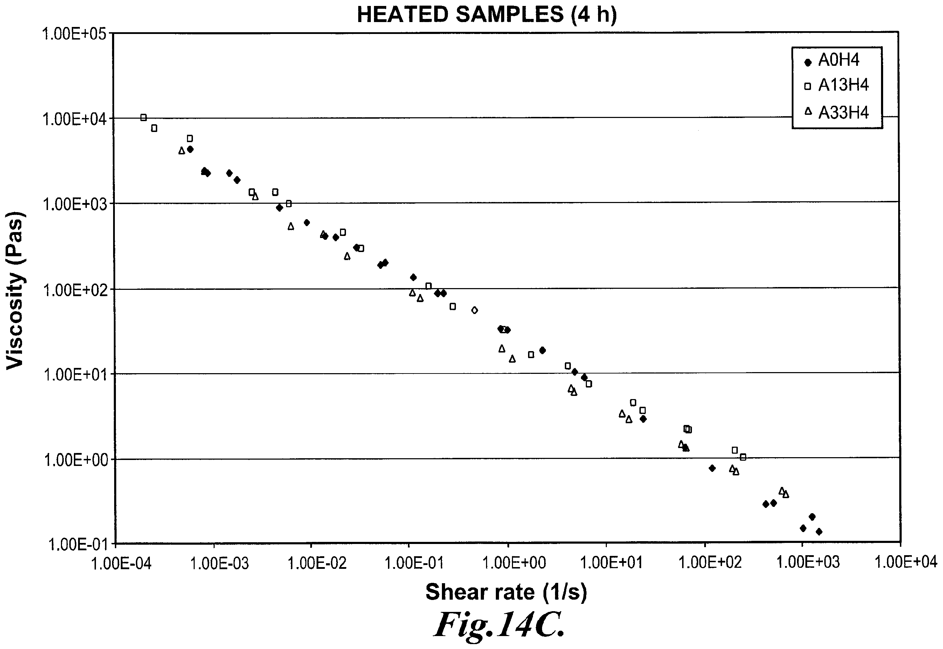

The flow curves (measured at 25°C) for the unheated two-way (A=0), and ternary mixtures are shown in FIGURE 14A. The flow curves (measured at 25°C) for the two-way and ternary mixtures that were heated for one hour are shown in FIGURE 14B. The flow curves (measured at 25°C) for the two-way and ternary mixtures that were heated for four hours are shown in FIGURE 14C.

As shown in FIGURE 14A, for the unheated mixtures, the viscosities appeared to decrease with an increase in the content of sodium alginate, as would be expected if the two more powerful viscosifiers (KM and XG) were being replaced by the weaker viscosifier sodium alginate.

As shown in FIGURE 14B, for the mixtures heated for one hour, the ternary mixtures with an increased sodium alginate content maintained their viscosity to levels higher than the mixtures with the same ratio of alginate that were not heated (shown in FIGURE 14A). As shown in FIGURE 14C, the viscosities of the mixtures that were heated for 4 hours were similar to those observed after heating for one hour. These results indicate that heating the ternary mixture comprising sodium alginate resulted in a ternary interaction between the polysaccharides.

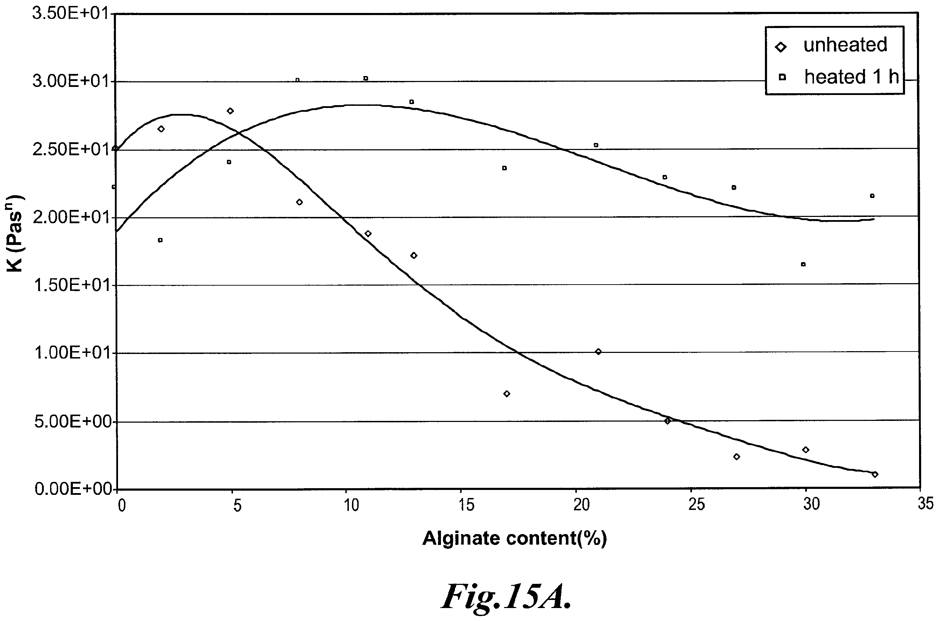

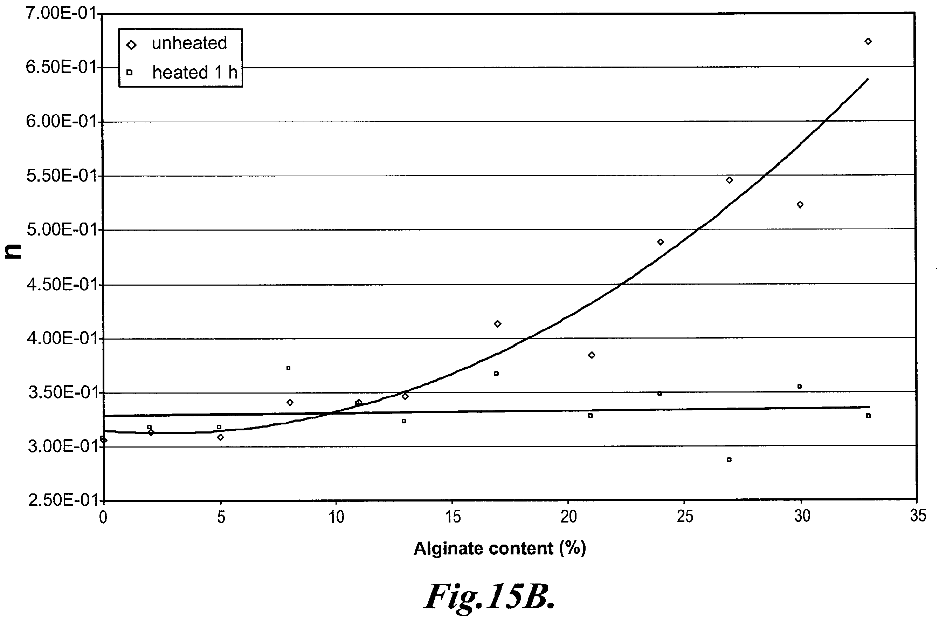

FIGURES 15A and 15B illustrate the dependency of both $K$ and $\eta$ on the proportion of sodium alginate in the mixture for 0.5% aqueous solutions of mixtures of konjac glucomannan, xanthan gum, and sodium alginate at a constant KM:XG ratio (4.12:1) and variable amounts of alginate (0 to 33%). FIGURE 15A graphically illustrates the dependence of the power law $K$ value on unheated and heated ternary mixtures on the alginate content. The flow curves for solutions of the ternary mixtures which had not been heat treated conformed to the power law and principally showed a decrease in viscosity with increase in the content of sodium alginate. As shown in FIGURE 15A, for unheated solutions, the power law $K$ value showed a small initial increase with increase in sodium alginate content, but this was followed by a major decrease. There appeared to be a maximum $K$ value at about 3 to 5% sodium alginate content. As shown in FIGURE 15B, the $\eta$ value increased (towards Newtonian behavior), with an increase in the content of sodium alginate in the mixture. This indicated a progression from a highly viscous and shear thinning binary mixture of konjac glucomannan and xanthan gum (with no sodium alginate) towards a significantly less viscous and less shear thinning ternary mixture at 33% sodium alginate. These results indicate that the sodium alginate was acting as a weaker viscosifier.

The decline in $K$ and increase in $\eta$ values above about 5% sodium alginate, as shown in FIGURE 15A and FIGURE 15B would be expected when the two more powerful viscosifiers (konjac glucomannan and xanthan gum) were being replaced by a weaker, less shear thinning viscosifier (sodium alginate). As shown in FIGURE 15A, similar data was obtained for solutions heated for one hour, indicating an initial decline in the $K$ value due to heat treatment at sodium alginate content below about 5%, but at 11% and above, the decline in $K$ value was much less steep than that observed for the unheated solutions. A maximum in $K$ value occurred in the heat treated solutions at the slightly higher sodium alginate content of 8% to 11% in the ternary mixtures. As shown in FIGURE 15B, the $\eta$ value of heat treated solutions remained low and unchanged across the range of sodium alginate contents. For the limited number of solutions heated for four hours, the $K$ and $\eta$ values were similar to those obtained for the same solutions heated for only one hour (data not shown).

**Summary of Results:**

Overall, these results indicate that heat treatment of the ternary solution significantly increased the overall level of macromolecular interactions. In contrast to the situation during processing where the flow behavior before and after processing was similar, these samples were heat treated in dilute solution and were made up from freshly mixed components. Since the $K$ value for solutions of powder mixtures containing between 0% and about 5% sodium alginate actually declined after heat treatment, this higher level of interactions was unlikely to be due to an enhancement of the interaction between konjac glucomannan and xanthan gum. Rather, it appears that the heat treatment enhanced the interaction of sodium alginate with one or both of the konjac glucomannan and xanthan gum.

Overall, these data suggest that after the heat treatment of the mixture, sodium alginate either restored and strengthened the interaction between konjac glucomannan and xanthan gum, or became involved itself in interactions with the other two polysaccharides in solution. Therefore, these results suggest that sodium alginate may be added to glucomannan and xanthan gum at levels of above 8% to 20% in combination with heat treatment without significantly compromising the rheology of the binary mixture.

### *3. Sedimentation in the Analytical Ultracentrifuge*

**Rationale:**

The unexpectedly high viscosity of VFC (konjac/xanthan/alginate (70:13:17)) granules (i.e., the fiber blend was processed by granulation to form a complex, commercially known as PGX®), also referred to as PolyGlycopleX® ($\alpha$-D-glucurono-$\alpha$-D-manno-$\beta$-D-manno-$\beta$-D-gluco), ($\alpha$-L-gulurono-$\beta$-D mannurono), $\beta$-D-gluco-$\beta$-D-mannan (PGX®), led us to investigate the hydrodynamic properties of mixtures of konjac glucomannan, xanthan, and alginate, as manifested by their sedimentation velocity behavior in the analytical ultracentrifuge, in order to look for interactions at the molecular level which may provide a molecular basis behind these macroscopic observations. In this study, the technique of sedimentation velocity in the analytical ultracentrifuge was used as the probe for investigating the properties of mixtures in which glucomannan was the dominant component, supplemented by xanthan and alginate.

**Methods:**

### *Polysaccharides*

All the polysaccharides used in the study were supplied by InovoBiologic Inc, (Calgary, Alberta, Canada) namely: konjac glucomannan, lot No. 2538; xanthan gum, lot No. 2504; and sodium alginate, lot No. 2455/2639. The polysaccharides were studied individually and as ternary mixtures comprising granulated VFC (referred to in this study as "PGX®"), and ungranulated VFB (referred to in this study as "TM1"). Samples were dissolved in deionized distilled water and then dialyzed into solutions of ionic strength 0.0001M, 0.001M, 0.01M, 0.1M, and 0.2M in phosphate-chloride buffer at pH ~6.8. Ionic strengths >0.05M were supplemented by the addition of NaCl.

### *Analytical ultracentrifugation*

The technique of sedimentation velocity in the analytical ultracentrifuge was used as the probe for the interaction studies. This free-solution method has the advantage over other methods as it does not need columns, membrane materials, other separation media or immobilization which might otherwise disrupt or interfere with interaction phenomena (S.E. Harding Analytical Ultracentrifugation Techniques and Methods, pp. 231-252, Cambridge: Royal Society of Chemistry (2005)). A Beckman XL-I ultracentrifuge was used equipped with Rayleigh interference optics. Data were captured using a CCD camera system. Initial scans were made at a low rotor speed of 3000 rpm to monitor for the presence of very high molecular weight particulates (which were not detected), before adjustment to a rotor speed of 45000 rpm. Sedimentation coefficients $s$ were corrected to standard conditions of the density and viscosity of water at 20.0°C to yield $s_{20,w}$. Scans were taken at two minute intervals for a run time of ~12 hours. Data were analyzed in terms of distributions of sedimentation coefficient distribution g($s$) vs. $s$ (see, e.g., S.E. Harding, Carbohydrate Research 34:811-826 (2005)) using the "least squares g($s$)" SEDFIT algorithm (Dam & Schuck, Methods in Enzymology 384:185 (2003)) based on the finite-element analysis method of Claverie et al., Biopolymers 14:1685-1700 (1975). Analysis of the change in sedimentation coefficient distributions was used to ascertain the presence of an interaction. A total loading concentration of either 2.0 mg/ml or 0.5% (0.5 g in 100g of water) was employed for the controls and mixtures.

## Results and Discussion

### Integrity of the reactants

Konjac glucomannan, xanthan, and alginate were first characterized separately by the analytical ultracentrifuge to establish their molecular integrity.

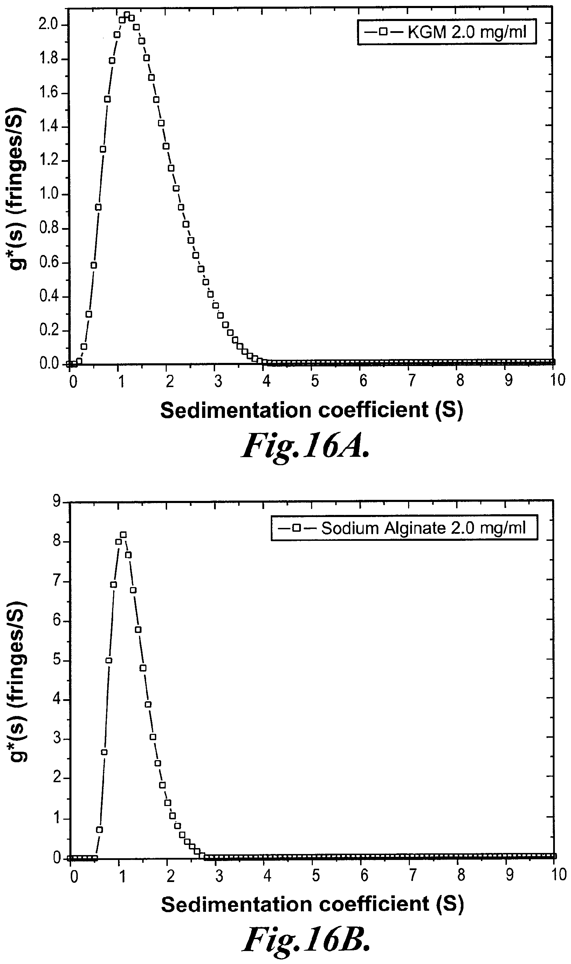

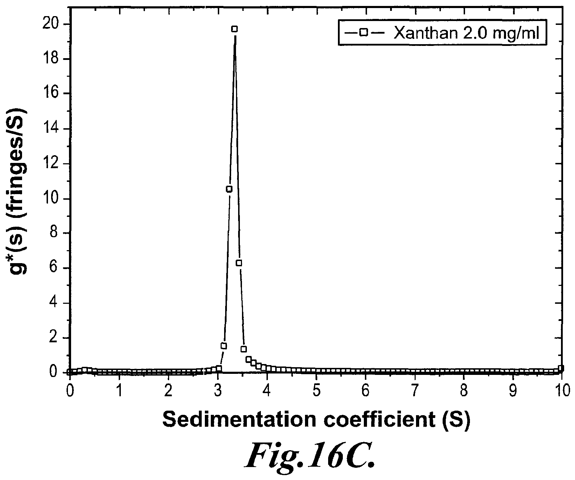

FIGURE 16 graphically illustrates the apparent sedimentation concentration distributions g*(s) vs. sedimentation coefficient (s) for glucomannan (FIGURE 16A), sodium alginate (FIGURE 16B) and xanthan (FIGURE 16C) at a loading concentration of 2 mg/ml and at 1=0.0. Rotor speed 45000 rpm, temperature = 20.0°C. The ordinate is expressed in fringe units per Svedberg (S), and the abscissa is in Svedberg units.

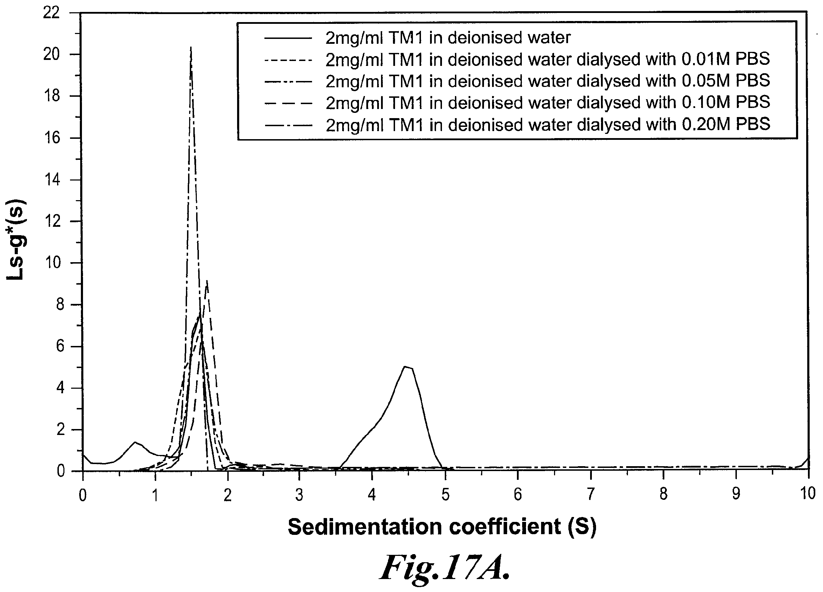

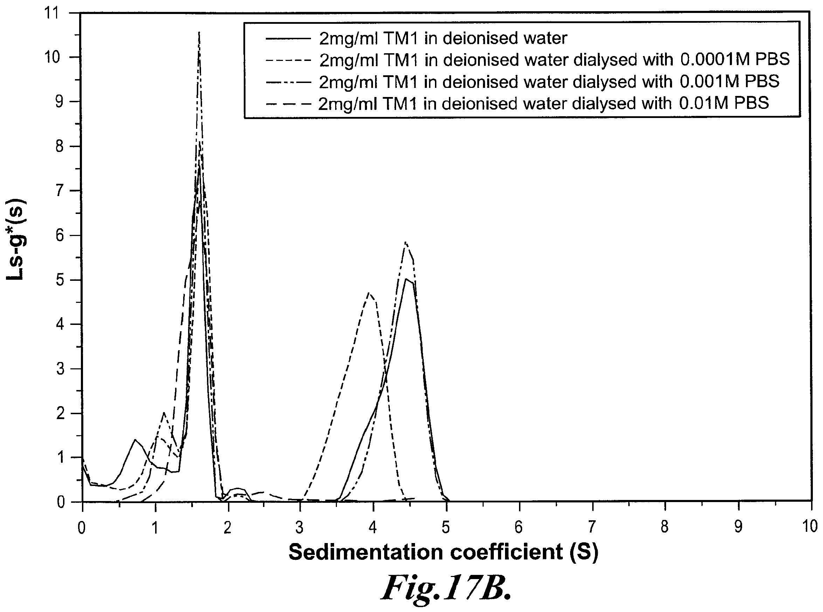

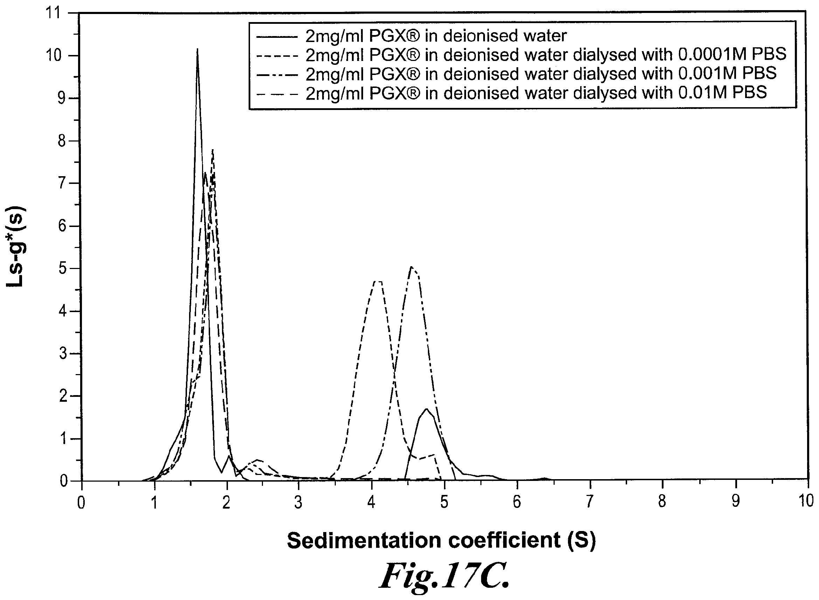

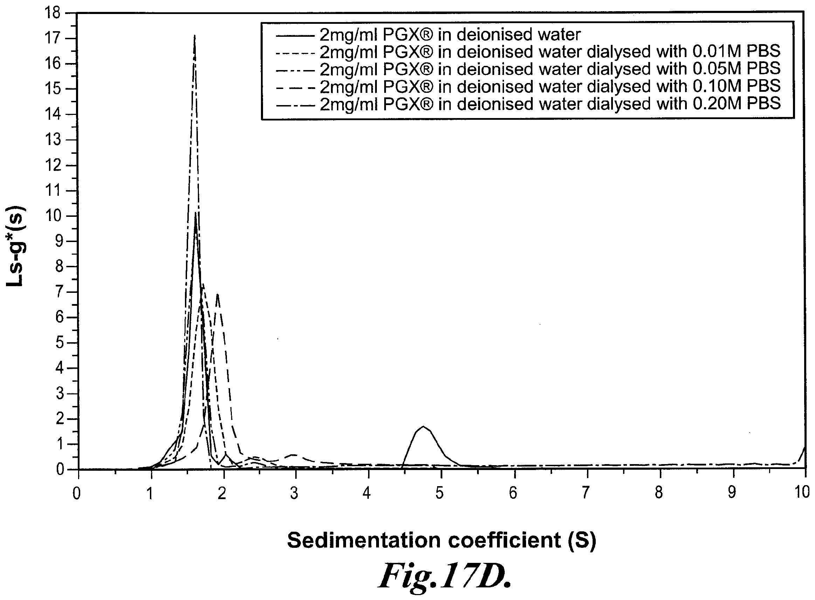

FIGURE 17 graphically illustrates the apparent sedimentation concentration distributions for unprocessed/ungranulated VFB (TM1) at ionic strengths 0-0.2 M (FIGURE 17A); TM1 at ionic strengths 0-0.01 M (FIGURE 17B); granulated VFC (PGX®) at ionic strengths 0-0.01 M (FIGURE 17C); and granulated VFC (PGX®) at ionic strengths 0-0.2M (FIGURE 17D). Rotor speed 45000 rpm, temperature=20.0°C.

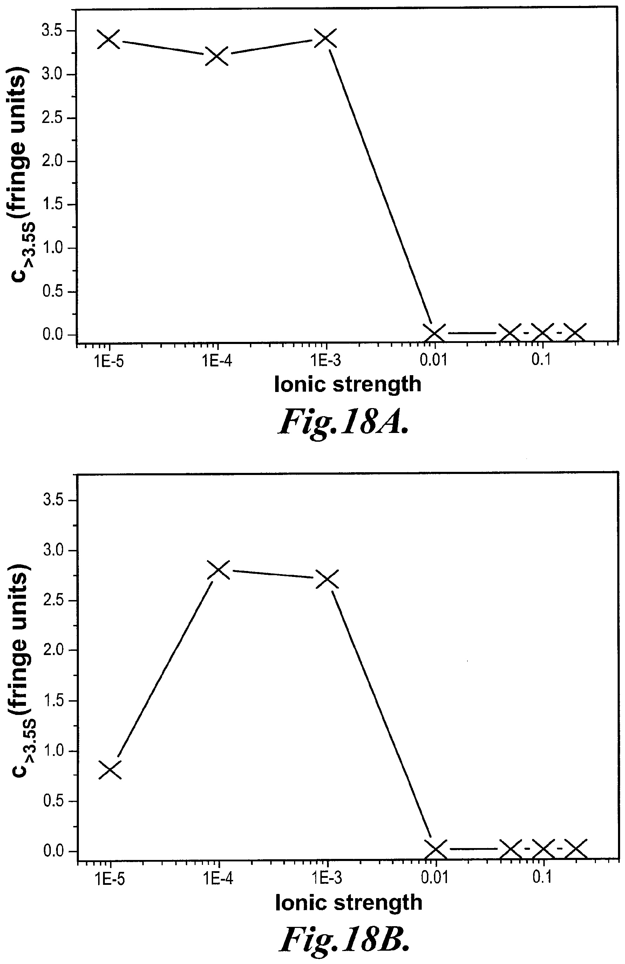

FIGURE 18 graphically illustrates the effect of ionic strength (expressed in molar concentration units M) on the amount of material with a sedimentation coefficient >3.5S for unprocessed/ungranulated VFB (TM1) (FIGURE 18A); or granulated VFC (PGX®) (FIGURE 18B). To facilitate the logarithmic scale the 1=0.00 value is represented at 1=0.00001 M.

Unimodal plots were seen in all cases for the apparent sedimentation coefficient distributions (FIGURES 16A, B, C). Under these conditions, konjac glucomannan has an apparent weight average sedimentation coefficient $s_{20,w}$ of ~1.6S, alginate ~1.3S, and xanthan ~3.5S, where 1S = $10^{-13}$ s.

## Complex Formation and the Effect of Added Electrolyte

Sedimentation coefficient distribution plots were then generated for the following ternary mixtures: unprocessed/ungranulated/unheated VFB (TM1) (FIGURES 17A, B) and granulated/heated VFC (PGX®) (FIGURES 17C, D) at the same total loading concentration used in the controls (2 mg/ml), up to a maximum of 10S. As our criterion for interaction, we estimated the amount of material with apparent sedimentation coefficients greater than that of the highest sedimenting species in the controls - xanthan: material sedimenting at >3.5S is regarded as an interaction product.

Table 17 shows the concentration of sedimenting material >3.5S. The ultracentrifuge cell loading concentration in each case was 2.0 mg/ml.

TABLE 17: Concentration of sedimenting material >3.5S

| Sample | c>3.5S (fringe units) |
|---|---|
| Glucomannan | 0 |
| Alginate | 0 |
| Xanthan | 0.1 ± 0.1 |
| TM1 (unprocessed/ungranulated VFB) | 3.4 ± 0.1 |
| PGX® (granulated VFC) | 0.8 ± 0.1 |

TABLE 17 shows the clear increase in concentration of sedimenting material for both the TM1 and granulated VFC mixtures, in comparison to the individual components, although there is still a considerable proportion of unreacted material particularly at low sedimentation coefficients (~2S). FIGURE 18 and Table 18 also show the effect of an increase in ionic strength on the appearance of the higher sedimenting material.

TABLE 18 shows the results of the effect of ionic strength on TM1 (unprocessed/ungranulated VFB). Ultracentrifuge cell loading concentration in each case was 2.0 mg/ml.

TABLE 18: Effect of ionic strength on TM1 (ungranulated
VFB)

| Ionic Strength (M) | c>3.5S (fringe units) |
| --- | --- |
| 0.0 | 3.4 ± 0.1 |
| 0.0001 | 3.2 ± 0.1 |
| 0.001 | 3.4 ± 0.1 |
| 0.01 | 0 |
| 0.05 | 0 |
| 0.1 | 0 |
| 0.2 | 0 |

TABLE 19 shows the results of the effect of ionic strength on PGX® (granulated VFC). Ultracentrifuge cell loading concentration in each case was 2.0 mg/ml.

TABLE 19: Effect of ionic strength on PGX® (granulated
VFB)

| Ionic Strength (M) | c>3.5S (fringe units) |
| --- | --- |
| 0.0 | 0.8 ± 0.1 |
| 0.0001 | 2.8 ± 0.1 |
| 0.001 | 2.7 ± 0.1 |
| 0.01 | 0 |
| 0.05 | 0 |
| 0.1 | 0 |
| 0.2 | 0 |

It can be seen that, for both granulated and ungranulated mixtures, significant amounts of higher sedimenting material were observed up to an ionic strength of 0.01 M above which the appearance of such material was suppressed (FIGURES 18A, B). FIGURE 18A graphically illustrates the effect of ionic strength (expressed in molar concentration units M) on the amount of material with a sedimentation coefficient > 3.5S for unprocessed/ungranulated VFB (TM1). FIGURE 18B graphically illustrates the effect of ionic strength (expressed in molar concentration units M) on the amount of material with a sedimentation coefficient > 3.5S for processed (e.g., granulated) VFC (PGX®).

### *Distribution of sedimentation coefficients of ternary mixtures*

Sedimentation coefficient distributions for mixtures containing a fixed glucomannan: xanthan gum ratio and varying alginate concentrations (from 0% to 33%). The mixtures were either unheated (0) or heat treated for one hour (HI) or four hours (H4).

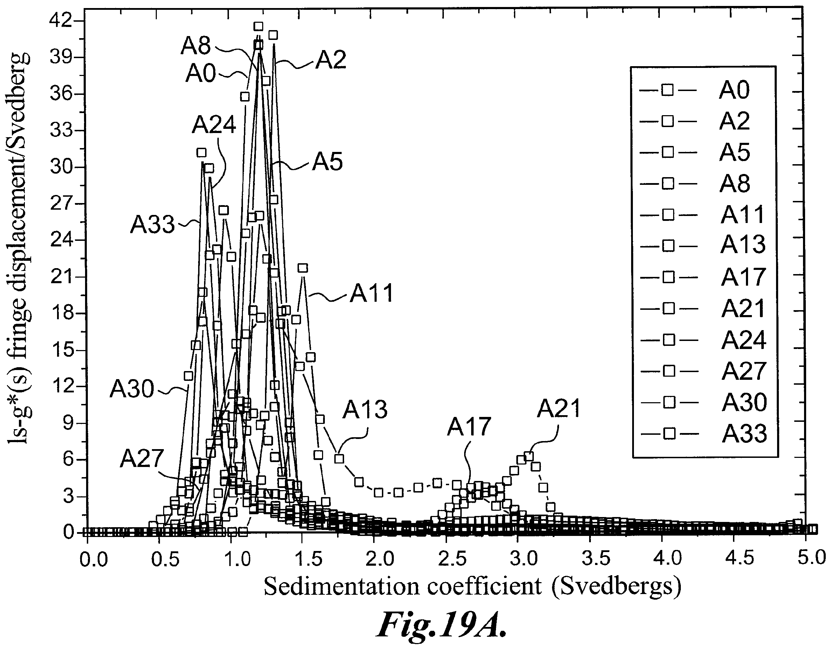

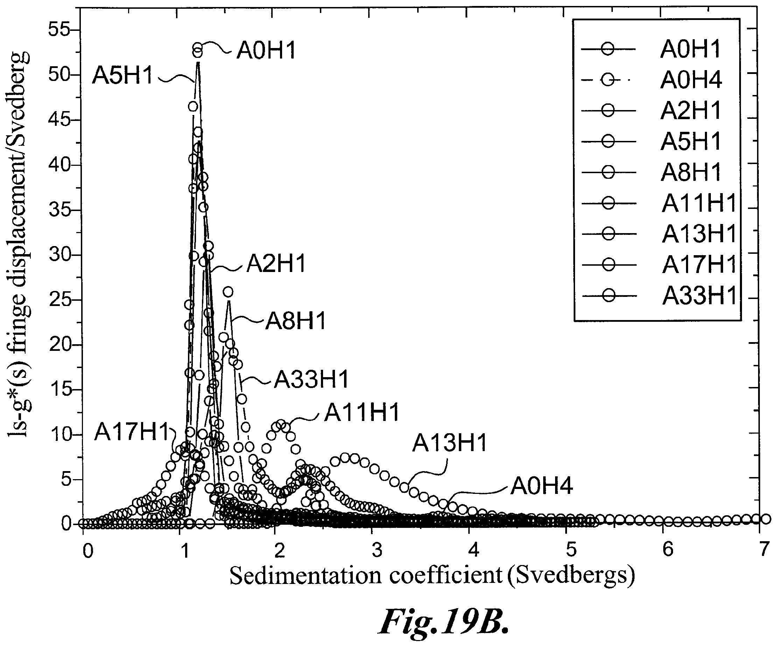

The sedimentation coefficient distributions were determined from the samples in deionised distilled water at a total loading concentration of 5 mg/ml (0.5%). The results for the unheated samples are shown in FIGURE 19A. The results for the heat treated samples are shown in FIGURE 19B. As shown in FIGURES 19A and B, in the absence of alginate, no significant interaction product is observed for the binary glucomannan dominated glucomannan:xanthan mixture for either unheated (A0) or samples treated for one hour (A0H1) or four hours (A0H4), with a sedimentation coefficient distribution essentially that of the glucomannan control (see Abdelhameed et al., Carbohydrate Polymers, 2010). However, the situation is different in the presence of alginate. As shown in FIGURE 19A, the unheated ternary mixture showed some interaction of an alginate content of 13%, 17%, 21%, up to 24%, based on the appearance of higher sedimentation coefficient material, but no significant effects were observed above an alginate concentration of 27%. For the heat-treated samples, as shown in FIGURE 19B, complexes were observed above an alginate concentration of about 8%, consistent with the rheological measurements. It is noted that some of the higher alginate content samples that had been heat treated for an hour, such as A21H1 (21% alginate mixture, heated for one hour), A24H1, A27H1, A30H1 and all four hour treated samples containing alginate had formed gels after the heat treating process and could not be analyzed by the sedimentation velocity method. This implies the presence of interactions in the original solutions of sufficient strength to flip these into the gel state. In contrast, the molecular interactions in the unheated samples were insufficient to promote such a gelation phenomena.

## Conclusions

Mixtures of glucomannan, xanthan, and alginate show the presence of interaction products which are removed on the addition of moderate amounts of electrolyte. These observations are consistent with an interaction within the ternary mixtures which can be suppressed by inclusion of a supporting electrolyte beyond an ionic strength of 0.01 M. The interaction is not stoichiometric, as there is a considerable proportion of material sedimenting at lower sedimentation coefficients (<3.5S, under the conditions we have studied).

## 4. Comparative Treatment of VFC and Sodium Alginate with Calcium Chloride

### Rationale:

Granulated VFC (PGX®) produces highly viscous solutions in water but does not form cohesive gels. The results described above are consistent with the formation of complex interactions of the three components (konjac mannan, xanthan gum, and alginate) at the polymer level. In order to determine whether alginate could be separated from the VFC, an experiment was carried out to test whether the alginate could be separated from VFC in solution by calcium ions. Alginates are known to have calcium mediated precipitation and gelling characteristics (K. Clare, "Algin," in Whistler R.L. and BeMiller J.N. Eds., Industrial Gums, Academic Press 116 (1993); A. Haug et al., Acta Chem. Scand. 19:341-351 (1965)). Pure solutions of sodium alginate react strongly and instantaneously to the addition of calcium ions to form either precipitates or gels depending upon the mode of calcium addition (Clare et al. (1993); Haug et al. (1965)). Thus, in a typical gelling reaction, an insoluble calcium salt such as anhydrous dicalcium phosphate is added to a sodium alginate solution followed by a slow-release acid such as glucono deltalacetone which causes the $Ca^{++}$ ions to be released slowly to cause a homogeneous gel to form. If, however, $Ca^{++}$ ions are added rapidly, as in the case of calcium chloride, then an instantaneous precipitate occurs. The polyguluronate segments of the alginate macromolecule are known to bind most strongly with $Ca^{++}$ ions (Kohn etal., Acta Chemica Scandinavica 22:3098-3102 (1968)), but if these segments were to become less accessible due to interactions with one or both of the other two polysaccharides, calcium alginate precipitation might be restricted.

### Methods:

Aliquots of solutions of unprocessed VFB (TM1) and processed/granulated VFC (PGX®) in deionized distilled water at 0.5% (0.5g in 100g water) were diluted to 0.1, 0.05, and 0.01% w/w. At each concentration, 5 ml of 10% $CaCl_2.2H_2O$ solution was added and thoroughly mixed into the solution to achieve a $Ca^{2+}$ ion concentration of 0.5%. The same addition of Ca2+ ions was also made to (1) a parallel series of control solutions of sodium alginate alone containing the same alginate concentrations as those of the unprocessed VFB (TM1) and processed/granulated VFC (PGX®) solutions; and (2) solutions of binary mixtures of either konjac glucomannan or xanthan gum with sodium alginate containing the same sodium alginate concentrations (measured in g in 100g water) and the same relative proportions of the two polysaccharides as in the 0.5% solutions of the unprocessed VFB (TM1) and processed/granulated VFC (PGX®). The solutions were allowed to stand for 30 minutes before visual inspection for the presence/absence of a precipitate.

### Results:

The results are shown below in TABLE 20.

TABLE 20: Sample Concentrations and Results

| % VFC (granulated PGX®) (lot no. 900495) | Calcium Precipitation (Y/N) | Alginate % (lot no. 2638) | Calcium Precipitation (Y/N) |
|---|---|---|---|
| 0.5 | N | 0.075 | Y |
| 0.1 | N | 0.015 | Y |

(continued)

| % VFC (granulated PGX®) (lot no. 900495) | Calcium Precipitation (Y/N) | Alginate % (lot no. 2638) | Calcium Precipitation (Y/N) |
|---|---|---|---|
| 0.05 | N | 0.0075 | Y |
| 0.01 | N | 0.001 | N |

As shown in the results summarized in TABLE 20, it is clear that in the presence of 0.5% Ca$^{++}$ ions, which will precipitate calcium alginate down to a level of at least 0.0075%, there is no indication of precipitation in solutions of granulated VFC (PGX®) down to the equivalent alginate level. This finding is consistent with the VFB/C components interacting in solution to form junction zones and networks (i.e., at the secondary and tertiary levels of the polysaccharide structures) which then prevent individual components from exhibiting the properties that they would show in a pure state. Calcium alginate precipitates were formed in the corresponding sodium alginate solutions except for the most dilute solution which contained insufficient alginate to be precipitated by 0.5% Ca$^{++}$ ions.

**Conclusion:**

In this study of alginate behavior in granulated VFC (PGX®) it was demonstrated that no precipitation or gel formation occurred when calcium ions were rapidly introduced by the addition of calcium chloride. In a parallel control experiment, calcium chloride was added to pure solutions of sodium alginate of decreasing concentration, which produced an instantaneous precipitate of calcium alginate, even at very low alginate concentrations. These results suggest that the polyguluronate segments of the alginate macromolecule that normally bind strongly with Ca$^{++}$ ions in solution were less available (or unavailable) for such interaction in the VFB/C solutions where sodium alginate was in the presence of konjac glucomannan and xanthan gum. This may be due to these segments of the macromolecule being less accessible or unaccessible to Ca$^{++}$ ions due to alternative interactions with one or both of the other two polysaccharides. Calcium alginate precipitates were observed when Ca$^{++}$ ions were added to the binary solutions of either konjac glucomannan or xanthan gum with sodium alginate, which suggest that the sodium alginate requires the presence of both the other two polysaccharides to interact.

**Discussion of Overall Results**

The results of the primary structural analysis of VFB/C described in Example 5 show that after granulation, the primary structures of the component polysaccharides present in granulated VFC remain unchanged, and that no covalent interactions have occurred either before (TM1) or after a processing involving heat input (granulated VFC). However, the results of the analysis of macromolecular associations described in this example reveal that non-covalent interactions do occur, resulting in a novel polysaccharide complex that is produced at the macromolecular level in VFB/C. The rheological studies clearly show that the solution viscosities of both unprocessed/ungranulated VFB (TM1) and granulated VFC (PGX®) are significantly higher than would be expected from the combination of the viscosifying behaviors of the individual polysaccharides in the mixture. The overall flow characteristics of VFC in solution are closest to those of xanthan gum alone, but the viscosities of VFC are even higher than those of xanthan gum. Considering that the embodiment of VFC tested in this example (70% KM, 17% xanthan, 13% sodium alginate) only contains 17% of the strongest viscosifier, xanthan gum, and 83% of the two weaker viscosifiers, konjac mannan (70%) and sodium alginate (13%), it would be expected that its flow behavior in water would be similar to that of konjac mannan. However, it was determined that the solution flow behavior of VFC was actually closer to that of xanthan gum alone, and its viscosities were even higher than xanthan gum.

Further studies of the rheological and sedimentation behaviour of solutions of ternary mixtures containing variable alginate content prepared in the laboratory confirmed a ternary interaction, and this was enhanced by heat treating the solutions, particularly when the sodium alginate content of the mixture was greater than about 5%. Further, Ca$^{++}$ ion addition experiments showed the presence of both the other two polysaccharides was required to prevent calcium alginate precipitation.

These results demonstrate that, in solution, sodium alginate is interacting with konjac glucomannan and xanthan gum to establish networks and junction zones to form a novel polysaccharide complex with the following nomenclature: α-D-glucurono-α-D-manno-β-D-manno-β-D-gluco),(α-L-gulurono-β-D-mannurono),β-D-gluco-β-D-mannan.

As described in Examples 1-4, it has been determined that the administration of granulated VFC (PGX®) is useful for the prevention, treatment, or amelioration of one or more symptoms associated with a metabolic disease or disorder, such as metabolic syndrome, type I diabetes, type II diabetes, pancreatic disease, or hyperlipidemia, in a subject in need thereof.

**Claims**

1. A medical food for use in the prevention, treatment, or amelioration of one or more symptoms associated with metabolic syndrome comprising a highly viscous polysaccharide dietary fiber composition comprising a viscous fiber blend or complex thereof, comprising from 48% to 90% (w/w) glucomannan, from 5% to 20% (w/w) xanthan gum, and from 5% to 30% (w/w) alginate, and at least one macronutrient selected from the group consisting of protein, carbohydrate, and fat, the medical food being compounded for the prevention, treatment, or amelioration of one or more symptoms associated with metabolic syndrome; wherein the one or more symptoms are selected from the group consisting of glucose induced organ damage, lipid accumulation in the liver and loss of pancreatic beta cells.

2. The medical food for use of Claim 1, wherein the medical food is compounded to provide a daily dose of from 10 g to 100 g of the highly viscous polysaccharide dietary fiber composition, preferably wherein the medical food is compounded to provide a daily dose of from 15 g to 35 g of the highly viscous polysaccharide dietary fiber composition.

3. The medical food for use of Claim 1, wherein the dietary fiber composition comprises from 60% to 80% (w/w) glucomannan, from 10% to 20% (w/w) xanthan gum, and from 10% to 20% (w/w) alginate.

4. The medical food for use of any preceding claim, wherein the highly viscous polysaccharide dietary fiber composition is granulated.

5. Use of a highly viscous polysaccharide dietary fiber composition comprising a fiber blend or complex thereof, comprising from 48% to 90% (w/w) glucomannan, from 5% to 20% (w/w) xanthan gum, and from 5% to 30% (w/w) alginate, in the manufacture of a medical food for use in preventing, treating or ameliorating one or more symptoms associated with metabolic syndrome in a subject, wherein the medical food is administered in an amount to provide from 25 mg/kg/day to 1000 mg/kg/day of the highly viscous polysaccharide dietary fiber composition; wherein the one or more symptoms are selected from glucose induced organ damage, lipid accumulation in the liver and loss of pancreatic beta cells.

6. The use of claim 5, wherein the highly viscous polysaccharide dietary fiber blend is granulated.

7. The use of Claim 5, wherein the subject is suffering from, or at risk for, developing insulin resistance .

8. The use of Claim 5, wherein the medical food is administered at least once a day for a time period of at least two weeks.

**Patentansprüche**

1. Medizinisches Nahrungsmittel zur Verwendung bei der Prävention, Behandlung oder Milderung eines oder mehrerer Symptome in Verbindung mit metabolischem Syndrom, umfassend eine hochviskose Polysaccharid-Ballaststoffzusammensetzung umfassend ein viskoses Ballaststoffgemisch oder einen Komplex davon, umfassend von 48 % bis 90 % (Gew./Gew.) Glucomannan, von 5 % bis 20 % (Gew./Gew.) Xanthangummi und von 5 % bis 30 % (Gew./Gew.) Alginat, und wenigstens einen Makronährstoff ausgewählt aus der Gruppe bestehend aus Protein, Kohlenhydrat und Fett, wobei das medizinische Nahrungsmittel zur Prävention, Behandlung oder Milderung eines oder mehrerer Symptome in Verbindung mit metabolischem Syndrom zusammengesetzt ist; wobei das eine oder die mehreren Symptome ausgewählt sind aus der Gruppe bestehend aus glucoseinduzierter Organschädigung, Lipidakkumulation in der Leber und Verlust von pankreatischen Beta-Zellen.

2. Medizinisches Nahrungsmittel zur Verwendung gemäß Anspruch 1, wobei das medizinische Nahrungsmittel zur Bereitstellung einer Tagesdosis von 10 g bis 100 g der hochviskosen Polysaccharid-Ballaststoffzusammensetzung zusammengesetzt ist, wobei das medizinische Nahrungsmittel vorzugsweise zur Bereitstellung einer Tagesdosis von 15 g bis 35 g der hochviskosen Polysaccharid-Ballaststoffzusammensetzung zusammengesetzt ist.

3. Medizinisches Nahrungsmittel zur Verwendung gemäß Anspruch 1, wobei die Ballaststoffzusammensetzung von 60 % bis 80 % (Gew./Gew.) Glucomannan, von 10 % bis 20 % (Gew./Gew.) Xanthangummi und von 10 % bis 20 % (Gew./Gew.) Alginat umfasst.

**4.** Medizinisches Nahrungsmittel zur Verwendung gemäß einem der vorstehenden Ansprüche, wobei die hochviskose Polysaccharid-Ballaststoffzusammensetzung granuliert ist.

**5.** Verwendung einer hochviskosen Polysaccharid-Ballaststoffzusammensetzung umfassend ein Ballaststoffgemisch oder einen Komplex davon, umfassend von 48 % bis 90 % (Gew./Gew.) Glucomannan, von 5 % bis 20 % (Gew./Gew.) Xanthangummi und von 5 % bis 30 % (Gew./Gew.) Alginat, bei der Herstellung eines medizinischen Nahrungsmittels zur Verwendung bei der Prävention, Behandlung oder Milderung eines oder mehrerer Symptome in Verbindung mit metabolischem Syndrom bei einer Person, wobei das medizinische Nahrungsmittel in einer Menge zur Bereitstellung von 25 mg/kg/Tag bis 1000 mg/kg/Tag der hochviskosen Polysaccharid-Ballaststoffzusammensetzung verabreicht wird; wobei das eine oder die mehreren Symptome ausgewählt sind aus glucoseinduzierter Organschädigung, Lipidakkumulation in der Leber und Verlust von pankreatischen Beta-Zellen.

**6.** Verwendung gemäß Anspruch 5, wobei das hochviskose Polysaccharid-Ballaststoffgemisch granuliert ist.

**7.** Verwendung gemäß Anspruch 5, wobei die Person an der Entwicklung von Insulinresistenz oder dem Risiko dafür leidet.

**8.** Verwendung gemäß Anspruch 5, wobei das medizinische Nahrungsmittel über einen Zeitraum von wenigstens zwei Wochen wenigstens einmal pro Tag verabreicht wird.

**Revendications**

**1.** Aliment médical destiné à une utilisation dans la prévention, le traitement ou l'amélioration d'un ou plusieurs symptômes associés au syndrome métabolique, comprenant une composition de fibres alimentaires à base de polysaccharides et hautement visqueuse, comprenant un mélange de fibres visqueux ou un complexe de celui-ci, comprenant de 48% à 90% (p/p) de glucomannanes, de 5% à 20% (p/p) de gomme xanthane, et de 5% à 30% (p/p) d'alginates, et au moins un macronutriment choisi dans le groupe constitué par les protéines, les glucides et les matières grasses, l'aliment médical étant compoundé pour la prévention, le traitement ou l'amélioration d'un ou plusieurs symptômes associés au syndrome métabolique ; où les un ou plusieurs symptômes sont choisis dans le groupe constitué par les lésions d'organes induites par le glucose, l'accumulation de lipides dans le foie et la perte de cellules bêta pancréatiques.

**2.** Aliment médical destiné à une utilisation selon la revendication 1, où l'aliment médical est compoundé afin d'apporter une dose journalière allant de 10 g à 100 g de la composition de fibres alimentaires à base de polysaccharides et hautement visqueuse, préférablement où l'aliment médical est compoundé afin d'apporter une dose journalière allant de 15 g à 35 g de la composition de fibres alimentaires à base de polysaccharides et hautement visqueuse.

**3.** Aliment médical destiné à une utilisation selon la revendication 1, où la composition de fibres alimentaires comprend de 60% à 80% (p/p) de glucomannanes, de 10% à 20% (p/p) de gomme xanthane, et de 10% à 20% (p/p) d'alginates.

**4.** Aliment médical destiné à une utilisation selon l'une quelconque des revendications précédentes, où la composition de fibres alimentaires à base de polysaccharides et hautement visqueuse est granulée.

**5.** Utilisation d'une composition de fibres alimentaires à base de polysaccharides et hautement visqueuse, comprenant un mélange de fibres ou un complexe de celui-ci, comprenant de 48% à 90% (p/p) de glucomannanes, de 5% à 20% (p/p) de gomme xanthane, et de 5% à 30% (p/p) d'alginates, dans la préparation d'un aliment médical destiné à une utilisation dans la prévention, le traitement ou l'amélioration d'un ou plusieurs symptômes associés au syndrome métabolique chez un sujet, où l'aliment médical est administré selon une quantité destinée à apporter de 25 mg/kg/jour à 1000 mg/kg/jour de la composition de fibres alimentaires à base de polysaccharides et hautement visqueuse ; où les un ou plusieurs symptômes sont choisis dans le groupe constitué par les lésions d'organes induites par le glucose, l'accumulation de lipides dans le foie et la perte de cellules bêta pancréatiques.

**6.** Utilisation selon la revendication 5, où le mélange de fibres alimentaires à base de polysaccharides et hautement visqueux est granulé.

**7.** Utilisation selon la revendication 5, où le sujet soufre, ou risque de souffrir, d'un développement d'une insulinorésistance.

**8.** Utilisation selon la revendication 5, où l'aliment médical est administré au moins une fois par jour pendant une période de temps d'au moins deux semaines.

*Fig.1A.*

*Fig.1B.*

Fig.2A.

Fig.2B.

Fig.2C.

Fig.2D.

*Fig.3A.*

*Fig.3B.*

*Fig.3C.*

*Fig.4.*

*Fig.5A.*

*Fig.5B.*

*Fig.5C.*

*Fig.6.*

*Fig. 7A.*

*Fig. 7B.*

*Fig. 7C.*

*Fig.8A.*

*Fig.8B.*

61

*Fig.8C.*

*Fig.9.*

Fig.10A.

Fig.10B.

Fig.10C.

*Fig.11A.*

*Fig.11B.*

*Fig.11C.*

*Fig.12A.*

*Fig.12B.*

70

*Fig.13A.*

*Fig.13B.*

Fig.13C.

**UNHEATED SAMPLES**

*Fig.14A.*

EP 2 544 696 B1

**HEATED SAMPLES (1 h)**

*Fig.14B.*

EP 2 544 696 B1

**HEATED SAMPLES (4 h)**

Fig.14C.

EP 2 544 696 B1

*Fig.15A.*

*Fig.15B.*

**Fig.16A.**

**Fig.16B.**

*Fig.16C.*

*Fig.17A.*

EP 2 544 696 B1

Fig.17B.

**Fig.17C.**

Sedimentation coefficient (S)

*Fig.17D.*

EP 2 544 696 B1

*Fig.18A.*

*Fig.18B.*

*Fig.19A.*

*Fig.19B.*

**REFERENCES CITED IN THE DESCRIPTION**

**Patent documents cited in the description**

- WO 61312630 A
- WO 61357658 A
- US 2008027024 A
- US 2006228397 A
- US 40076806 A
- US 83061507 A

**Non-patent literature cited in the description**

- **E.J. GALLAGHER et al.** *Endocrinol. Metab. Clin. North Am.,* 2008, vol. 37, 559-79
- **YIP et al.** *Obesity Res.,* 2001, vol. 9, 341-347
- **D.E. MOLLER et al.** *Annu. Rev. Med.,* 2005, vol. 56, 45-62
- **LUDWIG et al.** *Pediatrics,* 1999, vol. 103 (3), E26
- **STRACHAN et al.** *Physiol. Behav.,* 2004, vol. 80 (5), 675-82
- **SCHULTES et al.** *J. Clin. Endocrinol. Metabol.,* 2003, vol. 88 (3), 1133-41
- **YLONEN et al.** *Diabetes Care,* 2003, vol. 26, 1979-85
- **MARCIANI et al.** *Am. J. Physiol. Gastrointest. Liver Physiol.,* 2001, vol. 280, G1227-33
- Chemical Engineers Handbook
- **C. DAUBIOUL et al.** *J. Nutr.,* 2002, vol. 132, 967-973
- **J.M. LENHARD et al.** *Biochem. & Biophys. Res. Comm.,* 2004, vol. 324, 92-97
- **J.N. WILSON.** *Atheriosclerosis,* 1984, vol. 4, 147-153
- **J.W. ANDERSON et al.** *J. Nutr.,* 1994, vol. 124, 78-83
- **P. ROZAN et al.** *Br. J. Nutr.,* 2008, vol. 99, 1-8
- **MOTULSKY H.** Intuitive Biostatistics. University Press, 1995
- **D.S. PRATT et al.** Harrison's Principles of Internal Medicine. 2001, 1711-1715
- **REAGAN-SHAW et al.** *FASEB Journal,* 2007, vol. 22, 659-661
- **S.P. MASSIMINO et al.** *J. Nutr.,* 1998, vol. 128, 1786-1793
- **R.A. REIMER et al.** *Endocrinology,* 1996, vol. 137, 3948-3956
- **Y.J. SONG et al.** *Clin. Exp. Pharm. Physiol.,* 2000, vol. 27, 41-45
- **H.R. BRADY ; B.M. BRENNER ; E. BRAUNWALD et al.** Pathogenesis of Glomerular Injury, in Harrison's Principles of Internal Medicine. 2001, 1572-1580
- **A. VILJANEN et al.** *J. Clin. Endocrinol. Metab.,* 2009, vol. 94, 50-55
- **W.U. JIE et al.** *Biomed. Environ. Sci.,* 1997, vol. 10, 27-37
- **A. SANDBERG et al.** *Am. J. Clin. Nutr.,* 1994, vol. 60, 751-756
- **R. WOOD et al.** *Metab. Clin. Exp.,* 2007, vol. 56, 58-67
- **N.M. DELZENNE et al.** *J. Nutr.,* 1999, vol. 129, 1467-1470
- **Z. MAO-YU et al.** *Biomed. Environ. Sci.,* 1990, vol. 3, 99-105
- **A.M. GADJA et al.** *An. Lab News,* 2007, vol. 13, 1-7
- **M. HAFIDI et al.** *Clin. Exp. Hyperten.,* 2006, vol. 28, 669-681
- **P. ROZAN et al.** *Br. J. Nutr.,* 2008, vol. 98, 1-8
- **A.M. GADJA et al.** *An Lab News,* 2007, vol. 13, 1-7
- **N.C. HOWARTH et al.** *Nutr. Rev.,* 2001, vol. 59, 163-169
- **G. GRUNBERGER et al.** *Diabet. Metab. Res. Rev.,* 2006, vol. 23, 56-62
- **J.R. PAXMAN et al.** *Nutr. Res.,* 2008, vol. 51, 501-505
- **N.N. KOK et al.** *J. Nutr.,* 1998, vol. 128, 1099-1103
- **W.U. JIE et al.** *Biomed. Environ Sci.,* 1997, vol. 10, 27-37
- **K. FUKIOKA.** *Obesity Res,* 2002, vol. 10 (12), 116-123
- **A. PEETERS et al.** *Ann. Intern. Med.,* 2003, vol. 138, 24-32
- **K.M. QUEENAN et al.** *Nutr. J.,* 2007
- **T.A. SHAMLIYAN et al.** *J. Family Practice,* 2006, vol. 55, 761-69
- **I.G. CARABIN et al.** *Nutrition J.,* 2008, vol. 8, 9
- **R. FERRARO et al.** *J. Clin. Invest.,* 1992, vol. 90, 780-784
- **J.B. DIXON et al.** *Aust. Fam. Physician,* 2006, vol. 35, 576-79

- **J.W. ANDERSON et al.** *Ann. Pharmacother.,* 2006, vol. 40, 1717-23
- **W.B. KANNEL et al.** *Am. J. Cardiol.,* 1990, vol. 66, 1B-10B
- **G. REAVEN et al.** *Recent Prog. Horm. Res.,* 2004, vol. 59, 207-23
- **REIMER ; RUSSELL.** *Obesity,* 2008, vol. 16, 40-46
- **P.D. CANI et al.** *Br. J. Nutr.,* 2004, vol. 92, 521-526
- **T.C. ADAM ; R.S. WESTERERP-PLANTENGA.** *Br. J. Nutr.,* 2005, vol. 93, 845-851
- **WREN ; BLOOM.** *Gastroenterology,* 2007, vol. 132, 2116-2130
- **VAN NUENEN et al.** *Microbial Ecology in Health and Disease,* 2003, vol. 15, 137-144
- **R.L. BATTERHAM et al.** *Nature,* 2002, vol. 418, 650-654
- **P.D. CANI et al.** *Am. J. Clin. Nutr.,* 2009
- **V. DUMOULIN et al.** *Endocrinology,* 1998, vol. 139, 3780-3786
- **H.L. CHEN et al.** *J. Am. Coll. Nutr.,* 2008, vol. 27, 102-108
- **A.M. WREN et al.** *J. Clin. Endocrinol. Metab.,* 2001, vol. 86, 5992-5995
- **M. TSCHOP et al.** *Nature,* 2000, vol. 407, 908-913
- **OVERDUIN et al.** *Endocrinology,* 2005, vol. 146, 845-850
- **K.S. JUNTUNEN et al.** *Am. J. Clin. Nutr.,* 2003, vol. 78, 957-964
- **CANI et al.** *Am J Clin Nutr,* 2009, vol. 90, 1236-1243
- **ICHIMURA A. et al.** *Prostaglandins & Other Lipid Mediators,* 2009, vol. 89, 82-88
- **GE H et al.** *Endocrinology,* 2008, vol. 149, 4519-4526
- **PARNELL J.A. et al.** *Am J Clin Nutr,* 2009, vol. 89, 1751-1759
- **BEWLEY et al.** Biochemistry of Storage Carbohydrates in Green Plants. Academic Press, 1985, 289-304
- **GRASDALEN, H. et al.** *Carbohydr Res,* 1981, vol. 89, 179-191
- **GRANT, G.T. et al.** *FEBS Lett,* 1973, vol. 32, 195-198
- **H. BJÖRNDAL et al.** *Carbohydrate Research,* 1967, vol. 5, 433-40
- **PERCIVAL et al.** Chemistry and Enzymology of Marine Algal Polysaccharides. Academic Press, 1967, 101
- **PERCIVAL et al.** Chemistry and Enzymology of Marine Algal Polysaccharides. Academic Press, 1967, 104
- **FISCHER et al.** *Hoppe-Seyler's Z Physiol Chem,* 1955, vol. 302, 186
- **L. HOUGH et al.** *Carbohydrate Research,* 1972, vol. 21, 9
- **SANTI et al.** *12th Int. Electronic Conf on Synthetic Organic Chemistry (ECSOC-12),* 2008, 1-30
- **S.E. HARDING.** Analytical Ultracentrifugation Techniques and Methods. Royal Society of Chemistry, 2005, 231-252
- **S.E. HARDING.** *Carbohydrate Research,* 2005, vol. 34, 811-826
- **DAM ; SCHUCK.** *Methods in Enzymology,* 2003, vol. 384, 185
- **CLAVERIE et al.** *Biopolymers,* 1975, vol. 14, 1685-1700
- **ABDELHAMEED et al.** *Carbohydrate Polymers,* 2010
- Industrial Gums. **K. CLARE.** Algin. Academic Press, 1993, 116
- **A. HAUG et al.** *Acta Chem. Scand.,* 1965, vol. 19, 341-351
- **KOHN et al.** *Acta Chemica Scandinavica,* 1968, vol. 22, 3098-3102